(19) Europäisches Patentamt
European Patent Office
Office européen des brevets

(11) EP 2 956 172 B1

# (12) FASCICULE DE BREVET EUROPEEN

(45) Date de publication et mention de la délivrance du brevet:
**25.04.2018 Bulletin 2018/17**

(21) Numéro de dépôt: **14703897.0**

(22) Date de dépôt: **12.02.2014**

(51) Int Cl.:
***A61K 47/36*** (2006.01) ***A61K 38/28*** (2006.01)
***A61P 3/10*** (2006.01) ***A61K 9/00*** (2006.01)
***A61K 47/10*** (2017.01) ***A61K 9/08*** (2006.01)

(86) Numéro de dépôt international:
**PCT/EP2014/052762**

(87) Numéro de publication internationale:
**WO 2014/124993 (21.08.2014 Gazette 2014/34)**

(54) **SOLUTION INJECTABLE A PH7 COMPRENANT AU MOINS UNE INSULINE BASALE DONT LE POINT ISOELECTRIQUE EST COMPRIS ENTRE 5,8 ET 8,5 ET UN POLYMERE ANIONIQUE HYDROPHOBISE**

INJEKTIONSLÖSUNG MIT EINEM PH-WERT VON 7 MIT MINDESTENS EINEM BASALINSULIN MIT EINEM ISOELEKTRISCHEN PUNKT ZWISCHEN 5,8 UND 8,5 SOWIE HYDROPHOBIERTEN ANIONISCHEN POLYMER

INJECTABLE SOLUTION AT PH 7 COMPRISING AT LEAST ONE BASAL INSULIN HAVING AN ISOELECTRIC POINT OF BETWEEN 5.8 AND 8.5, AND A HYDROPHOBISED ANIONIC POLYMER

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.02.2013 FR 1351200**
**12.02.2013 US 201361763769 P**

(43) Date de publication de la demande:
**23.12.2015 Bulletin 2015/52**

(73) Titulaire: **Adocia**
**69003 Lyon (FR)**

(72) Inventeurs:
- **SOULA, Olivier**
  **F-69330 Meyzieu (FR)**
- **CHARVET, Richard**
  **F-69140 Rillieux-la-Pape (FR)**

(74) Mandataire: **Tripoz, Inès**
**Cabinet Tripoz**
**Le Pôle Sud**
**22 rue Seguin**
**69002 Lyon (FR)**

(56) Documents cités:
**EP-A1- 2 360 188 WO-A1-2007/116143**

- **BAUDYS M ET AL: "Extending insulin action in vivo by conjugation to carboxymethyl dextran", BIOCONJUGATE CHEMISTRY, ACS, WASHINGTON, DC, US, vol. 9, no. 2, 5 février 1998 (1998-02-05) , pages 176-183, XP002363506, ISSN: 1043-1802, DOI: 10.1021/BC970180A**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 956 172 B1

## Description

L'invention concerne les thérapies par injection d'insuline(s) pour traiter le diabète.

L'insulinothérapie, ou thérapie du diabète par injection d'insuline, a connu ces dernières années des progrès remarquables grâce notamment à la mise au point de nouvelles insulines offrant une meilleure correction de la glycémie des patients en comparaison de l'insuline humaine et qui permettent de mieux simuler l'activité physiologique du pancréas.

Lorsqu'un diabète de type II est diagnostiqué chez un patient, un traitement graduel est mis en place. Le patient prend en premier lieu des antidiabétiques oraux (OAD) comme la Metformine. Lorsque les OAD seuls ne suffisent plus à réguler le niveau de glucose dans le sang, un changement dans le traitement doit être fait et, en fonction des spécificités des patients, différentes associations de traitement peuvent être mises en place. Le patient peut par exemple avoir un traitement à base d'une insuline basale de type glargine ou detemir en complément des OAD, puis ensuite, en fonction de l'évolution de la pathologie, un traitement à base d'insuline basale et d'insuline prandiale.

Par ailleurs, aujourd'hui, pour assurer la transition des traitements par les OAD, lorsque ceux-ci ne sont plus en mesure de contrôler le niveau de glucose dans le sang, vers un traitement insuline basale/insuline prandiale, l'injection d'analogues de GLP-1 est préconisée.

Les GLP-1 pour Glucagon-Like Peptide-1, sont des peptides insulinotropiques ou incrétines, et appartiennent à la famille des hormones gastro-intestinales (ou Gut Hormones) qui stimulent la sécrétion d'insuline lorsque la glycémie est trop élevée, par exemple après un repas.

Les hormones gastro-intestinales (Gut hormones) sont aussi appelées hormones de satiété. Elles comprennent notamment le GLP-1 (Glucagon like peptide-1) et le GIP (Glucose-dependent insulinotropic peptide), l'oxyntomoduline (un dérivé du proglucagon), le peptide YY, l'amyline, la cholecystokinine, le polypeptide pancréatique (PP), la ghreline et l'entérostatine, qui ont des structures peptidiques ou protéiques. Elles stimulent également la sécrétion d'insuline, en réponse au glucose et aux acides gras et sont donc à ce titre des candidats potentiels pour le traitement du diabète.

Parmi celles-ci, les GLP-1 sont celles qui ont apporté à ce jour les meilleurs résultats dans le développement de médicaments. Elles ont permis à des patients atteints de diabète de type II de perdre du poids tout en ayant un meilleur contrôle de leur glycémie.

Des analogues ou des dérivés de GLP-1 ont ainsi été développés notamment pour améliorer leur stabilité.

D'autre part, pour couvrir ses besoins journaliers en insuline, un patient diabétique dispose, actuellement, de façon schématisée, de deux types d'insulines ayant des actions complémentaires : les insulines prandiales (ou insulines dites à action rapide) et les insulines basales (ou insulines dites à action lente).

Les insulines prandiales permettent une prise en charge rapide (métabolisation et/ou stockage) du glucose apporté lors des repas et collations. Le patient doit s'injecter une insuline prandiale avant chaque prise alimentaire, soit environ 2 à 3 injections par jour. Les insulines prandiales les plus utilisées sont : l'insuline humaine recombinante, NovoLog® (insuline aspart de NOVO NORDISK), Humalog® (insuline lispro de ELI LILLY) et Apidra® (insuline glulisine de SANOFI-AVENTIS).

Les insulines basales assurent le maintien de l'homéostasie glycémique du patient, en dehors des périodes de prise alimentaire. Elles agissent essentiellement pour bloquer la production endogène de glucose (glucose hépatique). La dose journalière d'insuline basale correspond généralement à 40-50 % des besoins totaux journaliers en insuline. Selon l'insuline basale utilisée, cette dose est dispensée en 1 ou 2 injections, régulièrement réparties au cours de la journée. Les insulines basales les plus utilisées sont Levemir® (insuline detemir de NOVO NORDISK) et Lantus® (insuline glargine de SANOFI-AVENTIS).

On notera pour être exhaustif que la NPH (insuline NPH pour Neutral Protamine Hagedorn ; Humuline NPH®, Insulatard®) est la plus ancienne insuline basale. Cette formulation est le résultat d'une précipitation de l'insuline humaine (anionique à pH neutre) par une protéine cationique, la protamine. Les microcristaux ainsi formés sont dispersés dans une suspension aqueuse et se dissolvent lentement après injection sous-cutanée. Cette dissolution lente assure une libération prolongée de l'insuline. Cependant, cette libération n'assure pas une concentration constante d'insuline au cours du temps. Le profil de libération est en forme de cloche et dure seulement entre 12 et 16 heures. Elle est donc injectée deux fois par jour. Cette insuline basale NPH est bien moins performante que les insulines basales modernes, Levemir® et Lantus®. La NPH est une insuline basale à action intermédiaire.

Le principe de la NPH a évolué avec l'apparition des insulines analogues rapides pour donner des produits appelés « Premix » offrant à la fois une action rapide et une action intermédiaire. NovoLog Mix® (NOVO NORDISK) et Humalog Mix® (ELI LILLY) sont des formulations comprenant une insuline analogue rapide, Novolog® et Humalog®, complexée partiellement par la protamine. Ces formulations contiennent ainsi des microcristaux d'insuline analogue dont l'action est dite intermédiaire et une partie d'insuline restée soluble dont l'action est rapide. Ces formulations offrent bien l'avantage d'une insuline rapide mais elles ont aussi le défaut de la NPH, c.-à-d. une durée d'action limitée entre 12 et 16 heures et une insuline libérée en « cloche ». Cependant, ces produits permettent au patient de s'injecter en une seule fois une insuline basale à action intermédiaire avec une insuline prandiale à action rapide. Or, nombreux sont

les patients soucieux de réduire leur nombre d'injections.

Les insulines basales actuellement commercialisées et actuellement en développement clinique peuvent être classées en fonction de la solution technique qui permet d'obtenir l'action prolongée et à ce jour, deux approches sont utilisées.

La première, celle de l'insuline detemir, est la liaison à l'albumine *in vivo.* Il s'agit d'un analogue, soluble à pH 7, qui comprend une chaine latérale d'acide gras (tetradecanoyl) fixée à la position B29 qui, *in vivo,* permet à cette insuline de s'associer à l'albumine. Son action prolongée est principalement due à cette affinité pour l'albumine après injection sous-cutanée.

Cependant, son profil pharmacocinétique ne permet pas de couvrir une journée, ce qui fait qu'elle est le plus souvent utilisée en deux injections par jour.

D'autres insulines basales solubles à pH 7, comme Degludec®, sont actuellement en développement. Degludec® comprend également une chaîne latérale d'acide gras fixée sur l'insuline (hexadecandioyl-γ-L-Glu).

La seconde, celle de l'insuline glargine, est la précipitation à pH physiologique. L'insuline glargine est un analogue de l'insuline humaine obtenu par élongation de la partie C-terminale de la chaine B de l'insuline humaine par deux résidus arginine, et par substitution du résidu d'asparagine A21, par un résidu de glycine (US 5,656,722). L'addition de deux résidus d'arginine a été pensée pour ajuster le pI (point isoélectrique) d'insuline glargine au pH physiologique, et ainsi rendre cet analogue de l'insuline humaine insoluble en milieu physiologique.

Aussi, la substitution de l'A21 a été pensée afin de rendre l'insuline glargine stable à pH acide et pouvoir ainsi la formuler sous forme de solution injectable à pH acide. Lors de l'injection sous-cutanée, le passage de l'insuline glargine d'un pH acide (pH 4-4,5) à un pH physiologique (pH neutre) provoque sa précipitation sous la peau. La redissolution lente des micro-particules d'insuline glargine assure une action lente et prolongée.

L'effet hypoglycémiant de l'insuline glargine est quasi-constant sur une durée de 24 heures, ce qui permet à la plupart des patients de se limiter à une seule injection par jour.

L'insuline glargine est considérée aujourd'hui comme la meilleure insuline basale commercialisée.

Cependant, le pH nécessairement acide des formulations d'insulines basales, dont le point isoélectrique est compris entre 5,8 et 8,5, de type insuline glargine, peut être un réel inconvénient, car ce pH acide de la formulation d'insuline glargine entraîne parfois chez les patients des douleurs à l'injection et surtout empêche toute formulation avec d'autres protéines et en particulier avec les insulines prandiales, car ces dernières ne sont pas stables à pH acide. L'impossibilité de formuler une insuline prandiale, à pH acide, tient au fait qu'une insuline prandiale subit, dans ces conditions, une réaction secondaire de déamidation en position A21 ; ce qui ne permet pas de répondre à l'exigence de la Pharmacopée US, à savoir moins de 5 % de produits secondaires après 4 semaines à 30°C.

Ainsi, personne n'a à ce jour cherché à solubiliser ces insulines basales, de type insuline glargine dont le point isoélectrique est compris entre 5,8 et 8,5, à pH neutre tout en maintenant une différence de solubilité entre le milieu *in-vitro* (le contenant) et le milieu *in-vivo* (sous la peau), indépendamment du pH.

De l'analyse des compositions décrites dans la littérature et les brevets, il apparait que l'insolubilité à pH 7 des insulines basales, du type insuline glargine, est un prérequis pour avoir une action lente.

En effet, le principe de fonctionnement des insulines basales de type insuline glargine, dont le point isoélectrique est compris entre 5,8 et 8,5, est qu'elles sont solubles à pH acide et précipitent à pH physiologique. Ceci détourne l'homme de l'art de toute solution dans laquelle l'insuline de type insuline glargine serait solubilisée à pH 6-8 tout en gardant sa propriété essentielle, qui est de précipiter en milieu sous-cutané.

De plus, ce pH acide des formulations des insulines basales, dont le point isoélectrique est compris entre 5,8 et 8,5, de type insuline glargine, empêche même toute combinaison extemporanée avec les insulines prandiales à pH neutre.

En effet, une étude clinique récente, présentée au 69th Scientific Sessions of the American Diabetes Association, New Orleans, Louisiana, 5-9 Juin 2009, 0019-OR, a permis de vérifier cette limitation d'usage d'insuline glargine. Une dose d'insuline glargine et une dose d'insuline prandiale (en l'occurrence, l'insuline lispro) ont été mélangées juste avant injection (E. Cengiz et al., 2010 ; Diabetes care - 33(5) : 1009-12). Cette expérience a permis de mettre en évidence un retard significatif dans les profils pharmacocinétique et pharmacodynamique de l'insuline prandiale, pouvant donner lieu à des hyperglycémies postprandiales et à des hypoglycémies nocturnes. Cette étude confirme bien l'incompatibilité de l'insuline glargine avec les insulines à action rapide actuellement commercialisées.

D'ailleurs, la notice d'utilisation de Lantus®, le produit commercial à base d'insuline glargine de la société SANOFI-AVENTIS, indique explicitement aux utilisateurs de ne pas réaliser de mélange avec une solution d'insuline prandiale, quelle qu'elle soit, en raison du risque sérieux de modifier la pharmacocinétique et la pharmacodynamie de l'insuline glargine et/ou de l'insuline prandiale mélangée.

Pourtant, d'un point de vue thérapeutique, il a été démontré, tel qu'illustré ci-après, que les traitements associant soit une insuline glargine et une insuline prandiale, soit une insuline glargine et un analogue de GLP-1, présentent un réel intérêt.

S'agissant de l'association d'une insuline glargine et d'une insuline prandiale, des études cliniques rendues

publiques lors des 70èmes séances scientifiques annuelles de *l'American Diabetes Association* (ADA) de 2010, abstract 2163-PO et abstract numéro 0001-LB, en particulier celles menées par la société SANOFI-AVENTIS, ont montré que les traitements qui associent Lantus®, insuline glargine, et une insuline prandiale sont bien plus efficaces que les traitements à base de produits du type « Premix », Novolog Mix® ou Humalog Mix®.

S'agissant de l'association d'une insuline glargine et d'un analogue de GLP-1, la FDA (Food and Drug Administration, Agence américaine du médicament) a approuvé, en octobre 2011, l'injection d'exenatide (Byetta®, Amylin Pharmaceuticals, Inc et Eli Lilly and Company) en tant que thérapie complémentaire à l'insuline glargine pour les patients atteints de diabète de type II qui ne sont pas en mesure d'atteindre un contrôle de leur glycémie avec l'analogue d'insuline basale seul.

Or, du fait que le principe même, exposé ci-dessus, des insulines basales dont le point isoélectrique est compris entre 5,8 et 8,5, est qu'elles sont solubles à pH acide et précipitent à pH physiologique, toutes les solutions proposées pour les combiner à d'autres produits comme les insulines prandiales ou des analogues ou dérivés de GLP-1 sont basées sur des essais de solubilisation des insulines prandiales ou des analogues ou dérivés de GLP-1 à pH acide, voir par exemple WO2007/121256, WO2009/021955, WO2011/144673, WO2011/147980 ou encore WO2009/063072.

Par exemple, s'agissant des combinaisons d'insuline glargine et d'insuline rapide, la société BIODEL, a décrit notamment dans la demande de brevet US 7,718,609 des compositions comprenant une insuline basale et une insuline prandiale à un pH compris entre 3,0 et 4,2 en présence d'un agent chélatant et de polyacides. Ce brevet enseigne comment rendre compatible une insuline prandiale à pH acide en présence de l'insuline glargine. Il n'enseigne pas comment préparer une combinaison d'insuline de type insuline glargine et d'une insuline prandiale à pH neutre.

Également à titre d'exemple, s'agissant de la solubilisation de l'insuline glargine à pH neutre et des combinaisons avec un analogue de GLP-1, on citera la demande de brevet WO2011/144676 publiée le 24 novembre 2011, au nom de SANOFI-AVENTIS, qui décrit des formulations à pH 9,5 d'insuline glargine avec la cyclodextrine SVE4-β-CYD dans lesquelles la solubilité de l'insuline glargine est améliorée de 0,75 mM à 1,25 mM. Cette demande mentionne également des compositions comprenant en outre un GLP-1, bien que non-exemplifiées. L'effet solubilisant à pH 7,4 dans un tampon phosphate est mentionné. Ces résultats de solubilisation à pH 7,4 sont décrits dans la publication intitulée « Effect of sulfobutyl ether-β-cyclodextrin on bioavailability of insulin glargine and blood glucose level after subcutaneous injection to rats » (International Journal of Pharmaceutics, 419 (2011), 71-76) à la figure 3A. La sulfobutyl ether-β-cyclodextrine améliore la solubilité de l'insuline glargine à pH 7,4 de 5 μM à 8 μM, ce qui ne présente pas d'intérêt thérapeutique, dans la mesure où la concentration commerciale de l'insuline glargine est de 600 μM (100 UI/mL). Le problème n'a ainsi pas été résolu de façon satisfaisante par l'invention décrite dans cette demande de brevet.

A notre connaissance, il n'a donc jamais été décrit de formulation, stable à pH physiologique, comprenant une insuline basale dont le point isoélectrique est compris entre 5,8 et 8,5 seule ou en association avec une insuline prandiale et/ou une hormone gastro-intestinale, dans laquelle la solubilité de l'insuline est suffisante pour un traitement thérapeutique.

La présente invention telle que définie dans les revendications, en résolvant ce problème de solubilité à pH compris entre 6,6 et 7,8 permet :

- de proposer une composition injectable, destinée au traitement du diabète, comprenant une insuline basale, dont le point isoélectrique est compris entre 5,8 et 8,5, sous la forme d'une solution homogène à pH compris entre 6,6 et 7,8, tout en conservant son activité biologique et son profil d'action lente,
- de proposer une composition injectable sous la forme d'une solution homogène à pH compris entre 6,6 et 7,8 comprenant en outre une combinaison d'une insuline basale, dont le point isoélectrique est compris entre 5,8 et 8,5, et d'une insuline prandiale sans modification du profil d'activité de l'insuline prandiale soluble entre pH 6 et 8 et instable à pH acide tout en maintenant le profil d'action lente propre à l'insuline basale,
- de proposer une composition injectable sous la forme d'une solution homogène à pH compris entre 6,6 et 7,8 comprenant en outre une combinaison d'une insuline basale, dont le point isoélectrique est compris entre 5,8 et 8,5, et d'un dérivé ou un analogue d'une hormone gastro intestinale comme le GLP-1 ou « glucagon like peptide-1 »,
- de diminuer le nombre d'injections dans le cadre du traitement du diabète,
- aux dites compositions d'être conformes aux exigences des Pharmacopées américaines et européennes.

De façon surprenante, les compositions selon l'invention permettent de solubiliser à un pH compris entre 6,6 et 7,8 une insuline basale dont le point isoélectrique est compris entre 5,8 et 8,5.

De façon surprenante, les compositions selon l'invention permettent un maintien de la durée de l'activité hypoglycémiante de l'insuline basale dont le point isoélectrique est compris entre 5,8 et 8,5 malgré sa solubilisation à un pH compris entre 6,6 et 7,8 avant injection. Cette propriété remarquable provient du fait que l'insuline de type insuline glargine solubilisée à un pH compris entre 6,6 et 7,8 dans la composition de l'invention précipite en milieu sous-cutané par un changement de composition du milieu. L'élément déclenchant la précipitation de l'insuline de type insuline glargine n'est plus la modification de pH, mais une modification de la composition de l'environnement lors du passage de la

composition pharmaceutique du contenant au milieu physiologique. De façon surprenante, dans les combinaisons de l'insuline de type insuline glargine avec une insuline prandiale, objets de l'invention, l'action rapide de l'insuline prandiale est préservée malgré la précipitation de l'insuline de type insuline glargine en milieu sous-cutané.

La solution selon l'invention permettant de solubiliser l'insuline basale, dont le point isoélectrique est compris entre 5,8 et 8,5, à un pH compris entre 6,6 et 7,8, préserve son activité biologique.

Dans les combinaisons de l'insuline de type insuline glargine avec une insuline prandiale, objets de l'invention, l'action rapide de l'insuline prandiale est préservée malgré la précipitation de l'insuline de type insuline glargine en milieu sous-cutané. De plus, la présence de l'insuline prandiale ne modifie pas la solubilité de l'insuline basale à un pH compris entre 6,6 et 7,8 et ne modifie également pas les propriétés de précipitation de l'insuline basale.

La divulgation concerne une composition sous forme d'une solution aqueuse injectable, dont le pH est compris entre 6,6 et 7,8, comprenant au moins :

a) une insuline basale dont le point isoélectrique pI est compris entre 5,8 et 8,5 ;
b) un polymère anionique hydrophobisé de formule I :

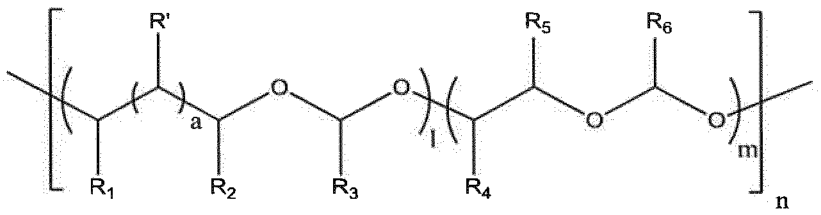

Formule I

dans laquelle,

- l = 0 ou 1,
- m = 0, 1 ou 2,
- a = 0 ou 1,
- n étant le degré de polymérisation, compris entre 3 et 1000, et,
- $-R_1$ est un hydrogène -H,
- $-R_2$, $-R_3$, $-R_4$ et $-R_6$ sont des radicaux $-CH_2R'$,
- $-R_5$ est soit un groupe -COOH, soit un radical $-CH_2R'$, soit un radical -*k*-[D], dans lequel :
    - -[D] est un radical -[Hy] ou -[E]-(*o*-[Hy])$_t$ ;
    - -[E]- est un radical au moins divalent comprenant de 1 à 15 atomes de carbone comprenant au moins un hétéroatome choisi parmi O, N et S, éventuellement porteur de fonctions carboxyle ou amine et/ou -*k*-[E]-(*o*)$_t$, comprenant de 2 à 16 atomes de carbone, est issu d'un acide aminé, d'un dialcool, d'une diamine, ou d'un amine-alcool ;
    - -[Hy] est un groupe alkyle, linéaire ou cyclique, un alkylaryle ou un arylalkyle, en C8 à C30, éventuellement substitué par un ou plusieurs groupes alkyles en C1 à C3, issu d'un composé hydrophobe ;
    - *k* résultant de la réaction entre une fonction carboxyle, amine ou alcool du précurseur de -*k*-[E]-(*o*)$_t$ et une fonction alcool, carboxyle ou amine du polymère et est une fonction choisie dans le groupe constitué par les fonctions ester, amide, carbonate et carbamate;
    - *o* résultant de la réaction entre une fonction carboxyle, amine ou alcool du précurseur de -*k*-[E]-(*o*)$_t$ et une fonction alcool ou acide du précurseur de -[Hy] est une fonction choisie dans le groupe constitué par les fonctions ester, amide, urée (carbamide), carbonate et carbamate ;
    - t est un entier positif égal à 1 ou 2 ;

  et/ou,
- $-R_1$ et $-R_3$ forment un cycle à six chaînons $-R_1-R_3- = -CH(NHCOCH_3)-$ et $-R_2$ est un radical $-CH_2R'$,
  et/ou,
- $-R_2$ et $-R_3$ forment un cycle à six chaînons et $-R_2-R_3- = -(CH(R'))_3-$ et $-R_1$ est un hydrogène,
  et/ou,
- $-R_4$ et $-R_6$ forment un cycle à six chaînons et $-R_4-R_6- = -(CH(R'))_2-$,
  et,.
- -R' est choisi dans le groupe constitué par les radicaux :

- -OH
- -O-Alk, Alk étant une chaine alkyle en C1 à C3,
- -(*f*-[A]-COOH), dans lequel :

  - -[A]- est un radical au moins divalent comprenant de 1 à 15 atomes de carbones comprenant au moins un hétéroatome choisi parmi O, N et S, éventuellement porteur de fonctions carboxyle ou amine et/ou -*f*-[A]-COOH, comprenant de 2 à 16 atomes de carbone, est issu d'un acide aminé, d'un diacide ou d'un alcoolacide et est lié au squelette de la molécule par une fonction *f* ;
  - *f* résultant de la réaction entre une fonction carboxyle ou alcool ou amine du précurseur de -*f*-[A]-COOH et une fonction hydroxyle du squelette est choisie dans le groupe constitué par les fonctions éther, ester, carbamate ou carbonate ;

- -*g*-[B]-(*k*-[D])$_p$, dans lequel :

  - -[B]- est un radical au moins divalent comprenant de 1 à 15 atomes de carbone comprenant au moins un hétéroatome choisi parmi O, N et S, éventuellement porteur de fonctions carboxyle ou amine et/ou -*g*-[B]-(k-)$_p$, comprenant de 2 à 16 atomes de carbone, est issu d'un acide aminé, d'un diacide, d'un dialcool, d'un alcoolacide, d'une diamine ou d'un amine-alcool et est lié au squelette de la molécule par une fonction g et est lié à au moins un radical -[D] par une fonction k, - *g* résultant de la réaction entre une fonction carboxyle ou alcool ou amine du précurseur de -*g*-[B]-(k-)$_p$ et une fonction du squelette est choisie dans le groupe constitué par les fonctions éther, amine, ester, carbamate ou carbonate,
  - *k* résultant de la réaction entre une fonction carboxyle ou alcool ou amine du précurseur de -*g*-[B]-(k-)$_p$ et une fonction alcool ou acide du précurseur de -[D] choisie dans le groupe constitué par les fonctions ester, amide ou carbamate ;
  - p est un entier positif égal à 1 ou 2;

et, -[A]-, -[B]- et -[E]- sont identiques ou différents,
et *k* et o sont identiques ou différentes ;
et, si -[B]- est un radical trivalent, alors -[D] est un radical -[Hy],
et, si m et a = 0 alors -$R_2$ = -$CH_2R'$,
et, le degré de substitution en charges carboxylates est le nombre moyen de charges carboxylates par monomère divisé par (l+m) et est supérieur ou égal à 0,4,
et, le degré de substitution en radicaux hydrophobes est le nombre moyen de radicaux hydrophobes par monomère divisé par (l+m) et est inférieur ou égal à 0,5,
et, si le polymère anionique hydrophobisé est un polysaccharide, alors les liaisons glycosidiques identiques ou différentes, peuvent être de type $\alpha$ et/ou de type $\beta$.

Les polymères anioniques hydrophobisés sont choisis parmi les polymères de formule I parmi lesquels les atomes de carbones asymétriques sont de configuration absolue R ou S .

Ils sont également choisis parmi les polymères dont les fonctions acides libres sont sous forme de sels de cations alcalins choisis dans le groupe constitué par $Na^+$ et $K^+$.

On entend par polymère hydrophobisé un polymère porteur d'un radical ou groupe hydrophobe.

On entend par radical ou groupe « hydrophobe », un radical ou un groupe issu d'un composé hydrophobe.

On entend par composé hydrophobe, un composé présentant un LogP supérieur ou égal à 2. Le LogP ou *Log Kow* ou coefficient de partition est une mesure de répartition d'un composé dans un mélange de solvant non miscible n-octanol/ eau . Le LogP peut être mesuré selon la méthode du flacon agité ou shake flask, ou lorsque ce n'est pas possible par méthode HPLC (OECD Guideline for the testing of chemicals, 117, 30.03.89, Partition coefficient (n-octanol/water : HPLC method and 107, 27.07.95, Partition coefficient (n-octanol/water) : Shake Flask Method). Ledit LogP d'un composé étant défini par l'équation :

$$\log P = \log(c_{oct}/c_{eau})$$

dans laquelle $c_{oct}$ est la concentration dudit composé dans le n-octanol et $c_{eau}$ est la concentration dudit composé dans l'eau.

Dans un mode de réalisation, la composition selon l'invention est caractérisée en ce que le polymère anionique hydrophobisé est choisi parmi les polymères anioniques hydrophobisés de formule I, dans laquelle lorsque -$R_1$ et -$R_3$ forment un cycle à six chaînons -$R_1$-$R_3$- = -CH($NHCOCH_3$)- et -$R_2$ est un radical -$CH_2R'$, alors -$R_4$ et -$R_6$ ne forment

pas un cycle à six chaînons.

Dans un mode de réalisation, la composition selon l'invention est caractérisée en ce que le polymère anionique hydrophobisé est choisi parmi les polymères anioniques hydrophobisés de formule I, dans laquelle $-R_1$ et $-R_3$ ne forment pas un cycle à six chaînons avec $-R_1-R_3- = -CH(NHCOCH_3)-$ et $-R_2$ est un radical $-CH_2R'$

Dans un mode de réalisation, la composition selon l'invention est caractérisée en ce que le polymère anionique hydrophobisé est choisi parmi les composés de formule I, dans laquelle le radical-*f*-[A]-COOH, comprenant de 2 à 8 atomes de carbone, est issu d'un acide aminé, d'un dialcool, d'une diamine, d'un diacide ou d'un amine-alcool.

Dans un mode de réalisation, la composition selon l'invention est caractérisée en ce que le polymère anionique hydrophobisé est choisi parmi les composés de formule I, dans laquelle le radical-*f*-[A]-COOH, comprenant de 2 à 6 atomes de carbone, est issu d'un acide aminé, d'un dialcool, d'une diamine, d'un diacide ou d'un amine-alcool.

Dans un mode de réalisation, la composition selon l'invention est caractérisée en ce que le polymère anionique hydrophobisé est choisi parmi les polymères anioniques hydrophobisés de formule I, dans laquelle le radical -*f*-[A]-COOH est choisi parmi les radicaux de formule II suivante :

-*f*-[A]-COOH =

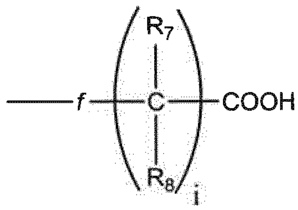

Formule II

dans laquelle :

- i est supérieur ou égal à 1 et inférieur ou égal à 12, et,
- $-R_7$ et $-R_8$, identiques ou différents, sont choisis dans le groupe constitué par un atome d'hydrogène, un alkyle saturé ou insaturé, linéaire, ramifié ou cyclique en C1 à C6, un benzyle, un alkyle-aryle et comportant éventuellement des hétéroatomes choisis dans le groupe constitué par O, N et/ou S, ou des fonctions choisies dans le groupe constitué par les fonctions acide carboxylique, amine, alcool ou thiol.

Dans un mode de réalisation, la composition selon l'invention est caractérisée en ce que le polymère anionique hydrophobisé est choisi parmi les polymères anioniques hydrophobisés de formule I, dans laquelle le radical -*f*-[A]-COOH est choisi dans le groupe constitué par les radicaux suivants, *f* ayant la signification donnée ci-dessus :

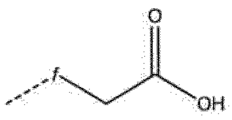
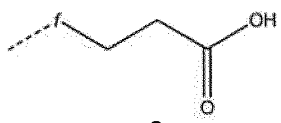
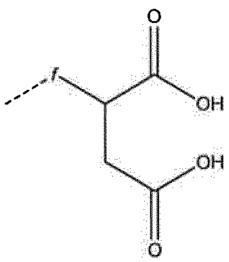
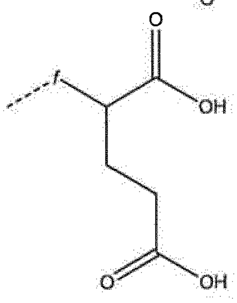

ou leur sels de cations alcalins choisis dans le groupe constitué de $Na^+$ et $K^+$.

Dans un mode de réalisation, la composition selon l'invention est caractérisée en ce que le polymère anionique hydrophobisé est choisi parmi les polymères anioniques hydrophobisés de formule I, dans laquelle le radical -[A]- est un radical-$CH_2$-.

Dans un mode de réalisation, la composition selon l'invention est caractérisée en ce que le polymère anionique

hydrophobisé est choisi parmi les polymères anioniques hydrophobisés de formule I, dans laquelle le radical -*f*-[A]-COOH est issu d'un acide aminé.

Dans un mode de réalisation, la composition selon l'invention est caractérisée en ce que le polymère anionique hydrophobisé est choisi parmi les polymères anioniques hydrophobisés de formule I, dans laquelle le radical -*f*-[A]-COOH est issu de la glycine.

Dans un mode de réalisation, la composition selon l'invention est caractérisée en ce que le polymère anionique hydrophobisé est choisi parmi les polymères anioniques hydrophobisés de formule I, dans laquelle le radical -*f*-[A]-COOH est issu de l'acide aspartique.

Dans un mode de réalisation, la composition selon l'invention est caractérisée en ce que le polymère anionique hydrophobisé est choisi parmi les polymères anioniques hydrophobisés de formule I, dans laquelle le radical -*f*-[A]-COOH est issu de l'acide glutamique.

Dans un mode de réalisation, la composition selon l'invention est caractérisée en ce que le polymère anionique hydrophobisé est choisi parmi les polymères anioniques hydrophobisés de formule I, dans laquelle le radical -*f*-[A]-COOH est issu de l'acide succinique.

Dans un mode de réalisation, la composition selon l'invention est caractérisée en ce que le polymère anionique hydrophobisé est choisi parmi les polymères anioniques hydrophobisés de formule I, dans laquelle la fonction *f* est une fonction éther.

Dans un mode de réalisation, la composition selon l'invention est caractérisée en ce que le polymère anionique hydrophobisé est choisi parmi les polymères anioniques hydrophobisés de formule I, dans laquelle la fonction *f* est une fonction carbamate.

Dans un mode de réalisation, la composition selon l'invention est caractérisée en ce que le polymère anionique hydrophobisé est choisi parmi les polymères anioniques hydrophobisés de formule I, dans laquelle la fonction *f* est une fonction ester.

Dans un mode de réalisation, la composition selon l'invention est caractérisée en ce que le polymère anionique hydrophobisé est choisi parmi les polymères anioniques hydrophobisés de formule I, dans laquelle la fonction *f* est une fonction carbonate.

Dans un mode de réalisation, la composition selon l'invention est caractérisée en ce que le polymère anionique hydrophobisé est choisi parmi les polymères anioniques hydrophobisés de formule I, dans laquelle la fonction *f* est une fonction amide.

Dans un mode de réalisation, la composition selon l'invention est caractérisée en ce que le polymère anionique hydrophobisé est choisi parmi les polymères anioniques hydrophobisés de formule I, dans laquelle le radical -g[B]- est choisi parmi les radicaux de formule III suivante :

-*g*-[B]- = 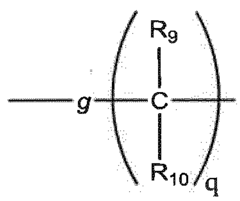

Formule III

dans laquelle :

- q est supérieur ou égal à 1 et inférieur ou égal à 12, et,
- $-R_9$ et $-R_{10}$ identiques ou différents sont choisis dans le groupe constitué par un atome d'hydrogène, un alkyle saturé ou insaturé, linéaire, ramifié ou cyclique en C1 à C6, un benzyle, un alkyle-aryle et comportant éventuellement des hétéroatomes choisis dans le groupe constitué par O, N et/ou S, ou des fonctions choisies dans le groupe constitué par les fonctions acide carboxylique, amine, alcool ou thiol.

Dans un mode de réalisation, la composition selon l'invention est caractérisée en ce que le polymère anionique hydrophobisé est choisi parmi les polymères anioniques hydrophobisés de formule I, dans laquelle le radical -*g*-[B]-*k*-[D] est choisi dans le groupe constitué par les radicaux suivants :

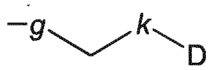 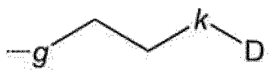

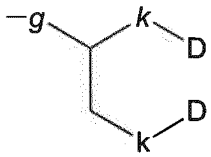 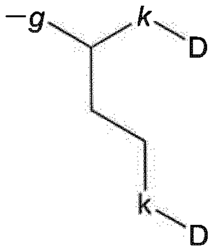

- *g, k* et -[D] ayant les significations données ci-dessus.

Dans un mode de réalisation, la composition selon l'invention est caractérisée en ce que le polymère anionique hydrophobisé est choisi parmi les polymères anioniques hydrophobisés de formule I, dans laquelle le radical -[B]- est un radical $-CH_2-$.

Dans un mode de réalisation, la composition selon l'invention est caractérisée en ce que le polymère anionique hydrophobisé est choisi parmi les polymères anioniques hydrophobisés de formule I, dans laquelle le radical -*g*-[B]-(*k*-[D])$_p$ est tel que -*g*-[B]-*k*- est issu d'un acide aminé.

Dans un mode de réalisation, la composition selon l'invention est caractérisée en ce que, le polymère anionique hydrophobisé est choisi parmi les polymères anioniques hydrophobisés de formule I, dans laquelle le radical -*g*-[B]-(*k*-[D])$_p$ est tel que -*g*-[B]-*k*- est issu de la glycine.

Dans un mode de réalisation, la composition selon l'invention est caractérisée en ce que le polymère anionique hydrophobisé est choisi parmi les polymères anioniques hydrophobisés de formule I, dans laquelle le radical -*g*-[B]-(*k*-[D])$_p$ est tel que -*g*-[B]-*k*- est issu de l'acide aspartique.

Dans un mode de réalisation, la composition selon l'invention est caractérisée en ce que le polymère anionique hydrophobisé est choisi parmi les polymères anioniques hydrophobisés de formule I, dans laquelle le radical -*g*-[B]-(*k*-[D])$_p$ est tel que -*g*-[B]-*k*- est issu de l'acide glutamique.

Dans un mode de réalisation, la composition selon l'invention est caractérisée en ce que le polymère anionique hydrophobisé est choisi parmi les polymères anioniques hydrophobisés de formule I, dans laquelle le radical -*g*-[B]-(*k*-[D])$_p$ est tel que -*g*-[B]-*k*- est issu de l'acide succinique.

Dans un mode de réalisation, la composition selon l'invention est caractérisée en ce que le polymère anionique hydrophobisé est choisi parmi les polymères anioniques hydrophobisés de formule I, dans laquelle la fonction g est une fonction éther.

Dans un mode de réalisation, la composition selon l'invention est caractérisée en ce que le polymère anionique hydrophobisé est choisi parmi les polymères anioniques hydrophobisés de formule I, dans laquelle la fonction *g* est une fonction carbamate.

Dans un mode de réalisation, la composition selon l'invention est caractérisée en ce que le polymère anionique hydrophobisé est choisi parmi les polymères anioniques hydrophobisés de formule I, dans laquelle la fonction *g* est une fonction ester.

Dans un mode de réalisation, la composition selon l'invention est caractérisée en ce que le polymère anionique hydrophobisé est choisi parmi les polymères anioniques hydrophobisés de formule I, dans laquelle la fonction *g* est une fonction amine.

Dans un mode de réalisation, la composition selon l'invention est caractérisée en ce que le polymère anionique hydrophobisé est choisi parmi les polymères anioniques hydrophobisés de formule I, dans laquelle la fonction *g* est une fonction carbonate.

Dans un mode de réalisation, la composition selon l'invention est caractérisée en ce que le polymère anionique hydrophobisé est choisi parmi les polymères anioniques hydrophobisés de formule I, dans laquelle la fonction *k* est une fonction amide.

Dans un mode de réalisation, la composition selon l'invention est caractérisée en ce que le polymère anionique hydrophobisé est choisi parmi les polymères anioniques hydrophobisés de formule I, dans laquelle la fonction *k* est une fonction carbamate.

Dans un mode de réalisation, la composition selon l'invention est caractérisée en ce que le polymère anionique hydrophobisé est choisi parmi les polymères anioniques hydrophobisés de formule I, dans laquelle la fonction *k* est une fonction ester.

Dans un mode de réalisation, la composition selon l'invention est caractérisée en ce que le polymère anionique hydrophobisé est choisi parmi les polymères anioniques hydrophobisés de formule I, dans laquelle le radical -*k*-[E]-(*o*)$_t$ est un radical au moins divalent issu d'un acide aminé.

Dans un mode de réalisation, la composition selon l'invention est caractérisée en ce que le polymère anionique hydrophobisé est choisi parmi les polymères anioniques hydrophobisés de formule I, dans laquelle le radical -*k*-[E]-(*o*)$_t$

est un radical au moins divalent issu d'un acide alpha aminé.

Dans un mode de réalisation, la composition selon l'invention est caractérisée en ce que le polymère anionique hydrophobisé est choisi parmi les polymères anioniques hydrophobisés de formule I, dans laquelle le radical -*k*-[E]-($o$)$_t$ est un radical au moins divalent issu d'un acide alpha aminé naturel choisi dans le groupe constitué par la glycine, la leucine, la phénylalanine, la lysine, l'isoleucine, l'alanine, la valine, l'acide aspartique et l'acide glutamique, sous leurs formes L, D ou racémique.

Dans un mode de réalisation, la composition selon l'invention est caractérisée en ce que le polymère anionique hydrophobisé est choisi parmi les polymères anioniques hydrophobisés de formule I, dans laquelle le radical -*k*-[E]-($o$)$_t$ est un radical au moins divalent issu d'un mono- ou polyéthylène glycol.

Dans un mode de réalisation, la composition selon l'invention est caractérisée en ce que le polymère anionique hydrophobisé est choisi parmi les polymères anioniques hydrophobisés de formule I, dans laquelle le radical -*k*-[E]-($o$)$_t$ est un radical au moins divalent issu de l'éthylène glycol.

Dans un mode de réalisation, la composition selon l'invention est caractérisée en ce que le polymère anionique hydrophobisé est choisi parmi les polymères anioniques hydrophobisés de formule I, dans laquelle le radical -*k*-[E]-($o$)$_t$ est un radical au moins divalent issu d'un polyéthylène glycol choisi dans le groupe constitué par le diéthylène glycol, le triéthylène glycol et le tétraéthylène glycol.

Dans un mode de réalisation, la composition selon l'invention est caractérisée en ce que, le polymère anionique hydrophobisé est choisi parmi les polymères anioniques hydrophobisés de formule I, dans laquelle le radical -*k*-[E]-($o$)$_t$ est un radical au moins divalent issu d'un mono- ou polyéthylène glycol amine.

Dans un mode de réalisation, la composition selon l'invention est caractérisée en ce que le polymère anionique hydrophobisé est choisi parmi les polymères anioniques hydrophobisés de formule I, dans laquelle le radical -*k*-[E]-($o$)$_t$ est un radical au moins divalent issu d'un mono- ou polyéthylène glycol amine choisi dans le groupe constitué par l'éthanolamine, le diéthylène glycol amine et le triéthylène glycol amine.

Dans un mode de réalisation, la composition selon l'invention est caractérisée en ce que le polymère anionique hydrophobisé est choisi parmi les polymères anioniques hydrophobisés de formule I, dans laquelle le radical -*k*-[E]-($o$)$_t$ est un radical au moins divalent issu d'un mono- ou polyéthylène glycol diamine.

Dans un mode de réalisation, la composition selon l'invention est caractérisée en ce que le polymère anionique hydrophobisé est choisi parmi les polymères anioniques hydrophobisés de formule I, dans laquelle le radical -*k*-[E]-($o$)$_t$ est un radical au moins divalent issu d'un mono- ou polyéthylène glycol diamine choisi dans le groupe constitué par le diéthylène glycol diamine et le triéthylène glycol diamine.

Dans un mode de réalisation, la composition selon l'invention est caractérisée en ce que le polymère anionique hydrophobisé est choisi parmi les polymères anioniques hydrophobisés de formule I, dans laquelle le radical -*k*-[E]-($o$)$_t$ est un radical au moins divalent issu de l'éthylènediamine.

Dans un mode de réalisation, la composition selon l'invention est caractérisée en ce que le polymère anionique hydrophobisé est choisi parmi les polymères anioniques hydrophobisés de formule I, dans laquelle la fonction *o* est une fonction ester.

Dans un mode de réalisation, la composition selon l'invention est caractérisée en ce que le polymère anionique hydrophobisé est choisi parmi les polymères anioniques hydrophobisés de formule I, dans laquelle la fonction *o* est une fonction amide.

Dans un mode de réalisation, la composition selon l'invention est caractérisée en ce que le polymère anionique hydrophobisé est choisi parmi les polymères anioniques hydrophobisés de formule I, dans laquelle la fonction o est une fonction carbamate.

Dans un mode de réalisation, la composition selon l'invention est caractérisée en ce que, le polymère anionique hydrophobisé est choisi parmi les polymères anioniques hydrophobisés de formule I, dans laquelle la fonction o est une fonction carbonate.

Dans un mode de réalisation, la composition selon l'invention est caractérisée en ce que, le polymère anionique hydrophobisé est choisi parmi les polymères anioniques hydrophobisés de formule I, dans laquelle la fonction o est une fonction carbamide.

Dans un mode de réalisation, la composition selon l'invention est caractérisée en ce que le polymère anionique hydrophobisé est choisi parmi les polymères anioniques hydrophobisés de formule I, dans laquelle le groupe -[Hy] est un groupe alkyle issu d'un alcool hydrophobe, insaturé et/ou saturé, ramifié ou non ramifié, comprenant de 8 à 30 atomes carbones.

Dans un mode de réalisation, la composition selon l'invention est caractérisée en ce que le polymère anionique hydrophobisé est choisi parmi les polymères anioniques hydrophobisés de formule I, dans laquelle le groupe -[Hy] est un groupe alkyle issu d'un alcool hydrophobe choisi dans le groupe constitué par l'octanol, le décanol, le dodécanol (alcool laurylique), le tétradécanol (alcool myristilique), l'hexadécanol (alcool cétylique), l'alcool stéarylique, l'alcool cétéarylique et l'alcool oléylique.

Dans un mode de réalisation, la composition selon l'invention est caractérisée en ce que le polymère anionique

hydrophobisé est choisi parmi les polymères anioniques hydrophobisés de formule I, dans laquelle le groupe -[Hy] est un groupe issu d'un stérol.

Dans un mode de réalisation, la composition selon l'invention est caractérisée en ce que le polymère anionique hydrophobisé est choisi parmi les polymères anioniques hydrophobisés de formule I, dans laquelle le groupe -[Hy] est un groupe issu d'un stérol, choisi dans le groupe constitué par le cholestérol et ses dérivés.

Dans un mode de réalisation, la composition selon l'invention est caractérisée en ce que le polymère anionique hydrophobisé est choisi parmi les polymères anioniques hydrophobisés de formule I, dans laquelle le groupe -[Hy] est un groupe issu du cholestérol.

Dans un mode de réalisation, la composition selon l'invention est caractérisée en ce que le polymère anionique hydrophobisé est choisi parmi les polymères anioniques hydrophobisés de formule I, dans laquelle le groupe -[Hy] est un groupe issu d'un tocophérol.

Dans un mode de réalisation, la composition selon l'invention est caractérisée en ce que le polymère anionique hydrophobisé est choisi parmi les polymères anioniques hydrophobisés de formule I, dans laquelle le groupe -[Hy] est un groupe issu d'un dérivé du tocophérol, choisi parmi le racémique, l'isomère L ou l'isomère D de l'α-tocophérol.

Dans un mode de réalisation, la composition selon l'invention est caractérisée en ce que le polymère anionique hydrophobisé est choisi parmi les polymères anioniques hydrophobisés de formule I, dans laquelle le groupe -[Hy] est un groupe issu du DL-α-tocophérol.

Dans un mode de réalisation, la composition selon l'invention est caractérisée en ce que le polymère anionique hydrophobisé est choisi parmi les polymères anioniques hydrophobisés de formule I, dans laquelle le groupe -[Hy] est un groupe issu du menthol.

Dans un mode de réalisation, la composition selon l'invention est caractérisée en ce que le polymère anionique hydrophobisé est choisi parmi les polymères anioniques hydrophobisés de formule I, dans laquelle le groupe -[Hy] est un groupe issu du menthol ou de ses dérivés, choisi parmi le racémique, l'isomère L ou l'isomère D du menthol.

Dans un mode de réalisation, la composition selon l'invention est caractérisée en ce que le polymère anionique hydrophobisé est choisi parmi les polymères anioniques hydrophobisés de formule I, dans laquelle le groupe -[Hy] est un groupe alkyle issu d'un acide hydrophobe.

Dans un mode de réalisation, la composition selon l'invention est caractérisée en ce que le polymère anionique hydrophobisé est choisi parmi les polymères anioniques hydrophobisés de formule I, dans laquelle le groupe -[Hy] est un groupe alkyle issu d'un acide hydrophobe linéaire, choisi dans le groupe constitué par l'acide dodécanoïque, l'acide tétradécanoïque et l'acide hexadécanoïque.

Dans un mode de réalisation, la composition selon l'invention est caractérisée en ce que le polymère anionique hydrophobisé est choisi parmi les polymères anioniques hydrophobisés de formule I, dans laquelle le groupe -[Hy] est un groupe alkyle issu d'un acide gras.

Dans un mode de réalisation, la composition selon l'invention est caractérisée en ce que le polymère anionique hydrophobisé est choisi parmi les polymères anioniques hydrophobisés de formule I, dans laquelle le groupe -[Hy] est un groupe alkyle issu d'un acide gras choisi dans le groupe constitué par les acides constitués d'une chaîne alkyle insaturée ou saturée, ramifiée ou non ramifiée, comprenant de 8 à 30 carbones.

Dans un mode de réalisation, la composition selon l'invention est caractérisée en ce que le polymère anionique hydrophobisé est choisi parmi les polymères anioniques hydrophobisés de formule I, dans laquelle le groupe -[Hy] est un groupe alkyle issu d'un acide gras choisi dans le groupe constitué par les acides gras linéaires.

Dans un mode de réalisation, la composition selon l'invention est caractérisée en ce que le polymère anionique hydrophobisé est choisi parmi les polymères anioniques hydrophobisés de formule I, dans laquelle le groupe -[Hy] est un groupe alkyle issu d'un acide gras linéaire saturé choisi dans le groupe constitué par l'acide caprylique (acide octanoïque), l'acide nonanoïque, l'acide caprique (acide décanoïque), l'acide undécanoïque, l'acide laurique (acide dodécanoïque), l'acide myristique (tétradécanoïque), l'acide palmitique (hexadécanoïque), l'acide stéarique (octadécanoïque), l'acide arachidique (eicosanoïque), l'acide béhénique (docosanoïque), l'acide tricosanoïque, l'acide lignocérique (tétracosanoïque), l'acide heptacosanoïque, l'acide octacosanoïque et l'acide mélissique (tricontanoïque).

Dans un mode de réalisation, la composition selon l'invention est caractérisée en ce que le polymère anionique hydrophobisé est choisi parmi les polymères anioniques hydrophobisés de formule I, dans laquelle le groupe -[Hy] est un groupe alkyle issu d'un acide gras insaturé.

Dans un mode de réalisation, la composition selon l'invention est caractérisée en ce que le polymère anionique hydrophobisé est choisi parmi les polymères anioniques hydrophobisés de formule I, dans laquelle le groupe -[Hy] est un groupe alkyle issu d'un acide gras insaturé choisi dans le groupe constitué par l'acide myristoléique ((Z)-tétradéc-9-énoïque), l'acide palmitoléique ((Z)-hexadéc-9-énoïque), l'acide oléique ((Z)-octadéc-9-énoïque), l'acide élaidique ((E)-octadéc-9-énoïque), l'acide linoléique ((9*Z*,12*Z*)-octadéca-9,12-diénoïque), l'acide alpha-linoléique ((9Z,12Z,15Z)-octadéca-9,12,15-triénoïque), l'acide arachidonique ((5Z,8Z,11Z,14Z)-octadéca-5,8,11,14-tétraénoïque), l'acide eicosapentaenoïque ((5Z,8Z,11Z,14Z,17Z)-eicosa-5,8,11,14,17-pentaénoïque), l'acide erucique (13-docoénoïque) et l'acide docosahexaenoïque ((4Z,7Z,10Z,13Z,16Z,19Z)-docosa-4,7,10,13,16,19-hexaénoïque) .

Dans un mode de réalisation, la composition selon l'invention est caractérisée en ce que le polymère anionique hydrophobisé est choisi parmi les polymères anioniques hydrophobisés de formule I, dans laquelle le groupe -[Hy] est un groupe alkyle issu d'un acide de la bile et ses dérivés.

Dans un mode de réalisation, la composition selon l'invention est caractérisée en ce que le polymère anionique hydrophobisé est choisi parmi les polymères anioniques hydrophobisés de formule I, dans laquelle le groupe -[Hy] est un groupe alkyle issu d'un acide de la bile et ses dérivés choisi dans le groupe constitué par l'acide cholique, l'acide déhydrocholique, l'acide désoxycholique et l'acide chénodésoxycholique.

Selon un mode de réalisation, la composition selon l'invention est caractérisée en ce que le polymère anionique hydrophobisé est choisi parmi les polymères anioniques hydrophobisés de formule I présentant une masse molaire moyenne en poids allant de 2 à 40 kg/mol.

Selon un mode de réalisation, la composition selon l'invention est caractérisée en ce que le polymère anionique hydrophobisé est choisi parmi les polymères anioniques hydrophobisés de formule I présentant une masse molaire moyenne en poids allant de 2 à 20 kg/mol.

Selon un mode de réalisation, la composition selon l'invention est caractérisée en ce que le polymère anionique hydrophobisé est choisi parmi les polymères anioniques hydrophobisés de formule I présentant une masse molaire moyenne en poids allant de 2 à 12 kg/mol.

Dans un mode de réalisation, la composition selon l'invention est caractérisée en ce que le polymère anionique hydrophobisé est choisi parmi les polymères anioniques hydrophobisés de formule I, dans laquelle $-R_2$, $-R_4$ et $-R_6$ sont des radicaux-$CH_2R'$.

Dans un mode de réalisation, la composition selon l'invention est caractérisée en ce que le polymère anionique hydrophobisé de formule I, dans laquelle $-R_2$, $-R_4$ et $-R_6$ sont des radicaux $-CH_2R'$ est choisi parmi les polymères anioniques hydrophobisés de formule XII :

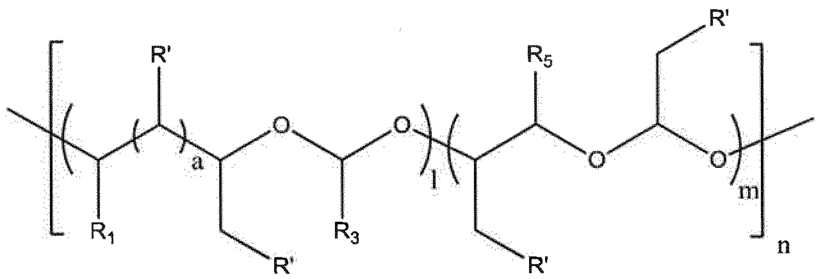

Formule XII

dans laquelle,

- l = 0 ou 1,
- m = 0, 1 ou 2,
- a = 0 ou 1,
- n est le degré de polymérisation, compris entre 3 et 1000,
  et
- $-R_1$ est un hydrogène -H,
- $-R_3$ est un radical $-CH_2R'$,
- $-R_5$ est soit un groupe -COOH, soit un radical $-CH_2R'$, soit un radical -*k*-[D],
  ou
- $-R_1$ et $-R_3$ forment un cycle à six chaînons $-R_1$-$R_3$- = $-CH(NHCOCH_3)-$,
  et,
- -R', n et -*k*-[D] tels que définis ci-dessus.

et, le degré de substitution en charges carboxylates est le nombre moyen de charges carboxylates par monomère divisé par (l+m) et est supérieur ou égal à 0,4, et, le degré de substitution en radicaux hydrophobes est le nombre moyen de radicaux hydrophobes par monomère divisé par (l+m) et est inférieur ou égal à 0,5.

Dans un mode de réalisation, la composition selon l'invention est caractérisée en ce que le polymère anionique hydrophobisé est choisi parmi les polymères anioniques hydrophobisés de formule XII, dans laquelle, l = 0 et m = 1, autrement exprimé, il est choisi parmi les polymères anioniques hydrophobisés de formule IV,

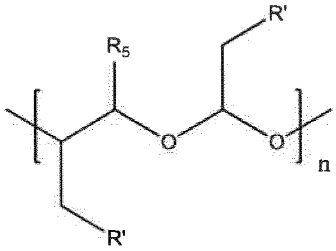

Formule IV

dans laquelle, $-R_5$ est soit un groupe -COOH, soit un radical $-CH_2R'$, soit un radical -*k*-[D], -R' et n tels que définis ci-dessus.

Dans un mode de réalisation, la composition selon l'invention est caractérisée en ce que le polymère anionique hydrophobisé est choisi parmi les polymères anioniques hydrophobisés de formule IV :

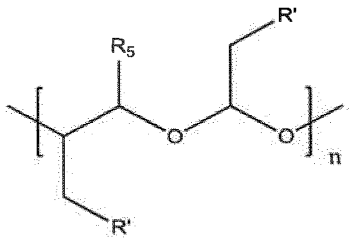

Formule IV

dans laquelle, $-R_5$ est soit un groupe -COOH, soit un radical -*k*-[D], -R' et n tels que définis ci-dessus.

Dans un mode de réalisation, la composition selon l'invention est caractérisée en ce que le polymère anionique hydrophobisé est choisi parmi les polymères anioniques hydrophobisés de formule IV :

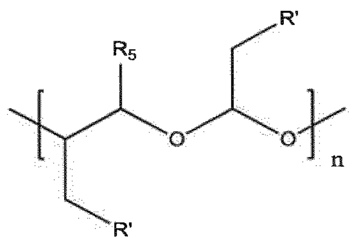

Formule IV

dans laquelle, $-R_5$ est un radical $-CH_2R'$, -R' et n tels que définis ci-dessus.

Dans un mode de réalisation, la composition selon l'invention est caractérisée en ce que le polymère anionique hydrophobisé est choisi parmi les polymères anioniques hydrophobisés de formule XII, dans laquelle, m = 0, l = 1, a = 0, autrement exprimé, il est choisi parmi les polymères anioniques hydrophobisés de formule V :

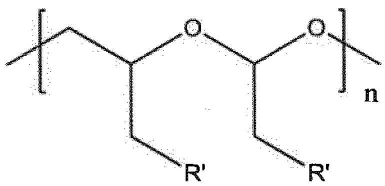

Formule V

et, -R' et n tels que définis ci-dessus.

Dans un mode de réalisation, la composition selon l'invention est caractérisée en ce que, le polymère anionique hydrophobisé est choisi parmi les polymères anioniques hydrophobisés de formule XII, dans laquelle, l = 1, m = 2 et a

= 0, autrement exprimé, il est choisi parmi les polymères anioniques hydrophobisés de formule VI :

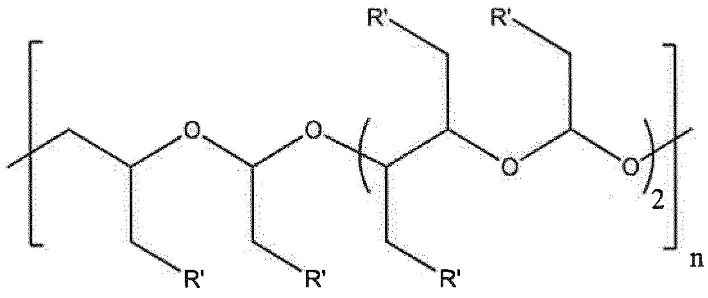

Formule VI

et, -R' et n tels que définis ci-dessus.

Dans un mode de réalisation, la composition selon l'invention est caractérisée en ce que le polymère anionique hydrophobisé est choisi parmi les polymères anioniques hydrophobisés de formule XII, dans laquelle, m = 1, l = 1, a = 1, $-R_1-R_3-$ = $-CH(NHCOCH_3)-$, $-R_2$ = $-CH_2R'$, $-R_4$ = $-CH_2R'$, $-R_5$ est soit un groupe - COOH, soit un radical -*k*-[D], $-R_6$ = $-CH_2R'$, autrement exprimé, il est choisi parmi les polymères anioniques hydrophobisés de formule VII :

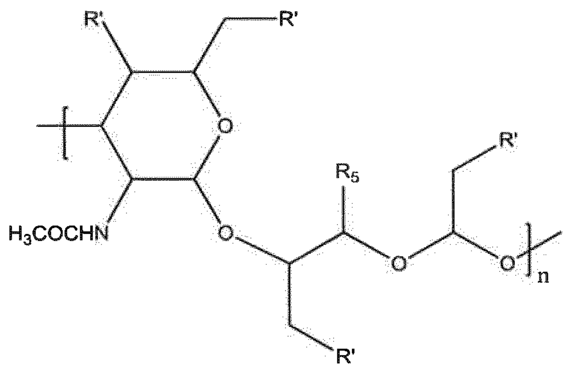

Formule VII

R' et n tels que définis ci-dessus.

Dans un mode de réalisation, la composition selon l'invention est caractérisée en ce que le polymère anionique hydrophobisé est choisi parmi les polymères anioniques hydrophobisés de formules IV à VII et XII, dans lesquelles le radical -*f*-[A]-COOH est choisi parmi les radicaux de formule II suivante :

-*f*-[A]-COOH =

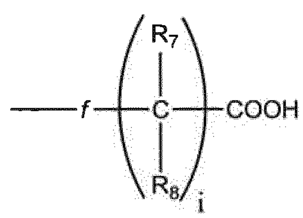

Formule II

dans laquelle :

- i supérieur ou égal à 1 et inférieur ou égal à 12, et,
- $-R_7$ et $-R_8$, identiques ou différents, sont choisis dans le groupe constitué par un atome d'hydrogène, un alkyle saturé ou insaturé, linéaire, ramifié ou cyclique en C1 à C6, un benzyle, un alkyle-aryle et comportant éventuellement des hétéroatomes choisis dans le groupe constitué par O, N et/ou S, ou des fonctions choisies dans le groupe constitué par les fonctions acide carboxylique, amine, alcool ou thiol.

Dans un mode de réalisation, la composition selon l'invention est caractérisée en ce que le polymère anionique hydrophobisé est choisi parmi les composés de formules IV à VII et XII, dans lesquelles le radical -f-[A]-COOH, comprenant de 2 à 8 atomes de carbone, est issu d'un acide aminé, d'un dialcool, d'une diamine, d'un diacide ou d'un amine-alcool.

Dans un mode de réalisation, la composition selon l'invention est caractérisée en ce que le polymère anionique hydrophobisé est choisi parmi les composés de formules IV à VII et XII, dans lesquelles le radical -f-[A]-COOH, comprenant de 2 à 6 atomes de carbone, est issu d'un acide aminé, d'un dialcool, d'une diamine, d'un diacide ou d'un amine-alcool.

Dans un mode de réalisation particulier, la composition selon l'invention est caractérisée en ce que le polymère anionique hydrophobisé est choisi parmi les polymères anioniques hydrophobisés de formules IV à VII et XII répondant aux conditions suivantes :

- lorsque -*g*-[B]-(*k*-[D])$_p$ comporte une chaine Hy et que Hy est un alkyle en C8 à C15, alors le produit entre le degré de substitution en radicaux hydrophobes et le degré moyen de polymérisation (n) est supérieur ou égal à 2.
- lorsque -*g*-[B]-(*k*-[D])$_p$ comporte une chaine Hy et que Hy est un alkyle en C16 à C20, alors le produit entre le degré de substitution en radicaux hydrophobes et le degré moyen de polymérisation (n) est supérieur ou égal à 1
- lorsque -*g*-[B]-(*k*-[D])$_p$ comporte deux chaines Hy et que Hy est un alkyle en C8 à C9, alors le produit du degré de substitution en radicaux hydrophobes et du degré moyen de polymérisation (n) est supérieur ou égal à 2,
- lorsque -*g*-[B]-(*k*-[D])$_p$ comporte deux chaines Hy et que Hy est un alkyle en C10 à C16, alors le produit du degré de substitution en radicaux hydrophobes et du degré moyen de polymérisation (n) est supérieur ou égal à 0,2.

Dans un mode de réalisation, la composition selon l'invention est caractérisée en ce que le polymère anionique hydrophobisé est choisi parmi les polymères anioniques hydrophobisés de formules IV à VII et XII, dans lesquelles le radical -*f*-[A]-COOH est choisi dans le groupe constitué par les radicaux suivants, *f* ayant la signification donnée ci-dessus :

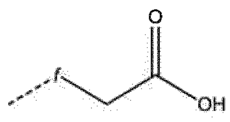
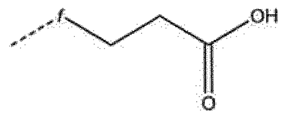
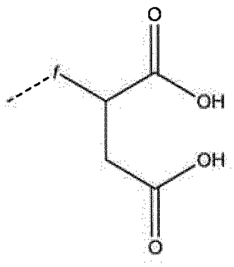
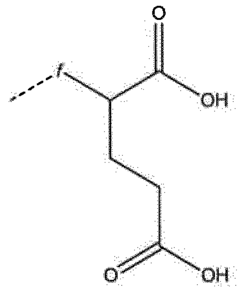

ou leur sels de cations alcalins choisis dans le groupe constitué de $Na^+$ et $K^+$,

Dans un mode de réalisation, la composition selon l'invention est caractérisée en ce que le polymère anionique hydrophobisé est choisi parmi les polymères anioniques hydrophobisés de formules IV à VII et XII, dans lesquelles le radical -[A]- est un radical $-CH_2-$.

Dans un mode de réalisation, la composition selon l'invention est caractérisée en ce que le polymère anionique hydrophobisé est choisi parmi les polymères anioniques hydrophobisés de formules IV à VII et XII, dans lesquelles le radical -*f*-[A]-COOH est issu d'un acide aminé.

Dans un mode de réalisation, la composition selon l'invention est caractérisée en ce que le polymère anionique hydrophobisé est choisi parmi les polymères anioniques hydrophobisés de formules IV à VII et XII, dans lesquelles le radical -*f*-[A]-COOH est issu de la glycine.

Dans un mode de réalisation, la composition selon l'invention est caractérisée en ce que le polymère anionique hydrophobisé est choisi parmi les polymères anioniques hydrophobisés de formules IV à VII et XII, dans lesquelles le radical -*f*-[A]-COOH est issu de l'acide aspartique.

Dans un mode de réalisation, la composition selon l'invention est caractérisée en ce que le polymère anionique hydrophobisé est choisi parmi les polymères anioniques hydrophobisés de formules IV à VII et XII, dans lesquelles le radical -*f*-[A]-COOH est issu de l'acide glutamique.

Dans un mode de réalisation, la composition selon l'invention est caractérisée en ce que le polymère anionique hydrophobisé est choisi parmi les polymères anioniques hydrophobisés de formules IV à VII et XII, dans lesquelles le radical -*f*-[A]-COOH est issu de l'acide succinique.

Dans un mode de réalisation, la composition selon l'invention est caractérisée en ce que le polymère anionique hydrophobisé est choisi parmi les polymères anioniques hydrophobisés de formules IV à VII et XII, dans lesquelles la fonction *f* est une fonction éther.

Dans un mode de réalisation, la composition selon l'invention est caractérisée en ce que le polymère anionique hydrophobisé est choisi parmi les polymères anioniques hydrophobisés de formules IV à VII et XII, dans lesquelles la fonction *f* est une fonction carbamate.

Dans un mode de réalisation, la composition selon l'invention est caractérisée en ce que le polymère anionique hydrophobisé est choisi parmi les polymères anioniques hydrophobisés de formules IV à VII et XII, dans lesquelles la fonction *f* est une fonction ester.

Dans un mode de réalisation, la composition selon l'invention est caractérisée en ce que le polymère anionique hydrophobisé est choisi parmi les polymères anioniques hydrophobisés de formules IV à VII et XII, dans lesquelles la fonction *f* est une fonction carbonate.

Dans un mode de réalisation, la composition selon l'invention est caractérisée en ce que le polymère anionique hydrophobisé est choisi parmi les polymères anioniques hydrophobisés de formules IV à VII et XII, dans lesquelles la fonction *f* est une fonction amide.

Dans un mode de réalisation, la composition selon l'invention est caractérisée en ce que le polymère anionique hydrophobisé est choisi parmi les polymères anioniques hydrophobisés de formule IV à VII et XII, dans lesquelles le radical -g-[B]- est choisi parmi les radicaux de formule III suivante :

-*g*-[B]- = 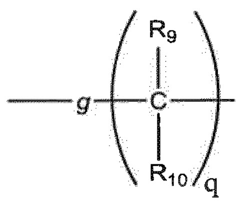

Formule III

dans laquelle :

- q supérieur ou égal à 1 et inférieur ou égal à 12, et,
- $-R_9$ et $-R_{10}$ identiques ou différents sont choisis dans le groupe constitué par un atome d'hydrogène, un alkyle saturé ou insaturé, linéaire, ramifié ou cyclique en C1 à C6, un benzyle, un alkyle-aryle et comportant éventuellement des hétéroatomes choisis dans le groupe constitué par O, N et/ou S, ou des fonctions choisies dans le groupe constitué par les fonctions acide carboxylique, amine, alcool ou thiol.

Dans un mode de réalisation, la composition selon l'invention est caractérisée en ce que le polymère anionique hydrophobisé est choisi parmi les polymères anioniques hydrophobisés de formules IV à VII, dans lesquelles le radical -*g*-[B]-*k*-[D] est choisi dans le groupe constitué par les radicaux suivants ; *g, k* et -[D] ayant les significations données ci-dessus :

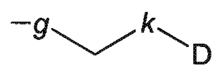 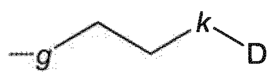

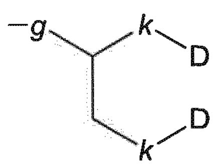 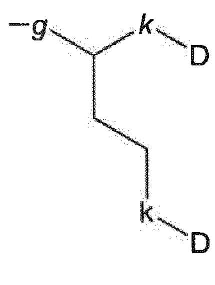

Dans un mode de réalisation, la composition selon l'invention est caractérisée en ce que le polymère anionique hydrophobisé est choisi parmi les polymères anioniques hydrophobisés de formules IV à VII et XII, dans lesquelles le radical -[B]- est un radical $-CH_2-$.

Dans un mode de réalisation, la composition selon l'invention est caractérisée en ce que le polymère anionique hydrophobisé est choisi parmi les polymères anioniques hydrophobisés de formules IV à VII et XII, dans lesquelles le radical -*g*-[B]-(*k*-[D])$_p$ est tel que -*g*-[B]-*k*- est issu d'un acide aminé.

Dans un mode de réalisation, la composition selon l'invention est caractérisée en ce que le polymère anionique hydrophobisé est choisi parmi les polymères anioniques hydrophobisés de formules IV à VII et XII, dans lesquelles le radical -*g*-[B]-(*k*-[D])$_p$ est tel que -*g*-[B]-*k*- est issu de la glycine.

Dans un mode de réalisation, la composition selon l'invention est caractérisée en ce que le polymère anionique hydrophobisé est choisi parmi les polymères anioniques hydrophobisés de formules IV à VII et XII, dans lesquelles le radical -*g*-[B]-(*k*-[D])$_p$ est tel que -*g*-[B]-*k*- est issu de l'acide aspartique.

Dans un mode de réalisation, la composition selon l'invention est caractérisée en ce que le polymère anionique hydrophobisé est choisi parmi les polymères anioniques hydrophobisés de formules IV à VII et XII, dans lesquelles le radical -*g*-[B]-(*k*-[D])$_p$ est tel que -*g*-[B]-*k*- est issu de l'acide glutamique.

Dans un mode de réalisation, la composition selon l'invention est caractérisée en ce que le polymère anionique hydrophobisé est choisi parmi les polymères anioniques hydrophobisés de formule IV à VII et XII, dans lesquelles le radical -*g*-[B]-(*k*-[D])$_p$ est tel que -*g*-[B]-*k*- est issu de l'acide succinique.

Dans un mode de réalisation, la composition selon l'invention est caractérisée en ce que le polymère anionique hydrophobisé est choisi parmi les polymères anioniques hydrophobisés de formules IV à VII et XII, dans lesquelles la fonction *g* est une fonction éther.

Dans un mode de réalisation, la composition selon l'invention est caractérisée en ce que le polymère anionique hydrophobisé est choisi parmi les polymères anioniques hydrophobisés de formules IV à VII et XII, dans lesquelles la fonction g est une fonction carbamate.

Dans un mode de réalisation, la composition selon l'invention est caractérisée en ce que le polymère anionique hydrophobisé est choisi parmi les polymères anioniques hydrophobisés de formules IV à VII et XII, dans lesquelles la fonction *g* est une fonction ester.

Dans un mode de réalisation, la composition selon l'invention est caractérisée en ce que le polymère anionique hydrophobisé est choisi parmi les polymères anioniques hydrophobisés de formules IV à VII et XII, dans lesquelles la fonction *g* est une fonction amine.

Dans un mode de réalisation, la composition selon l'invention est caractérisée en ce que le polymère anionique hydrophobisé est choisi parmi les polymères anioniques hydrophobisés de formules IV à VII et XII, dans lesquelles la fonction *g* est une fonction carbonate.

Dans un mode de réalisation, la composition selon l'invention est caractérisée en ce que le polymère anionique hydrophobisé est choisi parmi les polymères anioniques hydrophobisés de formules IV à VII et XII, dans lesquelles la fonction *k* est une fonction amide.

Dans un mode de réalisation, la composition selon l'invention est caractérisée en ce que le polymère anionique hydrophobisé est choisi parmi les polymères anioniques hydrophobisés de formules IV à VII et XII, dans lesquelles la fonction *k* est une fonction carbamate.

Dans un mode de réalisation, la composition selon l'invention est caractérisée en ce que le polymère anionique hydrophobisé est choisi parmi les polymères anioniques hydrophobisés de formules IV à VII et XII, dans lesquelles la fonction *k* est une fonction ester.

Dans un mode de réalisation, la composition selon l'invention est caractérisée en ce que le polymère anionique hydrophobisé est choisi parmi les polymères anioniques hydrophobisés de formules IV à VII et XII, dans lesquelles le radical -*k*-[E]-(*o*)$_t$ est un radical au moins divalent issu d'un acide aminé.

Dans un mode de réalisation, la composition selon l'invention est caractérisée en ce que le polymère anionique hydrophobisé est choisi parmi les polymères anioniques hydrophobisés de formules IV à VII et XII, dans lesquelles le radical -*k*-[E]-(*o*)$_t$ est un radical au moins divalent issu d'un acide alpha aminé.

Dans un mode de réalisation, la composition selon l'invention est caractérisée en ce que le polymère anionique hydrophobisé est choisi parmi les polymères anioniques hydrophobisés de formules IV à VII et XII, dans lesquelles le radical -*k*-[E]-(*o*)$_t$ est un radical au moins divalent issu d'un acide alpha aminé naturel choisi dans le groupe constitué par la glycine, la leucine, la phénylalanine, la lysine, l'isoleucine, l'alanine, la valine, l'acide aspartique et l'acide glutamique, sous leurs formes L, D ou racémique.

Dans un mode de réalisation, la composition selon l'invention est caractérisée en ce que le polymère anionique hydrophobisé est choisi parmi les polymères anioniques hydrophobisés de formules IV à VII et XII, dans lesquelles le radical -*k*-[E]-(*o*)$_t$ est un radical au moins divalent issu d'un mono- ou polyéthylène glycol.

Dans un mode de réalisation, la composition selon l'invention est caractérisée en ce que le polymère anionique hydrophobisé est choisi parmi les polymères anioniques hydrophobisés de formules IV à VII et XII, dans lesquelles le

radical -*k*-[E]-$(o)_t$ est un radical au moins divalent issu de l'éthylène glycol.

Dans un mode de réalisation, la composition selon l'invention est caractérisée en ce que le polymère anionique hydrophobisé est choisi parmi les polymères anioniques hydrophobisés de formules IV à VII et XII, dans lesquelles le radical -*k*-[E]-$(o)_t$ est un radical au moins divalent issu d'un polyéthylène glycol choisi dans le groupe constitué par le diéthylène glycol, le triéthylène glycol et le tétraéthylène glycol.

Dans un mode de réalisation, la composition selon l'invention est caractérisée en ce que le polymère anionique hydrophobisé est choisi parmi les polymères anioniques hydrophobisés de formules IV à VII et XII, dans lesquelles le radical -*k*-[E]-$(o)_t$ est un radical au moins divalent issu d'un mono- ou polyéthylène glycol amine.

Dans un mode de réalisation, la composition selon l'invention est caractérisée en ce que le polymère anionique hydrophobisé est choisi parmi les polymères anioniques hydrophobisés de formules IV à VII et XII, dans lesquelles le radical -*k*-[E]-$(o)_t$ est un radical au moins divalent issu d'un mono- ou polyéthylène glycol amine choisi dans le groupe constitué par l'éthanolamine, le diéthylène glycol amine et le triéthylène glycol amine.

Dans un mode de réalisation, la composition selon l'invention est caractérisée en ce que le polymère anionique hydrophobisé est choisi parmi les polymères anioniques hydrophobisés de formules IV à VII et XII, dans lesquelles le radical -*k*-[E]-$(o)_t$ est un radical au moins divalent issu d'un mono- ou polyéthylène glycol diamine.

Dans un mode de réalisation, la composition selon l'invention est caractérisée en ce que le polymère anionique hydrophobisé est choisi parmi les polymères anioniques hydrophobisés de formules IV à VII et XII, dans lesquelles le radical -*k*-[E]-$(o)_t$ est un radical au moins divalent issu d'un mono- ou polyéthylène glycol diamine choisi dans le groupe constitué par le diéthylène glycol diamine et le triéthylène glycol diamine.

Dans un mode de réalisation, la composition selon l'invention est caractérisée en ce que le polymère anionique hydrophobisé est choisi parmi les polymères anioniques hydrophobisés de formules IV à VII et XII, dans lesquelles le radical -*k*-[E]-$(o)_t$ est un radical au moins divalent issu de l'éthylènediamine.

Dans un mode de réalisation, la composition selon l'invention est caractérisée en ce que le polymère anionique hydrophobisé est choisi parmi les polymères anioniques hydrophobisés de formules IV à VII et XII, dans lesquelles la fonction *o* est une fonction ester.

Dans un mode de réalisation, la composition selon l'invention est caractérisée en ce que le polymère anionique hydrophobisé est choisi parmi les polymères anioniques hydrophobisés de formules IV à VII et XII, dans lesquelles la fonction *o* est une fonction amide.

Dans un mode de réalisation, la composition selon l'invention est caractérisée en ce que le polymère anionique hydrophobisé est choisi parmi les polymères anioniques hydrophobisés de formules IV à VII et XII, dans lesquelles la fonction *o* est une fonction carbamate.

Dans un mode de réalisation, la composition selon l'invention est caractérisée en ce que le polymère anionique hydrophobisé est choisi parmi les polymères anioniques hydrophobisés de formules IV à VII et XII, dans lesquelles la fonction *o* est une fonction carbonate.

Dans un mode de réalisation, la composition selon l'invention est caractérisée en ce que le polymère anionique hydrophobisé est choisi parmi les polymères anioniques hydrophobisés de formules IV à VII et XII, dans lesquelles le groupe -[Hy] est un groupe alkyle issu d'un alcool hydrophobe, insaturé et/ou saturé, ramifié ou non ramifié comprenant de 8 à 30 carbones.

Dans un mode de réalisation, la composition selon l'invention est caractérisée en ce que le polymère anionique hydrophobisé est choisi parmi les polymères anioniques hydrophobisés de formules IV à VII et XII, dans lesquelles le groupe -[Hy] est un groupe alkyle issu d'un alcool hydrophobe choisi dans le groupe constitué par l'octanol, le décanol, le dodécanol (alcool laurylique), le tétradécanol (alcool myristilique), l'hexadécanol (alcool cétylique), l'alcool stéarylique, l'alcool cétéarylique et l'alcool oléylique.

Dans un mode de réalisation, la composition selon l'invention est caractérisée en ce que le polymère anionique hydrophobisé est choisi parmi les polymères anioniques hydrophobisés de formules IV à VII et XII, dans lesquelles le groupe -[Hy] est un groupe issu d'un stérol.

Dans un mode de réalisation, la composition selon l'invention est caractérisée en ce que le polymère anionique hydrophobisé est choisi parmi les polymères anioniques hydrophobisés de formules IV à VII et XII, dans lesquelles le groupe -[Hy] est un groupe issu d'un stérol, choisi dans le groupe constitué par le cholestérol et ses dérivés.

Dans un mode de réalisation, la composition selon l'invention est caractérisée en ce que le polymère anionique hydrophobisé est choisi parmi les polymères anioniques hydrophobisés de formules IV à VII et XII, dans lesquelles le groupe -[Hy] est un groupe issu du cholestérol.

Dans un mode de réalisation, la composition selon l'invention est caractérisée en ce que le polymère anionique hydrophobisé est choisi parmi les polymères anioniques hydrophobisés de formules IV à VII et XII, dans lesquelles le groupe -[Hy] est un groupe issu d'un tocophérol.

Dans un mode de réalisation, la composition selon l'invention est caractérisée en ce que le polymère anionique hydrophobisé est choisi parmi les polymères anioniques hydrophobisés de formules IV à VII et XII, dans lesquelles le groupe -[Hy] est un groupe issu d'un dérivé du tocophérol, choisi parmi le racémique, l'isomère L ou l'isomère D de l'α-tocophérol.

Dans un mode de réalisation, la composition selon l'invention est caractérisée en ce que le polymère anionique hydrophobisé est choisi parmi les polymères anioniques hydrophobisés de formules IV à VII et XII, dans lesquelles le groupe -[Hy] est un groupe issu du DL-$\alpha$-tocophérol.

Dans un mode de réalisation, la composition selon l'invention est caractérisée en ce que le polymère anionique hydrophobisé est choisi parmi les polymères anioniques hydrophobisés de formules IV à VII et XII, dans lesquelles le groupe -[Hy] est un groupe issu du menthol.

Dans un mode de réalisation, la composition selon l'invention est caractérisée en ce que le polymère anionique hydrophobisé est choisi parmi les polymères anioniques hydrophobisés de formules IV à VII et XII, dans lesquelles le groupe -[Hy] est un groupe issu du menthol ou de ses dérivés, choisi parmi le racémique, l'isomère L ou l'isomère D du menthol.

Dans un mode de réalisation, la composition selon l'invention est caractérisée en ce que le polymère anionique hydrophobisé est choisi parmi les polymères anioniques hydrophobisés de formules IV à VII et XII, dans lesquelles le groupe -[Hy] est un groupe alkyle issu d'un acide hydrophobe.

Dans un mode de réalisation, la composition selon l'invention est caractérisée en ce que le polymère anionique hydrophobisé est choisi parmi les polymères anioniques hydrophobisés de formules IV à VII et XII, dans lesquelles le groupe -[Hy] est un groupe alkyle issu d'un acide hydrophobe linéaire, choisi dans le groupe constitué par l'acide dodécanoïque, l'acide tétradécanoïque et l'acide hexadécanoïque.

Dans un mode de réalisation, la composition selon l'invention est caractérisée en ce que le polymère anionique hydrophobisé est choisi parmi les polymères anioniques hydrophobisés de formules IV à VII et XII, dans lesquelles le groupe -[Hy] est un groupe alkyle issu d'un acide gras.

Dans un mode de réalisation, la composition selon l'invention est caractérisée en ce que le polymère anionique hydrophobisé est choisi parmi les polymères anioniques hydrophobisés de formules IV à VII et XII, dans lesquelles le groupe -[Hy] est un groupe alkyle issu d'un acide gras choisi dans le groupe constitué par les acides constitués d'une chaîne alkyle insaturée ou saturée, ramifiée ou non ramifiée, comprenant de 8 à 30 carbones.

Dans un mode de réalisation, la composition selon l'invention est caractérisée en ce que le polymère anionique hydrophobisé est choisi parmi les polymères anioniques hydrophobisés de formules IV à VII et XII, dans lesquelles le groupe -[Hy] est un groupe alkyle issu d'un acide gras choisi dans le groupe constitué par les acides gras linéaires.

Dans un mode de réalisation, la composition selon l'invention est caractérisée en ce que le polymère anionique hydrophobisé est choisi parmi les polymères anioniques hydrophobisés de formules IV à VII et XII, dans lesquelles le groupe -[Hy] est un groupe alkyle issu d'un acide gras linéaire saturé choisi dans le groupe constitué par l'acide caprylique (acide octanoïque), l'acide nonanoïque, l'acide caprique (acide décanoïque), l'acide undécanoïque, l'acide laurique (acide dodécanoïque), l'acide myristique (tétradécanoïque), l'acide palmitique (hexadécanoïque), l'acide stéarique (octadécanoïque), l'acide arachidique (eicosanoïque), l'acide béhénique (docosanoïque), l'acide tricosanoïque, l'acide lignocérique (tétracosanoïque), l'acide heptacosanoïque, l'acide octacosanoïque et l'acide mélissique (tricontanoïque).

Dans un mode de réalisation, la composition selon l'invention est caractérisée en ce que le polymère anionique hydrophobisé est choisi parmi les polymères anioniques hydrophobisés de formules IV à VII et XII, dans lesquelles le groupe -[Hy] est un groupe alkyle issu d'un acide gras insaturé.

Dans un mode de réalisation, la composition selon l'invention est caractérisée en ce que le polymère anionique hydrophobisé est choisi parmi les polymères anioniques hydrophobisés de formules IV à VII et XII, dans lesquelles le groupe -[Hy] est un groupe alkyle issu d'un acide gras insaturé choisi dans le groupe constitué par l'acide myristoléique ((Z)-tétradéc-9-énoïque), l'acide palmitoléique ((Z)-hexadéc-9-énoïque), l'acide oléique ((Z)-octadéc-9-énoïque), l'acide élaidique ((E)-octadéc-9-énoïque), l'acide linoléique ((9Z,12Z)-octadéca-9,12-diénoïque), l'acide alpha-linoléique ((9Z,12Z,15Z)-octadéca-9,12,15-triénoïque), l'acide arachidonique ((5Z,8Z,11Z,14Z)-octadéca-5,8,11,14-tétraénoïque), l'acide eicosapentaenoïque ((5Z,8Z,11Z,14Z,17Z)-eicosa-5,8,11,14,17-pentaénoïque), l'acide erucique (13-docoénoïque) et l'acide docosahexaenoïque ((4Z,7Z,10Z,13Z,16Z,19Z)-docosa-4,7,10,13,16,19-hexaénoïque) .

Dans un mode de réalisation, la composition selon l'invention est caractérisée en ce que le polymère anionique hydrophobisé est choisi parmi les polymères anioniques hydrophobisés de formules IV à VII et XII, dans lesquelles le groupe -[Hy] est un groupe alkyle issu d'un acide de la bile et ses dérivés.

Dans un mode de réalisation, la composition selon l'invention est caractérisée en ce que le polymère anionique hydrophobisé est choisi parmi les polymères anioniques hydrophobisés de formules IV à VII et XII, dans lesquelles le groupe -[Hy] est un groupe alkyle issu d'un acide de la bile et ses dérivés choisi dans le groupe constitué par l'acide cholique, l'acide déhydrocholique, l'acide désoxycholique et l'acide chénodésoxycholique.

Selon un mode de réalisation, la composition selon l'invention est caractérisée en ce que le polymère anionique hydrophobisé selon l'invention est choisi parmi les polymères anioniques hydrophobisés de formule V présentant une masse molaire moyenne en poids allant de 2 à 40 kg/mol.

Selon un mode de réalisation, le polymère anionique hydrophobisé selon l'invention est choisi parmi les polymères anioniques hydrophobisés de formule V présentant une masse molaire moyenne en poids allant de 2 à 20 kg/mol.

Selon un mode de réalisation, le polymère anionique hydrophobisé selon l'invention est choisi parmi les polymères anioniques hydrophobisés de formule V présentant une masse molaire moyenne en poids allant de 2 à 12 kg/mol.

Dans un mode de réalisation particulier, la composition selon l'invention est caractérisée en ce que le polymère anionique hydrophobisé selon l'invention est choisi parmi les polymères anioniques hydrophobisés de formule V répondant aux conditions suivantes :

- lorsque *g*-B-(*k*-D)p comporte une chaine Hy et que Hy est un alkyle en C8 à C15, alors le produit entre le degré de substitution en radicaux hydrophobes et le degré moyen de polymérisation (n) est supérieur ou égal à 2
- lorsque *g*-B-(*k*-D)p comporte une chaine Hy et que Hy est un alkyle en C16 à C20, alors le produit entre le degré de substitution en radicaux hydrophobes et le degré moyen de polymérisation (n) est supérieur ou égal à 1
- lorsque *g*-B-(*k*-D)p comporte deux chaines Hy et que Hy est un alkyle en C8 à C9, alors le produit du degré de substitution en radicaux hydrophobes et du degré moyen de polymérisation (n) est supérieur ou égal à 2,
- lorsque *g*-B-(*k*-D)p comporte deux chaines Hy et que Hy est un alkyle en C10 à C16, alors le produit du degré de substitution en radicaux hydrophobes et du degré moyen de polymérisation (n) est supérieur ou égal à 0,2.

Selon un mode de réalisation, la composition selon l'invention est caractérisée en ce que le polymère anionique hydrophobisé selon l'invention est choisi parmi les polymères anioniques hydrophobisés de formule V dans laquelle le radical -*f*-[A]-COOH est choisi dans le groupe constitué par les radicaux suivants, *f* ayant la signification donnée ci-dessus :

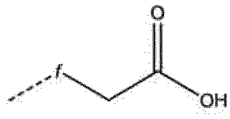

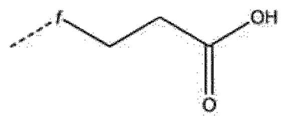

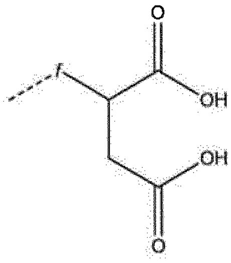

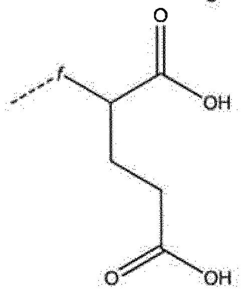

ou leurs sels de cations alcalins choisis dans le groupe constitué de $Na^+$ et $K^+$.

Dans un mode de réalisation, la composition selon l'invention est caractérisée en ce que le polymère anionique hydrophobisé selon l'invention est choisi parmi les polymères anioniques hydrophobisés de formule V, dans lesquelles le radical -[A]- est un radical $-CH_2-$.

Dans un mode de réalisation, la composition selon l'invention est caractérisée en ce que le polymère anionique hydrophobisé selon l'invention est choisi parmi les polymères anioniques hydrophobisés de formule V, dans laquelle la fonction *f* est une fonction éther.

Dans un mode de réalisation, la composition selon l'invention est caractérisée en ce que le polymère anionique hydrophobisé selon l'invention est choisi parmi les polymères anioniques hydrophobisés de formule V, dans laquelle le radical - *g*-[B]-*k*-[D] est choisi dans le groupe constitué par les radicaux suivants ; *g, k* et-[D] ayant les significations données ci-dessus :

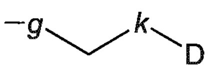

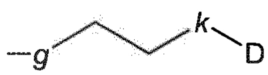

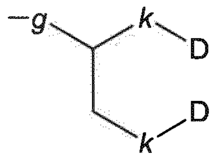

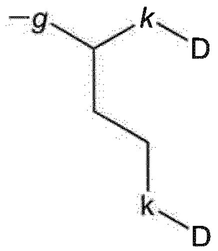

Dans un mode de réalisation, la composition selon l'invention est caractérisée en ce que le polymère anionique hydrophobisé selon l'invention est choisi parmi les polymères anioniques hydrophobisés de formule V, dans laquelle le radical - [B]- est un radical $-CH_2-$.

Dans un mode de réalisation, la composition selon l'invention est caractérisée en ce que le polymère anionique hydrophobisé selon l'invention est choisi parmi les polymères anioniques hydrophobisés de formule V, dans laquelle la fonction g est une fonction éther.

Dans un mode de réalisation, la composition selon l'invention est caractérisée en ce que le polymère anionique hydrophobisé selon l'invention est choisi parmi les polymères anioniques hydrophobisés de formule V, dans laquelle la fonction *k* est une fonction amide.

Dans un mode de réalisation, la composition selon l'invention est caractérisée en ce que le polymère anionique hydrophobisé selon l'invention est choisi parmi les polymères anioniques hydrophobisés de formule V, dans laquelle le radical - $k$-[E]-$(o)_t$ est un radical au moins divalent issu d'un acide aminé.

Dans un mode de réalisation, la composition selon l'invention est caractérisée en ce que le polymère anionique hydrophobisé selon l'invention est choisi parmi les polymères anioniques hydrophobisés de formule V, dans laquelle le radical - $k$-[E]-$(o)_t$ est un radical au moins divalent issu d'un acide alpha aminé.

Dans un mode de réalisation, la composition selon l'invention est caractérisée en ce que le polymère anionique hydrophobisé selon l'invention est choisi parmi les polymères anioniques hydrophobisés de formule V, dans laquelle le radical - $k$-[E]-$(o)_t$ est un radical au moins divalent issu d'un acide alpha aminé naturel choisi dans le groupe constitué par la glycine, la leucine, la phénylalanine, la lysine, l'isoleucine, l'alanine, la valine, l'acide aspartique et l'acide glutamique, sous leurs formes L, D ou racémique.

Dans un mode de réalisation, la composition selon l'invention est caractérisée en ce que le polymère anionique hydrophobisé selon l'invention est choisi parmi les polymères anioniques hydrophobisés de formule V, dans laquelle le radical - $k$-[E]-$(o)_t$ est un radical au moins divalent issu d'un mono- ou polyéthylène glycol amine choisi dans le groupe constitué par l'éthanolamine, le diéthylène glycol amine et le triéthylène glycol amine.

Dans un mode de réalisation, la composition selon l'invention est caractérisée en ce que le polymère anionique hydrophobisé selon l'invention est choisi parmi les polymères anioniques hydrophobisés de formule V, dans laquelle le radical - $k$-[E]-$(o)_t$ est un radical au moins divalent issu de l'éthylènediamine.

Dans un mode de réalisation, la composition selon l'invention est caractérisée en ce que le polymère anionique hydrophobisé selon l'invention est choisi parmi les polymères anioniques hydrophobisés de formule V, dans laquelle la fonction *o* est une fonction ester.

Dans un mode de réalisation, la composition selon l'invention est caractérisée en ce que le polymère anionique hydrophobisé selon l'invention est choisi parmi les polymères anioniques hydrophobisés de formule V, dans laquelle la fonction o est une fonction amide.

Dans un mode de réalisation, la composition selon l'invention est caractérisée en ce que le polymère anionique hydrophobisé selon l'invention est choisi parmi les polymères anioniques hydrophobisés de formule V, dans laquelle la fonction o est une fonction carbamate.

Dans un mode de réalisation, la composition selon l'invention est caractérisée en ce que le polymère anionique hydrophobisé selon l'invention est choisi parmi les polymères anioniques hydrophobisés de formule V, dans laquelle le groupe - [Hy] est un groupe alkyle issu d'un alcool hydrophobe présentant un LogP supérieur ou égal à 2.

Dans un mode de réalisation, la composition selon l'invention est caractérisée en ce que le polymère anionique hydrophobisé selon l'invention est choisi parmi les polymères anioniques hydrophobisés de formule V, dans laquelle le groupe - [Hy] est un groupe alkyle issu d'un alcool hydrophobe, insaturé et/ou saturé, ramifié ou non ramifié comprenant de 8 à 30 carbones.

Dans un mode de réalisation, la composition selon l'invention est caractérisée en ce que le polymère anionique hydrophobisé selon l'invention est choisi parmi les polymères anioniques hydrophobisés de formule V, dans laquelle le groupe - [Hy] est un groupe alkyle issu d'un alcool hydrophobe choisi dans le groupe constitué par l'octanol, le décanol, le dodécanol (alcool laurylique), le tétradécanol (alcool myristilique), l'hexadécanol (alcool cétylique), l'alcool stéarylique, l'alcool cétéarylique et l'alcool oléylique.

Dans un mode de réalisation, la composition selon l'invention est caractérisée en ce que le polymère anionique

hydrophobisé selon l'invention est choisi parmi les polymères anioniques hydrophobisés de formule V, dans laquelle le groupe - [Hy] est un groupe issu d'un stérol.

Dans un mode de réalisation, la composition selon l'invention est caractérisée en ce que le polymère anionique hydrophobisé selon l'invention est choisi parmi les polymères anioniques hydrophobisés de formule V, dans laquelle le groupe - [Hy] est un groupe issu d'un stérol, choisi dans le groupe constitué par le cholestérol et ses dérivés.

Dans un mode de réalisation, la composition selon l'invention est caractérisée en ce que le polymère anionique hydrophobisé selon l'invention est choisi parmi les polymères anioniques hydrophobisés de formule V, dans aquelle le groupe - [Hy] est un groupe issu du cholestérol.

Dans un mode de réalisation, la composition selon l'invention est caractérisée en ce que le polymère anionique hydrophobisé selon l'invention est choisi parmi les polymères anioniques hydrophobisés de formule V, dans laquelle le groupe - [Hy] est un groupe issu d'un tocophérol.

Dans un mode de réalisation, la composition selon l'invention est caractérisée en ce que le polymère anionique hydrophobisé selon l'invention est choisi parmi les polymères anioniques hydrophobisés de formule V, dans laquelle le groupe - [Hy] est un groupe issu d'un dérivé du tocophérol, choisi parmi le racémique, l'isomère L ou l'isomère D de l'α-tocophérol.

Dans un mode de réalisation, la composition selon l'invention est caractérisée en ce que le polymère anionique hydrophobisé selon l'invention est choisi parmi les polymères anioniques hydrophobisés de formule V, dans laquelle le groupe - [Hy] est un groupe issu du DL-α-tocophérol.

Dans un mode de réalisation, la composition selon l'invention est caractérisée en ce que le polymère anionique hydrophobisé selon l'invention est choisi parmi les polymères anioniques hydrophobisés de formule V, dans laquelle le groupe - [Hy] est un groupe issu du menthol.

Dans un mode de réalisation, la composition selon l'invention est caractérisée en ce que le polymère anionique hydrophobisé selon l'invention est choisi parmi les polymères anioniques hydrophobisés de formule V, dans laquelle le groupe - [Hy] est un groupe issu du menthol ou de ses dérivés, choisi parmi le racémique, l'isomère L ou l'isomère D du menthol.

Dans un mode de réalisation, la composition selon l'invention est caractérisée en ce que le polymère anionique hydrophobisé selon l'invention est choisi parmi les polymères anioniques hydrophobisés de formule V, dans laquelle le groupe - [Hy] est un groupe alkyle issu d'un acide hydrophobe.

Dans un mode de réalisation, la composition selon l'invention est caractérisée en ce que le polymère anionique hydrophobisé selon l'invention est choisi parmi les polymères anioniques hydrophobisés de formule V, dans laquelle le groupe - [Hy] est un groupe alkyle issu d'un acide hydrophobe présentant un LogP supérieur ou égal à 2.

Dans un mode de réalisation, la composition selon l'invention est caractérisée en ce que le polymère anionique hydrophobisé selon l'invention est choisi parmi les polymères anioniques hydrophobisés de formule V, dans laquelle le groupe - [Hy] est un groupe alkyle issu d'un acide hydrophobe linéaire, choisi dans le groupe constitué par l'acide dodécanoïque, l'acide tétradécanoïque et l'acide hexadécanoïque.

Dans un mode de réalisation, la composition selon l'invention est caractérisée en ce que le polymère anionique hydrophobisé selon l'invention est choisi parmi les polymères anioniques hydrophobisés de formule V, dans laquelle le groupe - [Hy] est un groupe alkyle issu d'un acide gras.

Dans un mode de réalisation, la composition selon l'invention est caractérisée en ce que le polymère anionique hydrophobisé selon l'invention est choisi parmi les polymères anioniques hydrophobisés de formule V, dans laquelle le groupe - [Hy] est un groupe alkyle issu d'un acide gras choisi dans le groupe constitué par les acides constitués d'une chaîne alkyle insaturée ou saturée, ramifiée ou non ramifiée, comprenant de 8 à 30 carbones.

Dans un mode de réalisation, la composition selon l'invention est caractérisée en ce que le polymère anionique hydrophobisé selon l'invention est choisi parmi les polymères anioniques hydrophobisés de formule V, dans laquelle le groupe - [Hy] est un groupe alkyle issu d'un acide gras choisi dans le groupe constitué par les acides gras linéaires.

Dans un mode de réalisation, la composition selon l'invention est caractérisée en ce que le polymère anionique hydrophobisé selon l'invention est choisi parmi les polymères anioniques hydrophobisés de formule V, dans laquelle le groupe - [Hy] est un groupe alkyle issu d'un acide gras linéaire saturé choisi dans le groupe constitué par l'acide caprylique (acide octanoïque), l'acide nonanoïque, l'acide caprique (acide décanoïque), l'acide undécanoïque, l'acide laurique (acide dodécanoïque), l'acide myristique (tétradécanoïque), l'acide palmitique (hexadécanoïque), l'acide stéarique (octadécanoïque), l'acide arachidique (eicosanoïque), l'acide béhénique (docosanoïque), l'acide tricosanoïque, l'acide lignocérique (tétracosanoïque), l'acide heptacosanoïque, l'acide octacosanoïque et l'acide mélissique (tricontanoïque).

Dans un mode de réalisation, la composition selon l'invention est caractérisée en ce que le polymère anionique hydrophobisé selon l'invention est choisi parmi les polymères anioniques hydrophobisés de formule V, dans laquelle le groupe - [Hy] est un groupe alkyle issu d'un acide gras insaturé.

Dans un mode de réalisation, la composition selon l'invention est caractérisée en ce que le polymère anionique hydrophobisé selon l'invention est choisi parmi les polymères anioniques hydrophobisés de formule V, dans laquelle le groupe - [Hy] est un groupe alkyle issu d'un acide gras insaturé choisi dans le groupe constitué par l'acide myristoléique

((Z)-tétradéc-9-énoïque), l'acide palmitoléique ((Z)-hexadéc-9-énoïque), l'acide oléique ((Z)-octadéc-9-énoïque), l'acide élaidique ((E)-octadéc-9-énoïque), l'acide linoléique ((9Z,12Z)-octadéca-9,12-diénoïque), l'acide alpha-linoléique ((9Z,12Z,15Z)-octadéca-9,12,15-triénoïque), l'acide arachidonique ((5Z,8Z,11Z,14Z)-octadéca-5,8,11,14-tétraénoïque), l'acide eicosapentaenoïque ((5Z,8Z,11Z,14Z,17Z)-eicosa-5,8,11,14,17-pentaénoïque), l'acide erucique (13-docoénoïque) et l'acide docosahexaenoïque ((4Z,7Z,10Z,13Z,16Z,19Z)-docosa-4,7,10,13,16,19-hexaénoïque) .

Dans un mode de réalisation, la composition selon l'invention est caractérisée en ce que le polymère anionique hydrophobisé selon l'invention est choisi parmi les polymères anioniques hydrophobisés de formule V, dans laquelle le groupe - [Hy] est un groupe alkyle issu d'un acide de la bile et ses dérivés.

Dans un mode de réalisation, la composition selon l'invention est caractérisée en ce que le polymère anionique hydrophobisé selon l'invention est choisi parmi les polymères anioniques hydrophobisés de formule V, dans laquelle le groupe - [Hy] est un groupe alkyle issu d'un acide de la bile et ses dérivés choisi dans le groupe constitué par l'acide cholique, l'acide déhydrocholique, l'acide désoxycholique et l'acide chénodésoxycholique.

Dans un mode de réalisation, la composition selon l'invention est caractérisée en ce que le polymère anionique hydrophobisé selon l'invention est choisi parmi les polymères anioniques hydrophobisés de formule V, dans laquelle -R' est soit un groupement -OH, soit un radical -*f*-[A]-COOH, soit un radical -*g*-[B]-*k*-[D], *f* étant une fonction éther, le radical -[A]- étant un radical -$CH_2$-, *g* étant une fonction éther, le radical -[B]- étant un radical -$CH_2$-, *k* étant une fonction amide, -[D] étant un radical -[E]-*o*-Hy, p et t = 1, le radical -*k*-[E]-$(o)_t$ étant issu d'un acide aminé, o étant une fonction ester et Hy étant issu d'un stérol ou d'un alcool gras, en particulier :

- le radical -*k*-[E]-$(o)_t$ étant issu de la leucine et Hy étant issu du cholestérol,
- le radical -*k*-[E]-$(o)_t$ étant issu de la glycine et Hy étant issu du dodécanol,
- le radical -*k*-[E]-$(o)_t$ étant issu de la leucine et Hy étant issu du tocophérol, et
- le radical -*k*-[E]-$(o)_t$ étant issu de la phénylalanine et Hy étant issu de l'octanol, et
- le radical -*k*-[E]-$(o)_t$ étant issu de la phénylalanine et Hy étant issu du 3,7-diméthyloctan-1-ol.

Dans un mode de réalisation, la composition selon l'invention est caractérisée en ce que le polymère anionique hydrophobisé selon l'invention est choisi parmi les polymères anioniques hydrophobisés de formule V, dans laquelle -R' est soit un groupement -OH, soit un radical -*f*-[A]-COOH, soit un radical -*g*-[B]-*k*-[D], *f* étant une fonction éther, le radical -[A]- étant un radical -$CH_2$-, *g* étant une fonction éther, le radical -[B]- étant un radical -$CH_2$-, *k* étant une fonction amide, -[D] étant un radical -[E]-$(o\text{-Hy})_2$, p = 1 et t = 2, le radical -*k*-[E]-$(o)_t$ étant issu d'un acide aminé, o étant une fonction ester et Hy étant issu d'un alcool gras, en particulier le radical - [E]- étant issu de l'acide aspartique et Hy étant issu du docécanol ou du décanol.

Dans un mode de réalisation, la composition selon l'invention est caractérisée en ce que le polymère anionique hydrophobisé selon l'invention est choisi parmi les polymères anioniques hydrophobisés de formule V, dans laquelle -R' est soit un groupement -OH, soit un radical -*f*-[A]-COOH, soit un radical -*g*-[B]-*k*-[D], *f* étant une fonction éther, le radical -[A]- étant un radical -$CH_2$-, *g* étant une fonction éther, le radical -[B]- étant un radical -$CH_2$-, *k* étant une fonction amide, -[D] étant un radical -[E]-*o*-Hy, p et t = 1, le radical -*k*-[E]-$(o)_t$ étant issu d'une diamine, *o* étant une fonction amide et Hy étant issu d'un acide gras, en particulier le radical -*k*-[E]-$(o)_t$ étant issu de l'éthylène diamine et Hy étant issu de l'acide dodécanoïque.

Dans un mode de réalisation, la composition selon l'invention est caractérisée en ce que le polymère anionique hydrophobisé selon l'invention est choisi parmi les polymères anioniques hydrophobisés de formule V, dans laquelle -R' est soit un groupement -OH, soit un radical -*f*-[A]-COOH, soit un radical -*g*-[B]-*k*-[D], *f* étant une fonction éther, le radical -[A]- étant un radical -$CH_2$-, *g* étant une fonction éther, le radical -[B]- étant un radical -$CH_2$-, *k* étant une fonction amide, -[D] étant un radical -[E]-*o*-Hy, p et t = 1, le radical -*k*-[E]-$(o)_t$ étant issu d'une diamine, *o* étant une fonction carbamate et Hy étant issu d'un stérol, en particulier le radical -*k*-[E]-$(o)_t$ étant issu de l'éthylène diamine et Hy étant issu du cholestérol.

Dans un mode de réalisation, la composition selon l'invention est caractérisée en ce que le polymère anionique hydrophobisé selon l'invention est choisi parmi les polymères anioniques hydrophobisés de formule V, dans laquelle -R' est soit un groupement -OH, soit un radical -*f*-[A]-COOH, soit un radical -*g*-[B]-*k*-[D], *f* étant une fonction éther, le radical -[A]- étant un radical -$CH_2$-, *g* étant une fonction éther, le radical -[B]- étant un radical -$CH_2$-, *k* étant une fonction amide, -[D] étant un radical -[E]-*o*-Hy, p et t = 1, le radical -*k*-[E]-$(o)_t$ étant issu d'un amino-alcool, *o* étant une fonction ester et Hy étant issu d'un alcool gras, en particulier le radical -*k*-[E]-$(o)_t$ étant issu du 2-(2-amino-éthoxy)éthanol et Hy étant issu du dodécanol.

L'invention concerne également un polymère anionique hydrophobisé de formule XII :

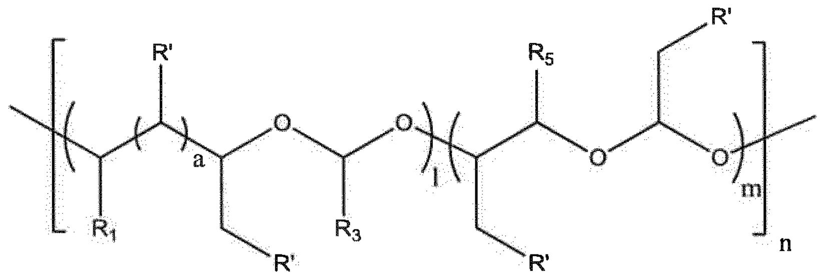

Formule XII

dans laquelle,

- l = 0 ou 1,
- m = 0, 1 ou 2,
- a = 0 ou 1,
- n est le degré de polymérisation, compris entre 3 et 1000,
  et
- $-R_1$ est un hydrogène -H,
- $-R_3$ est un radical $-CH_2R'$,
- $-R_5$ est soit un groupe -COOH, soit un radical $-CH_2R'$, soit un radical -*k*-[D], dans lequel :

  - -[D] est un radical -[Hy] ou -[E]-(*o*-[Hy])$_t$ ;
  - -[E]- est un radical au moins divalent comprenant de 1 à 15 atomes de carbone comprenant au moins un hétéroatome choisi parmi O, N et S, éventuellement porteur de fonctions carboxyle ou amine et/ou -*k*-[E]-(*o*)$_t$, comprenant de 2 à 16 atomes de carbone, est issu d'un acide aminé, d'un dialcool, d'une diamine, ou d'un amine-alcool ;
  - -[Hy] est un groupe alkyle, linéaire ou cyclique, un alkylaryle ou un arylalkyle, en C8 à C30, éventuellement substitué par un ou plusieurs groupes alkyles en C1 à C3, issu d'un composé hydrophobe ;
  - *k* résultant de la réaction entre une fonction carboxyle, amine ou alcool du précurseur de -*k*-[E]-(*o*)$_t$ et une fonction alcool, carboxyle ou amine du polymère et est une fonction choisie dans le groupe constitué par les fonctions ester, amide, carbonate et carbamate;
  - *o* résultant de la réaction entre une fonction carboxyle, amine ou alcool du précurseur de -*k*-[E]-(*o*)$_t$ et une fonction alcool ou acide du précurseur de -[Hy] est une fonction choisie dans le groupe constitué par les fonctions ester, amide, urée (carbamide), carbonate et carbamate ;
  - t est un entier positif égal à 1 ou 2 ;

  ou
- $-R_1$ et $-R_3$ forment un cycle à six chaînons $-R_1$-$R_3$- = $-CH(NHCOCH_3)-$, et,
- -R' est choisi dans le groupe constitué par les radicaux :

  ◦ -OH
  ◦ -O-Alk, Alk étant une chaine alkyle en C1 à C3,
  ◦ -(*f*-[A]-COOH), dans lequel :

    - -[A]- est un radical au moins divalent comprenant de 1 à 15 atomes de carbones comprenant au moins un hétéroatome choisi parmi O, N et S, éventuellement porteur de fonctions carboxyle ou amine et/ou -*f*-[A]-COOH est issu d'un acide aminé, d'un diacide ou d'un alcoolacide et est lié au squelette de la molécule par une fonction *f* ;
    - *f* résultant de la réaction entre une fonction carboxyle ou alcool ou amine du précurseur de -*f*-[A]-COOH et une fonction hydroxyle du squelette est choisie dans le groupe constitué par les fonctions éther, ester carbamate ou carbonate ;

  ◦ -*g*-[B]-(*k*-[D])$_p$, dans lequel :

    - -[B]- est un radical au moins divalent comprenant de 1 à 15 atomes de carbones comprenant au moins un hétéroatome choisi parmi O, N et S, éventuellement porteurs de fonctions carboxyle ou amine et/ou

-*g*-[B]-(k-)$_p$ est issu d'un acide aminé, d'un diacide, d'un dialcool, d'un alcoolacide, d'une diamine ou d'un amine-alcool et est lié au squelette de la molécule par une fonction g et est lié à au moins un radical -[D] par une fonction k,

- *g* résultant de la réaction entre une fonction carboxyle ou alcool ou amine du précurseur de -*g*-[B]-(k-)$_p$ et une fonction du squelette est choisie dans le groupe constitué par les fonctions éther, amine, ester, carbamate ou carbonate,
- *k* résultant de la réaction entre une fonction carboxyle ou alcool ou amine du précurseur de -*g*-[B]-(k-)$_p$ et une fonction alcool ou acide du précurseur de -[D] choisie dans le groupe constitué par les fonctions ester, amide ou carbamate ;
- p est un entier positif égal à 1 ou 2;

et, -[A]-, -[B]- et -[E]- sont identiques ou différents,
et *k* et *o* sont identiques ou différentes ;
et, si -[B]- est un radical trivalent, alors -[D] est un radical -[Hy],
et, le degré de substitution en charges carboxylates est le nombre moyen de charges carboxylates par monomère divisé par (l+m) et est supérieur ou égal à 0,4,
et, le degré de substitution en radicaux hydrophobes est le nombre moyen de radicaux hydrophobes par monomère divisé par (l+m) et est inférieur ou égal à 0,5, et, si le polymère anionique hydrophobisé est un polysaccharide, alors les liaisons glycosidiques identiques ou différentes, peuvent être de type α et/ou de type β.

Dans un mode de réalisation, le polymère anionique hydrophobisé selon l'invention est choisi parmi les polymères anioniques hydrophobisés de formule XII, dans laquelle, l = 0 et m = 1, autrement exprimé, il est choisi parmi les polymères anioniques hydrophobisés de formule IV,

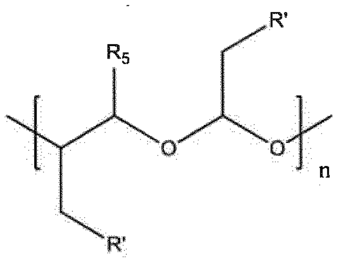

Formule IV

dans laquelle, -$R_5$ est soit un groupe -COOH, soit un radical -$CH_2R'$, soit un radical -*k*-[D], -R' et n tels que définis ci-dessus.

Dans un mode de réalisation, le polymère anionique hydrophobisé selon l'invention est choisi parmi les polymères anioniques hydrophobisés de formule IV :

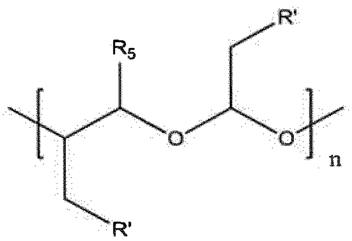

Formule IV

dans laquelle, -$R_5$ est soit un groupe -COOH, soit un radical -*k*-[D], -R' et n tels que définis ci-dessus.

Dans un mode de réalisation, le polymère anionique hydrophobisé selon l'invention est choisi parmi les polymères anioniques hydrophobisés de formule IV :

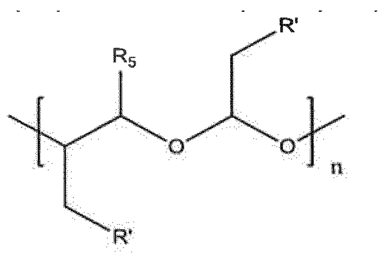

Formule IV

dans laquelle, $-R_5$ est un radical $-CH_2R'$, -R' et n tels que définis ci-dessus.

Dans un mode de réalisation, le polymère anionique hydrophobisé selon l'invention est choisi parmi les polymères anioniques hydrophobisés de formule XII, dans laquelle, m = 0, l = 1, a = 0, autrement exprimé, il est choisi parmi les polymères anioniques hydrophobisés de formule V :

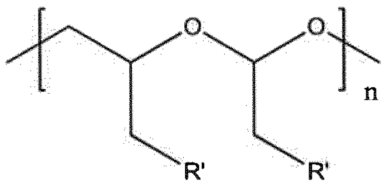

Formule V

et, -R' et n tels que définis ci-dessus.

Dans un mode de réalisation, le polymère anionique hydrophobisé selon l'invention est choisi parmi les polymères anioniques hydrophobisés de formule XII, dans laquelle, l = 1, m = 2 et a = 0, autrement exprimé, il est choisi parmi les polymères anioniques hydrophobisés de formule VI :

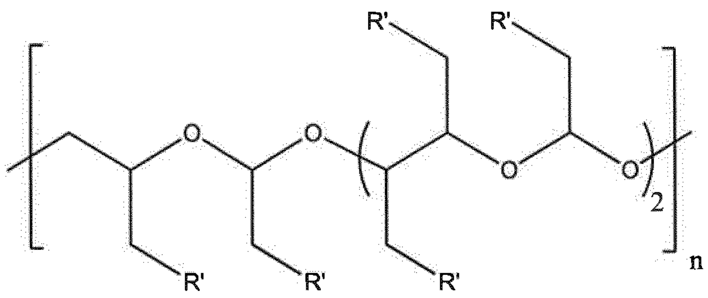

Formule VI

et, -R' et n tels que définis ci-dessus.

Dans un mode de réalisation, le polymère anionique hydrophobisé selon l'invention est choisi parmi les polymères anioniques hydrophobisés de formule XII, dans laquelle, m = 1, l = 1, a = 1, $-R_1$-$R_3$- = $-CH(NHCOCH_3)-$, $-R_2$ = $-CH_2R'$, $-R_4$ = $-CH_2R'$, $-R_5$ est soit un groupe -COOH, soit un radical -*k*-[D], $-R_6$ = $-CH_2R'$, autrement exprimé, il est choisi parmi les polymères anioniques hydrophobisés de formule VII :

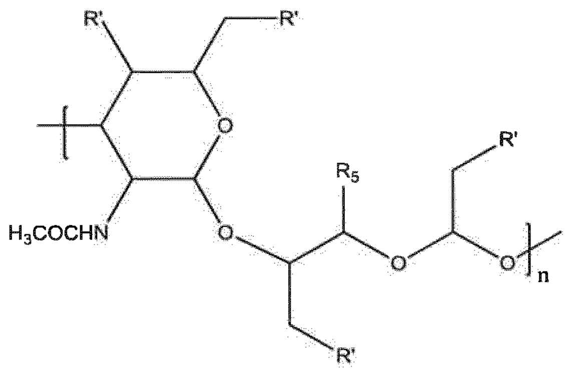

Formule VII

R' et n tels que définis ci-dessus.

Dans un mode de réalisation, le polymère anionique hydrophobisé selon l'invention est choisi parmi les polymères anioniques hydrophobisés de formules IV à VII et XII, dans lesquelles le radical -*f*-[A]-COOH est choisi parmi les radicaux de formule II suivante :

-*f*-[A]-COOH = 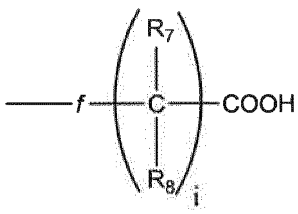

Formule II

dans laquelle :

- i supérieur ou égal à 1 et inférieur ou égal à 12, et,
- $-R_7$ et $-R_8$, identiques ou différents, sont choisis dans le groupe constitué par un atome d'hydrogène, un alkyle saturé ou insaturé, linéaire, ramifié ou cyclique en C1 à C6, un benzyle, un alkyle-aryle et comportant éventuellement des hétéroatomes choisis dans le groupe constitué par O, N et/ou S, ou des fonctions choisies dans le groupe constitué par les fonctions acide carboxylique, amine, alcool ou thiol.

Dans un mode de réalisation, le polymère anionique hydrophobisé selon l'invention est choisi parmi les composés de formule I, dans laquelle le radical -*f*-[A]-COOH, comprenant de 2 à 8 atomes de carbone, est issu d'un acide aminé, d'un dialcool, d'une diamine, d'un diacide ou d'un amine-alcool.

Dans un mode de réalisation, le polymère anionique hydrophobisé selon l'invention est choisi parmi les composés de formule I, dans laquelle le radical -*f*-[A]-COOH, comprenant de 2 à 6 atomes de carbone, est issu d'un acide aminé, d'un dialcool, d'une diamine, d'un diacide ou d'un amine-alcool.

Dans un mode de réalisation particulier, le polymère anionique hydrophobisé selon l'invention est choisi parmi les polymères anioniques hydrophobisés de formules IV à VII et XII répondant aux conditions suivantes :

- lorsque -*g*-[B]-(*k*-[D])$_p$ comporte une chaine Hy et que Hy est un alkyle en C8 à C15, alors le produit entre le degré de substitution en radicaux hydrophobes et le degré moyen de polymérisation (n) est supérieur ou égal à 2
- lorsque -*g*-[B]-(*k*-[D])$_p$ comporte une chaine Hy et que Hy est un alkyle en C16 à C20, alors le produit entre le degré de substitution en radicaux hydrophobes et le degré moyen de polymérisation (n) est supérieur ou égal à 1
- lorsque -*g*-[B]-(*k*-[D])$_p$ comporte deux chaines Hy et que Hy est un alkyle en C8 à C9, alors le produit du degré de substitution en radicaux hydrophobes et du degré moyen de polymérisation (n) est supérieur ou égal à 2,
- lorsque -*g*-[B]-(*k*-[D])$_p$ comporte deux chaines Hy et que Hy est un alkyle en C10 à C16, alors le produit du degré de substitution en radicaux hydrophobes et du degré moyen de polymérisation (n) est supérieur ou égal à 0,2.

Dans un mode de réalisation, le polymère anionique hydrophobisé selon l'invention est choisi parmi les polymères anioniques hydrophobisés de formules IV à VII et XII, dans lesquelles le radical -*f*-[A]-COOH est choisi dans le groupe constitué par les radicaux suivants, *f* ayant la signification donnée ci-dessus :

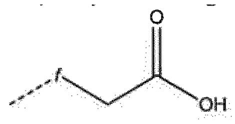
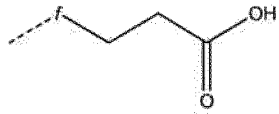
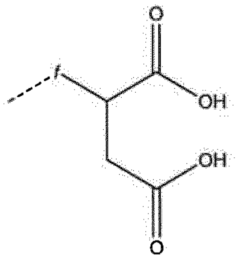
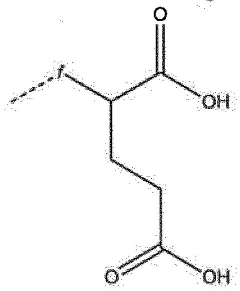

ou leur sels de cations alcalins choisis dans le groupe constitué de $Na^+$ et $K^+$.

Dans un mode de réalisation, le polymère anionique hydrophobisé selon l'invention est choisi parmi les polymères anioniques hydrophobisés de formules IV à VII et XII, dans lesquelles le radical -[A]- est un radical $-CH_2-$.

Dans un mode de réalisation, le polymère anionique hydrophobisé selon l'invention est choisi parmi les polymères anioniques hydrophobisés de formules IV à VII et XII, dans lesquelles le radical -*f*-[A]-COOH est issu d'un acide aminé.

Dans un mode de réalisation, le polymère anionique hydrophobisé selon l'invention est choisi parmi les polymères anioniques hydrophobisés de formules IV à VII et XII, dans lesquelles le radical -*f*-[A]-COOH est issu de la glycine.

Dans un mode de réalisation, le polymère anionique hydrophobisé selon l'invention est choisi parmi les polymères anioniques hydrophobisés de formules IV à VII et XII, dans lesquelles le radical -*f*-[A]-COOH est issu de l'acide aspartique.

Dans un mode de réalisation, le polymère anionique hydrophobisé selon l'invention est choisi parmi les polymères anioniques hydrophobisés de formules IV à VII et XII, dans lesquelles le radical -*f*-[A]-COOH est issu de l'acide glutamique.

Dans un mode de réalisation, le polymère anionique hydrophobisé selon l'invention est choisi parmi les polymères anioniques hydrophobisés de formules IV à VII et XII, dans lesquelles le radical -*f*-[A]-COOH est issu de l'acide succinique.

Dans un mode de réalisation, le polymère anionique hydrophobisé selon l'invention est choisi parmi les polymères anioniques hydrophobisés de formules IV à VII et XII, dans lesquelles la fonction *f* est une fonction éther.

Dans un mode de réalisation, le polymère anionique hydrophobisé selon l'invention est choisi parmi les polymères anioniques hydrophobisés de formules IV à VII et XII, dans lesquelles la fonction *f* est une fonction carbamate.

Dans un mode de réalisation, le polymère anionique hydrophobisé selon l'invention est choisi parmi les polymères anioniques hydrophobisés de formules IV à VII et XII, dans lesquelles la fonction *f* est une fonction ester.

Dans un mode de réalisation, le polymère anionique hydrophobisé selon l'invention est choisi parmi les polymères anioniques hydrophobisés de formules IV à VII et XII, dans lesquelles la fonction *f* est une fonction carbonate.

Dans un mode de réalisation, le polymère anionique hydrophobisé selon l'invention est choisi parmi les polymères anioniques hydrophobisés de formules IV à VII et XII, dans lesquelles la fonction *f* est une fonction amide.

Dans un mode de réalisation, le polymère anionique hydrophobisé selon l'invention est choisi parmi les polymères anioniques hydrophobisés de formule IV à VII et XII, dans lesquelles le radical -*g*-[B]-est choisi parmi les radicaux de formule III suivante :

-*g*-[B]- = 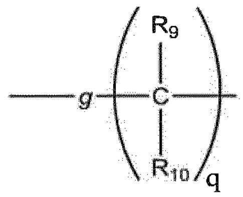

Formule III

dans laquelle :

- q supérieur ou égal à 1 et inférieur ou égal à 12, et,
- $-R_9$ et $-R_{10}$ identiques ou différents sont choisis dans le groupe constitué par un atome d'hydrogène, un alkyle saturé ou insaturé, linéaire, ramifié ou cyclique en C1 à C6, un benzyle, un alkyle-aryle et comportant éventuellement des hétéroatomes choisis dans le groupe constitué par O, N et/ou S, ou des fonctions choisies dans le groupe constitué par les fonctions acide carboxylique, amine, alcool ou thiol.

Dans un mode de réalisation, le polymère anionique hydrophobisé selon l'invention est choisi parmi les polymères anioniques hydrophobisés de formules IV à VII, dans lesquelles le radical -*g*-[B]-*k*-[D] est choisi dans le groupe constitué par les radicaux suivants ; *g, k* et-[D] ayant les significations données ci-dessus :

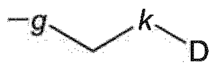 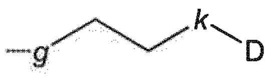

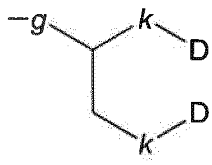 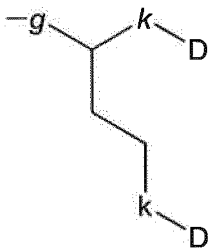

Dans un mode de réalisation, le polymère anionique hydrophobisé selon l'invention est choisi parmi les polymères anioniques hydrophobisés de formules IV à VII et XII, dans lesquelles le radical -[B]- est un radical $-CH_2-$.

Dans un mode de réalisation, le polymère anionique hydrophobisé selon l'invention est choisi parmi les polymères anioniques hydrophobisés de formules IV à VII et XII, dans lesquelles le radical -*g*-[B]-(*k*-[D])$_p$ est tel que -*g*-[B]-*k*- est issu d'un acide aminé.

Dans un mode de réalisation, le polymère anionique hydrophobisé selon l'invention est choisi parmi les polymères anioniques hydrophobisés de formules IV à VII et XII, dans lesquelles le radical -*g*-[B]-(*k*-[D])$_p$ est tel que -*g*-[B]-*k*- est issu de la glycine.

Dans un mode de réalisation, le polymère anionique hydrophobisé selon l'invention est choisi parmi les polymères anioniques hydrophobisés de formules IV à VII et XII, dans lesquelles le radical -*g*-[B]-(*k*-[D])$_p$ est tel que -*g*-[B]-*k*- est issu de l'acide aspartique.

Dans un mode de réalisation, le polymère anionique hydrophobisé selon l'invention est choisi parmi les polymères anioniques hydrophobisés de formules IV à VII et XII, dans lesquelles le radical -*g*-[B]-(*k*-[D])$_p$ est tel que -*g*-[B]-*k*- est issu de l'acide glutamique.

Dans un mode de réalisation, le polymère anionique hydrophobisé selon l'invention est choisi parmi les polymères anioniques hydrophobisés de formule IV à VII et XII, dans lesquelles le radical -*g*-[B]-(*k*-[D])$_p$ est tel que -*g*-[B]-*k*- est issu de l'acide succinique.

Dans un mode de réalisation, le polymère anionique hydrophobisé selon l'invention est choisi parmi les polymères anioniques hydrophobisés de formules IV à VII et XII, dans lesquelles la fonction *g* est une fonction éther.

Dans un mode de réalisation, le polymère anionique hydrophobisé selon l'invention est choisi parmi les polymères anioniques hydrophobisés de formules IV à VII et XII, dans lesquelles la fonction *g* est une fonction carbamate.

Dans un mode de réalisation, le polymère anionique hydrophobisé selon l'invention est choisi parmi les polymères anioniques hydrophobisés de formules IV à VII et XII, dans lesquelles la fonction *g* est une fonction ester.

Dans un mode de réalisation, le polymère anionique hydrophobisé selon l'invention est choisi parmi les polymères anioniques hydrophobisés de formules IV à VII et XII, dans lesquelles la fonction *g* est une fonction amine.

Dans un mode de réalisation, le polymère anionique hydrophobisé selon l'invention est choisi parmi les polymères anioniques hydrophobisés de formules IV à VII et XII, dans lesquelles la fonction *g* est une fonction carbonate.

Dans un mode de réalisation, le polymère anionique hydrophobisé selon l'invention est choisi parmi les polymères anioniques hydrophobisés de formules IV à VII et XII, dans lesquelles la fonction *k* est une fonction amide.

Dans un mode de réalisation, le polymère anionique hydrophobisé selon l'invention est choisi parmi les polymères anioniques hydrophobisés de formules IV à VII et XII, dans lesquelles la fonction *k* est une fonction carbamate.

Dans un mode de réalisation, le polymère anionique hydrophobisé selon l'invention est choisi parmi les polymères anioniques hydrophobisés de formules IV à VII et XII, dans lesquelles la fonction *k* est une fonction ester.

Dans un mode de réalisation, le polymère anionique hydrophobisé selon l'invention est choisi parmi les polymères anioniques hydrophobisés de formules IV à VII et XII, dans lesquelles le radical $-k-[E]-(o)_t$ est un radical au moins divalent issu d'un acide aminé.

Dans un mode de réalisation, le polymère anionique hydrophobisé selon l'invention est choisi parmi les polymères anioniques hydrophobisés de formules IV à VII et XII, dans lesquelles le radical $-k-[E]-(o)_t$ est un radical au moins divalent issu d'un acide alpha aminé.

Dans un mode de réalisation, le polymère anionique hydrophobisé selon l'invention est choisi parmi les polymères anioniques hydrophobisés de formules IV à VII et XII, dans lesquelles le radical $-k-[E]-(o)_t]-$ est un radical au moins divalent issu d'un acide alpha aminé naturel choisi dans le groupe constitué par la glycine, la leucine, la phénylalanine, la lysine, l'isoleucine, l'alanine, la valine, l'acide aspartique et l'acide glutamique, sous leurs formes L, D ou racémique.

Dans un mode de réalisation, le polymère anionique hydrophobisé selon l'invention est choisi parmi les polymères anioniques hydrophobisés de formules IV à VII et XII, dans lesquelles le radical $-k-[E]-(o)_t$ est un radical au moins divalent issu d'un mono- ou polyéthylène glycol.

Dans un mode de réalisation, le polymère anionique hydrophobisé selon l'invention est choisi parmi les polymères anioniques hydrophobisés de formules IV à VII et XII, dans lesquelles le radical $-k-[E]-(o)_t$ est un radical au moins divalent issu de l'éthylène glycol.

Dans un mode de réalisation, le polymère anionique hydrophobisé selon l'invention est choisi parmi les polymères anioniques hydrophobisés de formules IV à VII et XII, dans lesquelles le radical $-k-[E]-(o)_t$ est un radical au moins divalent issu d'un polyéthylène glycol choisi dans le groupe constitué par le diéthylène glycol, le triéthylène glycol et le tétraéthylène glycol.

Dans un mode de réalisation, le polymère anionique hydrophobisé selon l'invention est choisi parmi les polymères anioniques hydrophobisés de formules IV à VII et XII, dans lesquelles le radical $-k-[E]-(o)_t$ est un radical au moins divalent issu d'un mono- ou polyéthylène glycol amine.

Dans un mode de réalisation, le polymère anionique hydrophobisé selon l'invention est choisi parmi les polymères anioniques hydrophobisés de formules IV à VII et XII, dans lesquelles le radical $-k-[E]-(o)_t$ est un radical au moins divalent issu d'un mono- ou polyéthylène glycol amine choisi dans le groupe constitué par l'éthanolamine, le diéthylène glycol amine et le triéthylène glycol amine.

Dans un mode de réalisation, le polymère anionique hydrophobisé selon l'invention est choisi parmi les polymères anioniques hydrophobisés de formules IV à VII et XII, dans lesquelles le radical $-k-[E]-(o)_t$ est un radical au moins divalent issu d'un mono- ou polyéthylène glycol diamine.

Dans un mode de réalisation, le polymère anionique hydrophobisé selon l'invention est choisi parmi les polymères anioniques hydrophobisés de formules IV à VII et XII, dans lesquelles le radical $-k-[E]-(o)_t$ est un radical au moins divalent issu d'un mono- ou polyéthylène glycol diamine choisi dans le groupe constitué par le diéthylène glycol diamine et le triéthylène glycol diamine.

Dans un mode de réalisation, le polymère anionique hydrophobisé selon l'invention est choisi parmi les polymères anioniques hydrophobisés de formules IV à VII et XII, dans lesquelles le radical $-k-[E]-(o)_t$ est un radical au moins divalent issu de l'éthylènediamine.

Dans un mode de réalisation, le polymère anionique hydrophobisé selon l'invention est choisi parmi les polymères anioniques hydrophobisés de formules IV à VII et XII, dans lesquelles la fonction o est une fonction ester.

Dans un mode de réalisation, le polymère anionique hydrophobisé selon l'invention est choisi parmi les polymères anioniques hydrophobisés de formules IV à VII et XII, dans lesquelles la fonction o est une fonction amide.

Dans un mode de réalisation, le polymère anionique hydrophobisé selon l'invention est choisi parmi les polymères anioniques hydrophobisés de formules IV à VII et XII, dans lesquelles la fonction o est une fonction carbamate.

Dans un mode de réalisation, le polymère anionique hydrophobisé selon l'invention est choisi parmi les polymères anioniques hydrophobisés de formules IV à VII et XII, dans lesquelles la fonction o est une fonction carbonate.

Dans un mode de réalisation, le polymère anionique hydrophobisé selon l'invention est choisi parmi les polymères anioniques hydrophobisés de formules IV à VII et XII, dans lesquelles le groupe -[Hy] est un groupe alkyle issu d'un alcool hydrophobe présentant un LogP supérieur ou égal à 2.

Dans un mode de réalisation, le polymère anionique hydrophobisé selon l'invention est choisi parmi les polymères anioniques hydrophobisés de formules IV à VII et XII, dans lesquelles le groupe -[Hy] est un groupe alkyle issu d'un alcool hydrophobe, insaturé et/ou saturé, ramifié ou non ramifié comprenant de 8 à 30 carbones.

Dans un mode de réalisation, le polymère anionique hydrophobisé selon l'invention est choisi parmi les polymères anioniques hydrophobisés de formules IV à VII et XII, dans lesquelles le groupe -[Hy] est un groupe alkyle issu d'un alcool hydrophobe choisi dans le groupe constitué par l'octanol, le décanol, le dodécanol (alcool laurylique), le tétradécanol (alcool myristilique), l'hexadécanol (alcool cétylique), l'alcool stéarylique, l'alcool cétéarylique et l'alcool oléylique.

Dans un mode de réalisation, le polymère anionique hydrophobisé selon l'invention est choisi parmi les polymères anioniques hydrophobisés de formules IV à VII et XII, dans lesquelles le groupe -[Hy] est un groupe issu d'un stérol.

Dans un mode de réalisation, le polymère anionique hydrophobisé selon l'invention est choisi parmi les polymères anioniques hydrophobisés de formules IV à VII et XII, dans lesquelles le groupe -[Hy] est un groupe issu d'un stérol, choisi dans le groupe constitué par le cholestérol et ses dérivés.

Dans un mode de réalisation, le polymère anionique hydrophobisé selon l'invention est choisi parmi les polymères anioniques hydrophobisés de formules IV à VII et XII, dans lesquelles le groupe -[Hy] est un groupe issu du cholestérol.

Dans un mode de réalisation, le polymère anionique hydrophobisé selon l'invention est choisi parmi les polymères anioniques hydrophobisés de formules IV à VII et XII, dans lesquelles le groupe -[Hy] est un groupe issu d'un tocophérol.

Dans un mode de réalisation, le polymère anionique hydrophobisé selon l'invention est choisi parmi les polymères anioniques hydrophobisés de formules IV à VII et XII, dans lesquelles le groupe -[Hy] est un groupe issu d'un dérivé du tocophérol, choisi parmi le racémique, l'isomère L ou l'isomère D de l'a-tocophérol.

Dans un mode de réalisation, le polymère anionique hydrophobisé selon l'invention est choisi parmi les polymères anioniques hydrophobisés de formules IV à VII et XII, dans lesquelles le groupe -[Hy] est un groupe issu du DL-$\alpha$-tocophérol.

Dans un mode de réalisation, le polymère anionique hydrophobisé selon l'invention est choisi parmi les polymères anioniques hydrophobisés de formules IV à VII et XII, dans lesquelles le groupe -[Hy] est un groupe issu du menthol.

Dans un mode de réalisation, le polymère anionique hydrophobisé selon l'invention est choisi parmi les polymères anioniques hydrophobisés de formules IV à VII et XII, dans lesquelles le groupe -[Hy] est un groupe issu du menthol ou de ses dérivés, choisi parmi le racémique, l'isomère L ou l'isomère D du menthol.

Dans un mode de réalisation, le polymère anionique hydrophobisé selon l'invention est choisi parmi les polymères anioniques hydrophobisés de formules IV à VII et XII, dans lesquelles le groupe -[Hy] est un groupe alkyle issu d'un acide hydrophobe.

Dans un mode de réalisation, le polymère anionique hydrophobisé selon l'invention est choisi parmi les polymères anioniques hydrophobisés de formules IV à VII et XII, dans lesquelles le groupe -[Hy] est un groupe alkyle issu d'un acide hydrophobe présentant un LogP supérieur ou égal à 2.

Dans un mode de réalisation, le polymère anionique hydrophobisé selon l'invention est choisi parmi les polymères anioniques hydrophobisés de formules IV à VII et XII, dans lesquelles le groupe -[Hy] est un groupe alkyle issu d'un acide hydrophobe linéaire, choisi dans le groupe constitué par l'acide dodécanoïque, l'acide tétradécanoïque et l'acide hexadécanoïque.

Dans un mode de réalisation, le polymère anionique hydrophobisé selon l'invention est choisi parmi les polymères anioniques hydrophobisés de formules IV à VII et XII, dans lesquelles le groupe -[Hy] est un groupe alkyle issu d'un acide gras.

Dans un mode de réalisation, le polymère anionique hydrophobisé selon l'invention est choisi parmi les polymères anioniques hydrophobisés de formules IV à VII et XII, dans lesquelles le groupe -[Hy] est un groupe alkyle issu d'un acide gras choisi dans le groupe constitué par les acides constitués d'une chaîne alkyle insaturée ou saturée, ramifiée ou non ramifiée, comprenant de 8 à 30 carbones.

Dans un mode de réalisation, le polymère anionique hydrophobisé selon l'invention est choisi parmi les polymères anioniques hydrophobisés de formules IV à VII et XII, dans lesquelles le groupe -[Hy] est un groupe alkyle issu d'un acide gras choisi dans le groupe constitué par les acides gras linéaires.

Dans un mode de réalisation, le polymère anionique hydrophobisé selon l'invention est choisi parmi les polymères anioniques hydrophobisés de formules IV à VII et XII, dans lesquelles le groupe -[Hy] est un groupe alkyle issu d'un acide gras linéaire saturé choisi dans le groupe constitué par l'acide caprylique (acide octanoïque), l'acide nonanoïque, l'acide caprique (acide décanoïque), l'acide undécanoïque, l'acide laurique (acide dodécanoïque), l'acide myristique (tétradécanoïque), l'acide palmitique (hexadécanoïque), l'acide stéarique (octadécanoïque), l'acide arachidique (eicosanoïque), l'acide béhénique (docosanoïque), l'acide tricosanoïque, l'acide lignocérique (tétracosanoïque), l'acide

heptacosanoïque, l'acide octacosanoïque et l'acide mélissique (tricontanoïque).

Dans un mode de réalisation, le polymère anionique hydrophobisé selon l'invention est choisi parmi les polymères anioniques hydrophobisés de formules IV à VII et XII, dans lesquelles le groupe -[Hy] est un groupe alkyle issu d'un acide gras insaturé.

Dans un mode de réalisation, le polymère anionique hydrophobisé selon l'invention est choisi parmi les polymères anioniques hydrophobisés de formules IV à VII et XII, dans lesquelles le groupe -[Hy] est un groupe alkyle issu d'un acide gras insaturé choisi dans le groupe constitué par l'acide myristoléique ((Z)-tétradéc-9-énoïque), l'acide palmitoléique ((Z)-hexadéc-9-énoïque), l'acide oléique ((Z)-octadéc-9-énoïque), l'acide élaidique ((E)-octadéc-9-énoïque), l'acide linoléique ((9Z,12Z)-octadéca-9,12-diénoïque), l'acide alpha-linoléique ((9Z,12Z,15Z)-octadéca-9,12,15-triénoïque), l'acide arachidonique ((5Z,8Z,11Z,14Z)-octadéca-5,8,11,14-tétraénoïque), l'acide eicosapentaenoïque ((5Z,8Z,11Z,14Z,17Z)-eicosa-5,8,11,14,17-pentaénoïque), l'acide erucique (13-docoénoïque) et l'acide docosahexaenoïque ((4Z,7Z,10Z,13Z,16Z,19Z)-docosa-4,7,10,13,16,19-hexaénoïque).

Dans un mode de réalisation, le polymère anionique hydrophobisé selon l'invention est choisi parmi les polymères anioniques hydrophobisés de formules IV à VII et XII, dans lesquelles le groupe -[Hy] est un groupe alkyle issu d'un acide de la bile et ses dérivés.

Dans un mode de réalisation, le polymère anionique hydrophobisé selon l'invention est choisi parmi les polymères anioniques hydrophobisés de formules IV à VII et XII, dans lesquelles le groupe -[Hy] est un groupe alkyle issu d'un acide de la bile et ses dérivés choisi dans le groupe constitué par l'acide cholique, l'acide déhydrocholique, l'acide désoxycholique et l'acide chénodésoxycholique.

Dans un mode de réalisation, le polymère anionique hydrophobisé selon l'invention est choisi parmi les polymères anioniques hydrophobisés de formule V, dans laquelle -R' est soit un groupement -OH, soit un radical -f-[A]-COOH, soit un radical -*g*-[B]-*k*-[D], *f* étant une fonction éther, le radical -[A]- étant un radical $-CH_2-$, g étant une fonction éther, le radical -[B]- étant un radical $-CH_2-$, k étant une fonction amide, -[D] étant un radical -[E]-o-Hy, p et t = 1, le radical -[E]- étant issu de la leucine, o étant une fonction ester et Hy étant issu du cholestérol.

Les précurseurs des polymères anioniques hydrophobisés de formule I, dans laquelle $-R_2$, $-R_4$ et $-R_6$ sont des radicaux-$CH_2R'$, ou de formule IV à VII et XII peuvent être obtenus par un procédé tel que celui décrit dans Biomacromolecules, 2005, 6, 2659-2670. Ce procédé peut conduire à des polymères comportant des motifs autres que celui répété dans la formule I, dans laquelle $-R_2$, $-R_4$ et $-R_6$ sont des radicaux-$CH_2R'$, notamment des cycles hémiacétal, par exemple comme décrit dans la publication Carbohydrate Research 1978, 64, 189-197.

Ainsi, selon un mode de réalisation, les polymères anioniques hydrophobisés selon l'invention comprennent au moins 75% de leur unités de répétition sous la forme de celle définie dans la formule I, dans laquelle $-R_2$, $-R_4$ et $-R_6$ sont des radicaux-$CH_2R'$, ou dans les formules IV à VII et XII.

Selon un mode de réalisation, les polymères anioniques hydrophobisés selon l'invention comprennent au moins 85% de leur unités de répétition sous la forme de celle définie dans la formule I, dans laquelle $-R_2$, $-R_4$ et $-R_6$ sont des radicaux-$CH_2R'$, ou dans les formules IV à VII et XII.

Selon un mode de réalisation, les polymères anioniques hydrophobisés selon l'invention comprennent au moins 95% de leur unités de répétition sous la forme de celle définie dans la formule I, dans laquelle $-R_2$, $-R_4$ et $-R_6$ sont des radicaux-$CH_2R'$, ou dans les formules IV à VII et XII.

Selon un mode de réalisation, les polymères anioniques hydrophobisés selon l'invention comprennent au moins 98% de leur unités de répétition sous la forme de celle définie dans la formule I, dans laquelle $-R_2$, $-R_4$ et $-R_6$ sont des radicaux-$CH_2R'$, ou dans les formules IV à VII et XII.

Selon un mode de réalisation, les polymères anioniques hydrophobisés selon l'invention comprennent100% de leur unités de répétition sous la forme de celle définie dans la formule I, dans laquelle $-R_2$, $-R_4$ et $-R_6$ sont des radicaux-$CH_2R'$, ou dans les formules IV à VII et XII.

Selon un mode de réalisation, le polymère anionique hydrophobisé selon l'invention est choisi parmi les polymères anioniques hydrophobisés de formule V présentant une masse molaire moyenne en poids allant de 2 à 40 kg/mol.

Selon un mode de réalisation, le polymère anionique hydrophobisé selon l'invention est choisi parmi les polymères anioniques hydrophobisés de formule V présentant une masse molaire moyenne en poids allant de 2 à 20 kg/mol.

Selon un mode de réalisation, le polymère anionique hydrophobisé selon l'invention est choisi parmi les polymères anioniques hydrophobisés de formule V présentant une masse molaire moyenne en poids allant de 2 à 12 kg/mol.

Dans un mode de réalisation particulier, le polymère anionique hydrophobisé selon l'invention est choisi parmi les polymères anioniques hydrophobisés de formule V répondant aux conditions suivantes :

lorsque -*g*-[B]-(*k*-[D])$_p$ comporte une chaine Hy et que Hy est un alkyle en C8 à C15, alors le produit entre le degré de substitution en radicaux hydrophobes et le degré moyen de polymérisation (n) est supérieur ou égal à 2

- lorsque -*g*-[B]-(*k*-[D])$_p$ comporte une chaine Hy et que Hy est un alkyle en C16 à C20, alors le produit entre le degré de substitution en radicaux hydrophobes et le degré moyen de polymérisation (n) est supérieur ou égal à 1
- lorsque -*g*-[B]-(*k*-[D])$_p$ comporte deux chaines Hy et que Hy est un alkyle en C8 à C9, alors le produit du degré de

substitution en radicaux hydrophobes et du degré moyen de polymérisation (n) est supérieur ou égal à 2,
• lorsque -*g*-[B]-(*k*-[D])$_p$ comporte deux chaines Hy et que Hy est un alkyle en C10 à C16, alors le produit du degré de substitution en radicaux hydrophobes et du degré moyen de polymérisation (n) est supérieur ou égal à 0,2.

Selon un mode de réalisation, le polymère anionique hydrophobisé selon l'invention est choisi parmi les polymères anioniques hydrophobisés de formule V dans laquelle le radical -f-[A]-COOH est choisi dans le groupe constitué par les radicaux suivants, *f* ayant la signification donnée ci-dessus :

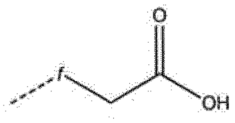
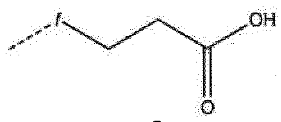
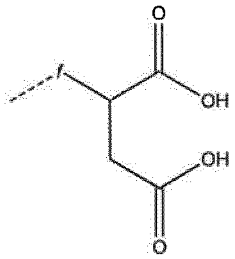
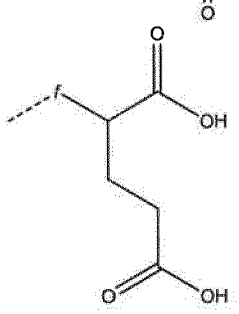

ou leur sels de cations alcalins choisis dans le groupe constitué de $Na^+$ et $K^+$.
Dans un mode de réalisation, le polymère anionique hydrophobisé selon l'invention est choisi parmi les polymères anioniques hydrophobisés de formule V, dans lesquelles le radical -[A]- est un radical $-CH_2-$.
Dans un mode de réalisation, le polymère anionique hydrophobisé selon l'invention est choisi parmi les polymères anioniques hydrophobisés de formule V, dans lesquelles la fonction *f* est une fonction éther.
Dans un mode de réalisation, le polymère anionique hydrophobisé selon l'invention est choisi parmi les polymères anioniques hydrophobisés de formule V, dans laquelle le radical -*g*-[B]-*k*-[D] est choisi dans le groupe constitué par les radicaux suivants ; *g, k* et-[D] ayant les significations données ci-dessus :

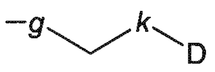
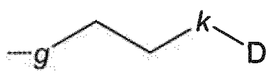
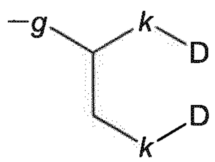
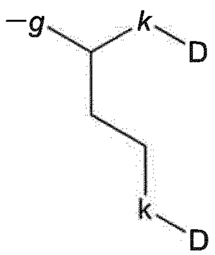

Dans un mode de réalisation, le polymère anionique hydrophobisé selon l'invention est choisi parmi les polymères anioniques hydrophobisés de formule V, dans laquelle le radical -[B]- est un radical $-CH_2-$.
Dans un mode de réalisation, le polymère anionique hydrophobisé selon l'invention est choisi parmi les polymères anioniques hydrophobisés de formule V, dans laquelle la fonction g est une fonction éther.
Dans un mode de réalisation, le polymère anionique hydrophobisé selon l'invention est choisi parmi les polymères anioniques hydrophobisés de formule V, dans laquelle la fonction k est une fonction amide.
Dans un mode de réalisation, le polymère anionique hydrophobisé selon l'invention est choisi parmi les polymères anioniques hydrophobisés de formule V, dans laquelle le radical -*k*-[E]-(*o*)$_t$ est un radical au moins divalent issu d'un acide aminé.
Dans un mode de réalisation, le polymère anionique hydrophobisé selon l'invention est choisi parmi les polymères anioniques hydrophobisés de formule V, dans laquelle le radical -*k*-[E]-(*o*)$_t$ est un radical au moins divalent issu d'un acide alpha aminé.

Dans un mode de réalisation, le polymère anionique hydrophobisé selon l'invention est choisi parmi les polymères anioniques hydrophobisés de formule V, dans laquelle le radical -*k*-[E]-$(o)_t$ est un radical au moins divalent issu d'un acide alpha aminé naturel choisi dans le groupe constitué par la glycine, la leucine, la phénylalanine, la lysine, l'isoleucine, l'alanine, la valine, l'acide aspartique et l'acide glutamique, sous leurs formes L, D ou racémique.

Dans un mode de réalisation, le polymère anionique hydrophobisé selon l'invention est choisi parmi les polymères anioniques hydrophobisés de formule V, dans laquelle le radical -*k*-[E]-$(o)_t$ est un radical au moins divalent issu d'un mono- ou polyéthylène glycol amine choisi dans le groupe constitué par l'éthanolamine, le diéthylène glycol amine et le triéthylène glycol amine.

Dans un mode de réalisation, le polymère anionique hydrophobisé selon l'invention est choisi parmi les polymères anioniques hydrophobisés de formule V, dans laquelle le radical -*k*-[E]-$(o)_t$ est un radical au moins divalent issu de l'éthylènediamine.

Dans un mode de réalisation, le polymère anionique hydrophobisé selon l'invention est choisi parmi les polymères anioniques hydrophobisés de formule V, dans laquelle la fonction o est une fonction ester.

Dans un mode de réalisation, le polymère anionique hydrophobisé selon l'invention est choisi parmi les polymères anioniques hydrophobisés de formule V, dans laquelle la fonction o est une fonction amide.

Dans un mode de réalisation, le polymère anionique hydrophobisé selon l'invention est choisi parmi les polymères anioniques hydrophobisés de formule V, dans laquelle la fonction o est une fonction carbamate.

Dans un mode de réalisation, le polymère anionique hydrophobisé selon l'invention est choisi parmi les polymères anioniques hydrophobisés de formule V, dans laquelle le groupe -[Hy] est un groupe alkyle issu d'un alcool hydrophobe présentant un LogP supérieur ou égal à 2.

Dans un mode de réalisation, le polymère anionique hydrophobisé selon l'invention est choisi parmi les polymères anioniques hydrophobisés de formule V, dans laquelle le groupe -[Hy] est un groupe alkyle issu d'un alcool hydrophobe, insaturé et/ou saturé, ramifié ou non ramifié comprenant de 8 à 30 carbones.

Dans un mode de réalisation, le polymère anionique hydrophobisé selon l'invention est choisi parmi les polymères anioniques hydrophobisés de formule V, dans laquelle le groupe -[Hy] est un groupe alkyle issu d'un alcool hydrophobe choisi dans le groupe constitué par l'octanol, le décanol, le dodécanol (alcool laurylique), le tétradécanol (alcool myristilique), l'hexadécanol (alcool cétylique), l'alcool stéarylique, l'alcool cétéarylique et l'alcool oléylique.

Dans un mode de réalisation, le polymère anionique hydrophobisé selon l'invention est choisi parmi les polymères anioniques hydrophobisés de formule V, dans laquelle le groupe -[Hy] est un groupe issu d'un stérol.

Dans un mode de réalisation, le polymère anionique hydrophobisé selon l'invention est choisi parmi les polymères anioniques hydrophobisés de formule V, dans laquelle le groupe -[Hy] est un groupe issu d'un stérol, choisi dans le groupe constitué par le cholestérol et ses dérivés.

Dans un mode de réalisation, le polymère anionique hydrophobisé selon l'invention est choisi parmi les polymères anioniques hydrophobisés de formule V, dans laquelle le groupe -[Hy] est un groupe issu du cholestérol.

Dans un mode de réalisation, le polymère anionique hydrophobisé selon l'invention est choisi parmi les polymères anioniques hydrophobisés de formule V, dans laquelle le groupe -[Hy] est un groupe issu d'un tocophérol.

Dans un mode de réalisation, le polymère anionique hydrophobisé selon l'invention est choisi parmi les polymères anioniques hydrophobisés de formule V, dans laquelle le groupe -[Hy] est un groupe issu d'un dérivé du tocophérol, choisi parmi le racémique, l'isomère L ou l'isomère D de l'a-tocophérol.

Dans un mode de réalisation, le polymère anionique hydrophobisé selon l'invention est choisi parmi les polymères anioniques hydrophobisés de formule V, dans laquelle le groupe -[Hy] est un groupe issu du DL-$\alpha$-tocophérol.

Dans un mode de réalisation, le polymère anionique hydrophobisé selon l'invention est choisi parmi les polymères anioniques hydrophobisés de formule V, dans laquelle le groupe -[Hy] est un groupe issu du menthol.

Dans un mode de réalisation, le polymère anionique hydrophobisé selon l'invention est choisi parmi les polymères anioniques hydrophobisés de formule V, dans laquelle le groupe -[Hy] est un groupe issu du menthol ou de ses dérivés, choisi parmi le racémique, l'isomère L ou l'isomère D du menthol.

Dans un mode de réalisation, le polymère anionique hydrophobisé selon l'invention est choisi parmi les polymères anioniques hydrophobisés de formule V, dans laquelle le groupe -[Hy] est un groupe alkyle issu d'un acide hydrophobe.

Dans un mode de réalisation, le polymère anionique hydrophobisé selon l'invention est choisi parmi les polymères anioniques hydrophobisés de formule V, dans laquelle le groupe -[Hy] est un groupe alkyle issu d'un acide hydrophobe présentant un LogP supérieur ou égal à 2.

Dans un mode de réalisation, le polymère anionique hydrophobisé selon l'invention est choisi parmi les polymères anioniques hydrophobisés de formule V, dans laquelle le groupe -[Hy] est un groupe alkyle issu d'un acide hydrophobe linéaire, choisi dans le groupe constitué par l'acide dodécanoïque, l'acide tétradécanoïque et l'acide hexadécanoïque.

Dans un mode de réalisation, le polymère anionique hydrophobisé selon l'invention est choisi parmi les polymères anioniques hydrophobisés de formule V, dans laquelle le groupe -[Hy] est un groupe alkyle issu d'un acide gras.

Dans un mode de réalisation, le polymère anionique hydrophobisé selon l'invention est choisi parmi les polymères anioniques hydrophobisés de formule V, dans laquelle le groupe -[Hy] est un groupe alkyle issu d'un acide gras choisi dans le groupe constitué par les acides constitués d'une chaîne alkyle insaturée ou saturée, ramifiée ou non ramifiée, comprenant de 8 à 30 carbones.

Dans un mode de réalisation, le polymère anionique hydrophobisé selon l'invention est choisi parmi les polymères anioniques hydrophobisés de formule V, dans laquelle le groupe -[Hy] est un groupe alkyle issu d'un acide gras choisi dans le groupe constitué par les acides gras linéaires.

Dans un mode de réalisation, le polymère anionique hydrophobisé selon l'invention est choisi parmi les polymères anioniques hydrophobisés de formule V, dans laquelle le groupe -[Hy] est un groupe alkyle issu d'un acide gras linéaire saturé choisi dans le groupe constitué par l'acide caprylique (acide octanoïque), l'acide nonanoïque, l'acide caprique (acide décanoïque), l'acide undécanoïque, l'acide laurique (acide dodécanoïque), l'acide myristique (tétradécanoïque), l'acide palmitique (hexadécanoïque), l'acide stéarique (octadécanoïque), l'acide arachidique (eicosanoïque), l'acide béhénique (docosanoïque), l'acide tricosanoïque, l'acide lignocérique (tétracosanoïque), l'acide heptacosanoïque, l'acide octacosanoïque et l'acide mélissique (tricontanoïque).

Dans un mode de réalisation, le polymère anionique hydrophobisé selon l'invention est choisi parmi les polymères anioniques hydrophobisés de formule V, dans laquelle le groupe -[Hy] est un groupe alkyle issu d'un acide gras insaturé.

Dans un mode de réalisation, le polymère anionique hydrophobisé selon l'invention est choisi parmi les polymères anioniques hydrophobisés de formule V, dans laquelle le groupe -[Hy] est un groupe alkyle issu d'un acide gras insaturé choisi dans le groupe constitué par l'acide myristoléique ((Z)-tétradéc-9-énoïque), l'acide palmitoléique ((Z)-hexadéc-9-énoïque), l'acide oléique ((Z)-octadéc-9-énoïque), l'acide élaidique ((E)-octadéc-9-énoïque), l'acide linoléique ((9Z,12Z)-octadéca-9,12-diénoïque), l'acide alpha-linoléique ((9Z,12Z,15Z)-octadéca-9,12,15-triénoïque), l'acide arachidonique ((5Z,8Z,11Z,14Z)-octadéca-5,8,11,14-tétraénoïque), l'acide eicosapentaenoïque ((5Z,8Z,11Z,14Z,17Z)-eicosa-5,8,11,14,17-pentaénoïque), l'acide erucique (13-docoénoïque) et l'acide docosahexaenoïque ((4Z,7Z,10Z,13Z,16Z,19Z)-docosa-4,7,10 ,13,16,19-hexaénoïque).

Dans un mode de réalisation, le polymère anionique hydrophobisé selon l'invention est choisi parmi les polymères anioniques hydrophobisés de formule V, dans laquelle le groupe -[Hy] est un groupe alkyle issu d'un acide de la bile et ses dérivés.

Dans un mode de réalisation, le polymère anionique hydrophobisé selon l'invention est choisi parmi les polymères anioniques hydrophobisés de formule V, dans laquelle le groupe -[Hy] est un groupe alkyle issu d'un acide de la bile et ses dérivés choisi dans le groupe constitué par l'acide cholique, l'acide déhydrocholique, l'acide désoxycholique et l'acide chénodésoxycholique.

Dans un mode de réalisation, le polymère anionique hydrophobisé selon l'invention est choisi parmi les polymères anioniques hydrophobisés de formule V, dans laquelle -R' est soit un groupement -OH, soit un radical -*f*-[A]-COOH, soit un radical -*g*-[B]-*k*-[D], *f* étant une fonction éther, le radical -[A]- étant un radical $-CH_2-$, *g* étant une fonction éther, le radical -[B]- étant un radical $-CH_2-$, k étant une fonction amide, -[D] étant un radical -[E]-o-Hy, p et t = 1, le radical -*k*-[E]-($o$)$_t$ étant issu d'un acide aminé, *o* étant une fonction ester et Hy étant issu d'un stérol ou d'un alcool gras, en particulier :

- le radical -*k*-[E]-($o$)$_t$ étant issu de la leucine et Hy étant issu du cholestérol,
- le radical -*k*-[E]-($o$)$_t$ étant issu de la glycine et Hy étant issu du dodécanol,
- le radical -*k*-[E]-($o$)$_t$ étant issu de la leucine et Hy étant issu du tocophérol, et
- le radical -*k*-[E]-($o$)$_t$ étant issu de la phénylalanine et Hy étant issu de l'octanol, et
- le radical -*k*-[E]-($o$)$_t$ étant issu de la phénylalanine et Hy étant issu du 3,7-diméthyloctan-1-ol.

Dans un mode de réalisation, le polymère anionique hydrophobisé selon l'invention est choisi parmi les polymères anioniques hydrophobisés de formule V, dans laquelle -R' est soit un groupement -OH, soit un radical -*f*-[A]-COOH, soit un radical -*g*-[B]-*k*-[D], *f* étant une fonction éther, le radical -[A]- étant un radical $-CH_2-$, g étant une fonction éther, le radical -[B]- étant un radical $-CH_2-$, *k* étant une fonction amide, -[D] étant un radical -[E]-($o$-Hy)$_2$, p = 1 et t = 2, le radical -*k*-[E]-($o$)$_t$ étant issu d'un acide aminé, *o* étant une fonction ester et Hy étant issu d'un alcool gras, en particulier le radical -*k*-[E]-($o$)$_t$ étant issu de l'acide aspartique et Hy étant issu du docécanol ou du décanol.

Dans un mode de réalisation, le polymère anionique hydrophobisé selon l'invention est choisi parmi les polymères anioniques hydrophobisés de formule V, dans laquelle -R' est soit un groupement -OH, soit un radical -*f*-[A]-COOH, soit un radical -*g*-[B]-*k*-[D], *f* étant une fonction éther, le radical -[A]- étant un radical $-CH_2-$, *g* étant une fonction éther, le radical -[B]- étant un radical $-CH_2-$, *k* étant une fonction amide, -[D] étant un radical -[E]-o-Hy, p et t = 1, le radical -*k*-[E]-($o$)$_t$ étant issu d'une diamine, *o* étant une fonction amide et Hy étant issu d'un acide gras, en particulier le radical -*k*-[E]-($o$)$_t$ étant issu de l'éthylène diamine et Hy étant issu de l'acide dodécanoïque.

Dans un mode de réalisation, le polymère anionique hydrophobisé selon l'invention est choisi parmi les polymères anioniques hydrophobisés de formule V, dans laquelle -R' est soit un groupement -OH, soit un radical -*f*-[A]-COOH, soit un radical -*g*-[B]-*k*-[D], *f* étant une fonction éther, le radical -[A]- étant un radical -$CH_2$-, *g* étant une fonction éther, le radical -[B]- étant un radical -$CH_2$-, *k* étant une fonction amide, -[D] étant un radical -[E]-o-Hy, p et t = 1, le radical -*k*-[E]-$(o)_t$ étant issu d'une diamine, *o* étant une fonction carbamate et Hy étant issu d'un stérol, en particulier le radical -[E]- étant issu de l'éthylène diamine et Hy étant issu du cholestérol.

Dans un mode de réalisation, le polymère anionique hydrophobisé selon l'invention est choisi parmi les polymères anioniques hydrophobisés de formule V, dans laquelle -R' est soit un groupement -OH, soit un radical -*f*-[A]-COOH, soit un radical -*g*-[B]-*k*-[D], *f* étant une fonction éther, le radical -[A]- étant un radical -$CH_2$-, *g* étant une fonction éther, le radical -[B]- étant un radical -$CH_2$-, *k* étant une fonction amide, -[D] étant un radical -[E]-o-Hy, p et t = 1, le radical -*k*-[E]-$(o)_t$ étant issu d'un amino-alcool, o étant une fonction ester et Hy étant issu d'un alcool gras, en particulier le radical -*k*-[E]-$(o)_t$ étant issu du 2-(2-amino-éthoxy)éthanol et Hy étant issu du dodécanol.

Dans un mode de réalisation, la composition selon l'invention est caractérisée en ce que le polymère anionique hydrophobisé est choisi parmi les polymères anioniques hydrophobisés de formule I, dans laquelle -$R_4$ et -$R_6$ forment un cycle à six chaînons et -$R_4$-$R_6$- = -$(CH(R'))_2$-.

Dans un mode de réalisation, la composition selon l'invention est caractérisée en ce que le polymère anionique hydrophobisé de formule I, dans laquelle -$R_4$ et -$R_6$ forment un cycle à six chaînons et -$R_4$-$R_6$- = -$(CH(R'))_2$-, est choisi parmi les polysaccharides anioniques hydrophobisés de formule XI :

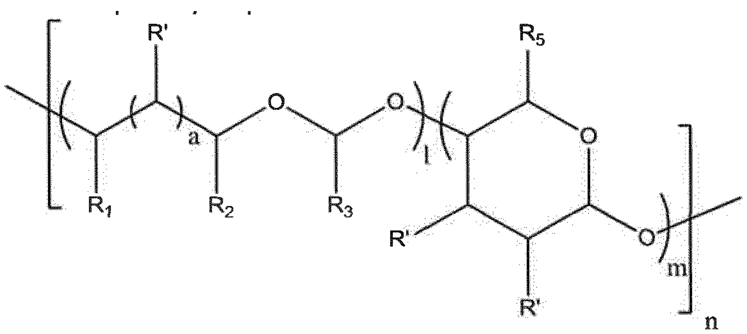

Formule XI

dans laquelle,

- l = 0 ou 1,
- m = 0, 1 ou 2,
- a = 0 ou 1,
- n étant le degré de polymérisation, compris entre 3 et 1000, et,
- -$R_1$ est un hydrogène,
- -$R_2$ est un radical -$CH_2R'$,
- -$R_5$ est soit un groupe -COOH, soit un radical -$CH_2R'$, soit un radical -k-[D], ou,
- -$R_2$ et -$R_3$ forment un cycle à six chaînons et -$R_2$-$R_3$- = -$(CH(R'))_3$- et -$R_1$ est un hydrogène, et,
- -R', n et -k-[D] tels que définis ci-dessus,

et, le degré de substitution en charges carboxylates est le nombre moyen de charges carboxylates par monomère divisé par (l+m) et est supérieur ou égal à 0,4, et, le degré de substitution en radicaux hydrophobes est le nombre moyen de radicaux hydrophobes par monomère divisé par (l+m) et est inférieur ou égal à 0,5.

Dans un mode de réalisation, la composition selon l'invention est caractérisée en ce que le polymère anionique hydrophobisé est un polysaccharide de formule XI, dans laquelle le ou les cycles à six chaînons identiques ou différents sont des unités saccharidiques choisies dans le groupe constitué des hexoses, des acides uroniques, et des N-acétylhexoamines.

Dans un mode de réalisation, la composition selon l'invention est caractérisée en ce que les unités saccharidiques sont choisies dans le groupe constitué par les hexoses sous forme cyclique.

Dans un mode de réalisation, la composition selon l'invention est caractérisée en ce que les unités saccharidiques sont choisies dans le groupe constitué par le fructose, le sorbose, le tagatose, le psicose, le glucose, le mannose, le galactose, l'allose, l'altrose, le talose, l'idose, le gulose, le fucose, le fuculose, le rhamnose.

Dans un mode de réalisation, la composition selon l'invention est caractérisée en ce que les unités saccharidiques sont choisies dans le groupe constitué par les acides uroniques.

Dans un mode de réalisation, la composition selon l'invention est caractérisée en ce que les unités saccharidiques sont choisies dans le groupe constitué par l'acide glucuronique, l'acide iduronique et l'acide galacturonique.

Dans un mode de réalisation, la composition selon l'invention est caractérisée en ce que les unités saccharidiques sont choisies dans le groupe constitué par les N-acétylhexoamines.

Dans un mode de réalisation, la composition selon l'invention est caractérisée en ce que les unités saccharidiques sont choisies dans le groupe constitué par la N-acétylglucosamine, la N-acétylgalactosamine, la N-acétylmannosamine.

Dans un mode de réalisation, la composition selon l'invention est caractérisée en ce que ce que les polysaccharides de formule XI sont liés par des liaisons glycosidiques de type $\alpha$ et/ou $\beta$, identiques ou différentes.

Dans un mode de réalisation, la composition selon l'invention est caractérisée en ce que le polysaccharide anionique hydrophobisé de formule XI, dans laquelle, l = 0, m = 1, et $-R_5$ est un radical $-CH_2R'$, autrement exprimé, il est choisi parmi les celluloses et les celluloses hydrosolubles de formule VIII :

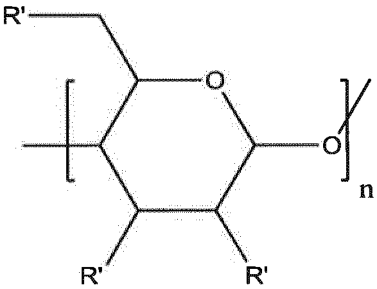

Formule VIII

- -R' tel que défini ci-dessus.

Dans un mode de réalisation, la composition selon l'invention est caractérisée en ce que le polysaccharide anionique hydrophobisé de formule XI, dans laquelle, l = 0, m = 2, - et $-R_5$ est soit un groupe -COOH, soit un radical -k-[D], autrement exprimé, il est choisi parmi les alginates de formule IX :

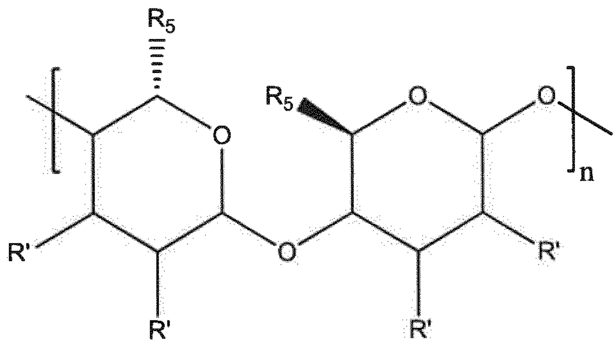

Formule IX

- -R', n et -k-[D] tels que définis ci-dessus.

Dans un mode de réalisation, la composition selon l'invention est caractérisée en ce que le polysaccharide anionique hydrophobisé de formule XI, dans laquelle, l = 1, m = 2, a = 0, $-R_1$ est un -H, $-R_2$ et $-R_3$ forment un cycle à six chaînons et $-R_2-R_3- = -(CH(R'))_3-$, et $-R_5$ est un radical $-CH_2R'$, autrement exprimé, il est choisi parmi les pullulanes de formule X :

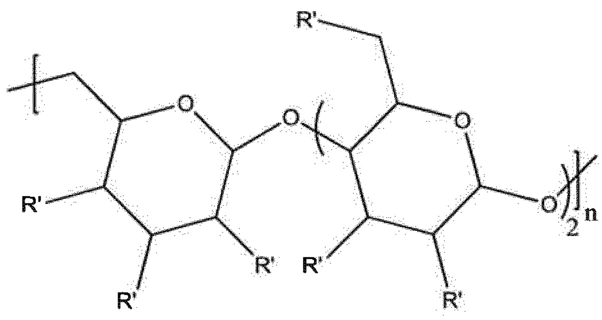

Formule X

- -R' tel que défini ci-dessus.

Dans un mode de réalisation, la composition selon l'invention est caractérisée en ce que le polymère anionique hydrophobisé est choisi parmi les polymères anioniques hydrophobisés de formules VIII à XI, dans lesquelles le radical - f-[A]-COOH est choisi parmi les radicaux de formule II suivante :

-*f*-[A]-COOH = 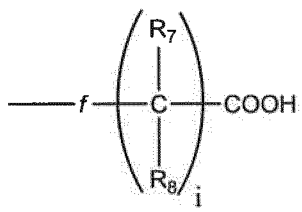

Formule II

dans laquelle :

- i supérieur ou égal à 1 et inférieur ou égal à 12, et,
- $-R_7$ et $-R_8$, identiques ou différents, sont choisis dans le groupe constitué par un atome d'hydrogène, un alkyle saturé ou insaturé, linéaire, ramifié ou cyclique en C1 à C6, un benzyle, un alkyle-aryle et comportant éventuellement des hétéroatomes choisis dans le groupe constitué par O, N et/ou S, ou des fonctions choisies dans le groupe constitué par les fonctions acide carboxylique, amine, alcool ou thiol.

Dans un mode de réalisation, la composition selon l'invention est caractérisée en ce que le polymère anionique hydrophobisé est choisi parmi les polymères anioniques hydrophobisés de formule VII à XI, dans lesquelles le radical -f-[A]-COOH est choisi dans le groupe constitué par les radicaux suivants, f ayant la signification donnée ci-dessus :

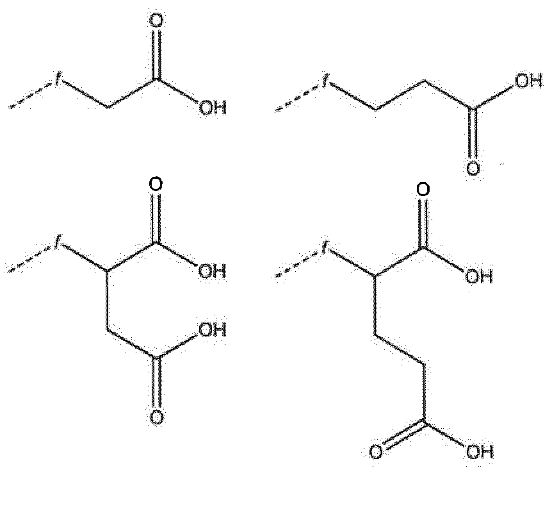

ou leur sels de cations alcalins choisis dans le groupe constitué de $Na^+$ et $K^+$.

Dans un mode de réalisation, la composition selon l'invention est caractérisée en ce que le polymère anionique hydrophobisé est choisi parmi les polymères anioniques hydrophobisés de formules VIII à XI, dans lesquelles le radical - [A]- est un radical $-CH_2-$.

Dans un mode de réalisation, la composition selon l'invention est caractérisée en ce que le polymère anionique hydrophobisé est choisi parmi les polymères anioniques hydrophobisés de formules VIII à XI, dans lesquelles le radical - f-[A]-COOH est issu d'un acide aminé.

Dans un mode de réalisation, la composition selon l'invention est caractérisée en ce que le polymère anionique hydrophobisé est choisi parmi les polymères anioniques hydrophobisés de formules VIII à XI, dans lesquelles le radical - f-[A]-COOH est issu de la glycine.

Dans un mode de réalisation, la composition selon l'invention est caractérisée en ce que le polymère anionique hydrophobisé est choisi parmi les polymères anioniques hydrophobisés de formules VIII à XI, dans lesquelles le radical - f-[A]-COOH est issu de l'acide aspartique.

Dans un mode de réalisation, la composition selon l'invention est caractérisée en ce que le polymère anionique hydrophobisé est choisi parmi les polymères anioniques hydrophobisés de formules VIII à XI, dans lesquelles le radical - f-[A]-COOH est issu de l'acide glutamique.

Dans un mode de réalisation, la composition selon l'invention est caractérisée en ce que le polymère anionique hydrophobisé est choisi parmi les polymères anioniques hydrophobisés de formules VIII à XI, dans lesquelles le radical - f-[A]-COOH est issu de l'acide succinique.

Dans un mode de réalisation, la composition selon l'invention est caractérisée en ce que le polymère anionique hydrophobisé est choisi parmi les polymères anioniques hydrophobisés de formules VIII à XI, dans lesquelles la fonction f est une fonction éther.

Dans un mode de réalisation, la composition selon l'invention est caractérisée en ce que le polymère anionique hydrophobisé est choisi parmi les polymères anioniques hydrophobisés de formules VIII à XI, dans lesquelles la fonction f est une fonction carbamate.

Dans un mode de réalisation, la composition selon l'invention est caractérisée en ce que le polymère anionique hydrophobisé est choisi parmi les polymères anioniques hydrophobisés de formules VIII à XI, dans lesquelles la fonction f est une fonction ester.

Dans un mode de réalisation, la composition selon l'invention est caractérisée en ce que le polymère anionique hydrophobisé est choisi parmi les polymères anioniques hydrophobisés de formules VIII à XI, dans lesquelles la fonction f est une fonction carbonate.

Dans un mode de réalisation, la composition selon l'invention est caractérisée en ce que le polymère anionique hydrophobisé est choisi parmi les polymères anioniques hydrophobisés de formules VIII à XI, dans lesquelles la fonction f est une fonction amide.

Dans un mode de réalisation, la composition selon l'invention est caractérisée en ce que le polymère anionique hydrophobisé est choisi parmi les polymères anioniques hydrophobisés de formules VIII à XI, dans lesquelles le radical - g-[B]- est choisi parmi les radicaux de formule III suivante :

-g-[B]- =

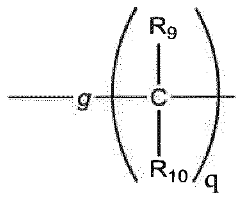

Formule III

dans laquelle :

- q supérieur ou égal à 1 et inférieur ou égal à 12, et,
- $-R_9$ et $-R_{10}$ identiques ou différents sont choisis dans le groupe constitué par un atome d'hydrogène, un alkyle saturé ou insaturé, linéaire, ramifié ou cyclique en C1 à C6, un benzyle, un alkyle-aryle et comportant éventuellement des hétéroatomes choisis dans le groupe constitué par O, N et/ou S, ou des fonctions choisies dans le groupe constitué par les fonctions acide carboxylique, amine, alcool ou thiol.

Dans un mode de réalisation, la composition selon l'invention est caractérisée en ce que le polymère anionique

hydrophobisé est choisi parmi les polymères anioniques hydrophobisés de formules VIII à XI, dans lesquelles le radical - g-[B]-k-[D] est choisi dans le groupe constitué par les radicaux suivants ; g, k et -[D] ayant les significations données ci-dessus :

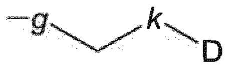

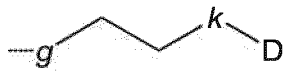

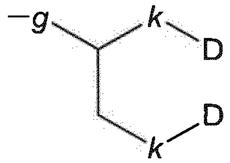

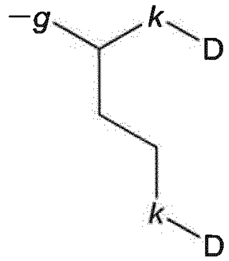

Dans un mode de réalisation, la composition selon l'invention est caractérisée en ce que le polymère anionique hydrophobisé est choisi parmi les polymères anioniques hydrophobisés de formules VIII à XI, dans lesquelles le radical - [B]- est un radical $-CH_2-$.

Dans un mode de réalisation, la composition selon l'invention est caractérisée en ce que le polymère anionique hydrophobisé est choisi parmi les polymères anioniques hydrophobisés de formules VIII à XI, dans lesquelles le radical - g-[B]-(k-[D])$_p$ est tel que -g-[B]-k- est issu d'un acide aminé.

Dans un mode de réalisation, la composition selon l'invention est caractérisée en ce que le polymère anionique hydrophobisé est choisi parmi les polymères anioniques hydrophobisés de formules VIII à XI, dans lesquelles le radical - g-[B]-(k-[D])$_p$ est tel que -g-[B]-k- est issu de la glycine.

Dans un mode de réalisation, la composition selon l'invention est caractérisée en ce que le polymère anionique hydrophobisé est choisi parmi les polymères anioniques hydrophobisés de formules VIII à XI, dans lesquelles le radical - g-[B]-(k-[D])$_p$ est tel que -g-[B]-k- est issu de l'acide aspartique.

Dans un mode de réalisation, la composition selon l'invention est caractérisée en ce que le polymère anionique hydrophobisé est choisi parmi les polymères anioniques hydrophobisés de formules VIII à XI, dans lesquelles le radical - g-[B]-(k-[D])$_p$ est tel que -g-[B]-k- est issu de l'acide glutamique.

Dans un mode de réalisation, la composition selon l'invention est caractérisée en ce que le polymère anionique hydrophobisé est choisi parmi les polymères anioniques hydrophobisés de formules VIII à XI, dans lesquelles le radical - g-[B]-(k-[D])$_p$ est tel que -g-[B]-k- est issu de l'acide succinique.

Dans un mode de réalisation, la composition selon l'invention est caractérisée en ce que le polymère anionique hydrophobisé est choisi parmi les polymères anioniques hydrophobisés de formules VIII à XI, dans lesquelles la fonction g est une fonction éther.

Dans un mode de réalisation, la composition selon l'invention est caractérisée en ce que le polymère anionique hydrophobisé est choisi parmi les polymères anioniques hydrophobisés de formules VIII à XI, dans lesquelles la fonction g est une fonction carbamate.

Dans un mode de réalisation, la composition selon l'invention est caractérisée en ce que le polymère anionique hydrophobisé est choisi parmi les polymères anioniques hydrophobisés de formules VIII à XI, dans lesquelles la fonction g est une fonction ester.

Dans un mode de réalisation, la composition selon l'invention est caractérisée en ce que le polymère anionique hydrophobisé est choisi parmi les polymères anioniques hydrophobisés de formules VIII à XI, dans lesquelles la fonction g est une fonction amine.

Dans un mode de réalisation, la composition selon l'invention est caractérisée en ce que le polymère anionique hydrophobisé est choisi parmi les polymères anioniques hydrophobisés de formules VIII à XI, dans lesquelles la fonction g est une fonction carbonate.

Dans un mode de réalisation, la composition selon l'invention est caractérisée en ce que le polymère anionique hydrophobisé est choisi parmi les polymères anioniques hydrophobisés de formules VIII à XI, dans lesquelles la fonction k est une fonction amide.

Dans un mode de réalisation, la composition selon l'invention est caractérisée en ce que le polymère anionique hydrophobisé est choisi parmi les polymères anioniques hydrophobisés de formules VIII à XI, dans lesquelles la fonction k est une fonction carbamate.

Dans un mode de réalisation, la composition selon l'invention est caractérisée en ce que le polymère anionique

hydrophobisé est choisi parmi les polymères anioniques hydrophobisés de formules VIII à XI, dans lesquelles la fonction k est une fonction ester.

Dans un mode de réalisation, la composition selon l'invention est caractérisée en ce que le polymère anionique hydrophobisé est choisi parmi les polymères anioniques hydrophobisés de formules VIII à XI, dans lesquelles le radical - *k*-[E]-($o$)$_t$ est un radical au moins divalent issu d'un acide aminé.

Dans un mode de réalisation, la composition selon l'invention est caractérisée en ce que le polymère anionique hydrophobisé est choisi parmi les polymères anioniques hydrophobisés de formules VIII à XI, dans lesquelles le radical - *k*-[E]-($o$)$_t$ est un radical au moins divalent issu d'un acide alpha aminé.

Dans un mode de réalisation, la composition selon l'invention est caractérisée en ce que le polymère anionique hydrophobisé est choisi parmi les polymères anioniques hydrophobisés de formules VIII à XI, dans lesquelles le radical - *k*-[E]-($o$)$_t$ est un radical au moins divalent issu d'un acide alpha aminé naturel choisi dans le groupe constitué par la glycine, la leucine, la phénylalanine, la lysine, l'isoleucine, l'alanine, la valine, l'acide aspartique et l'acide glutamique, sous leurs formes L, D ou racémique.

Dans un mode de réalisation, la composition selon l'invention est caractérisée en ce que le polymère anionique hydrophobisé est choisi parmi les polymères anioniques hydrophobisés de formules VIII à XI, dans lesquelles le radical - *k*-[E]-($o$)$_t$ est un radical au moins divalent issu d'un mono- ou polyéthylène glycol.

Dans un mode de réalisation, la composition selon l'invention est caractérisée en ce que le polymère anionique hydrophobisé est choisi parmi les polymères anioniques hydrophobisés de formules VIII à XI, dans lesquelles le radical - *k*-[E]-($o$)$_t$ est un radical au moins divalent issu de l'éthylène glycol.

Dans un mode de réalisation, la composition selon l'invention est caractérisée en ce que le polymère anionique hydrophobisé est choisi parmi les polymères anioniques hydrophobisés de formules VIII à XI, dans lesquelles le radical - *k*-[E]-($o$)$_t$ est un radical au moins divalent issu d'un polyéthylène glycol choisi dans le groupe constitué par le diéthylène glycol, le triéthylène glycol et le tétraéthylène glycol.

Dans un mode de réalisation, la composition selon l'invention est caractérisée en ce que le polymère anionique hydrophobisé est choisi parmi les polymères anioniques hydrophobisés de formules VIII à XI, dans lesquelles le radical - *k*-[E]-($o$)$_t$ est un radical au moins divalent issu d'un mono- ou polyéthylène glycol amine.

Dans un mode de réalisation, la composition selon l'invention est caractérisée en ce que le polymère anionique hydrophobisé est choisi parmi les polymères anioniques hydrophobisés de formules VIII à XI, dans lesquelles le radical - *k*-[E]-($o$)$_t$ est un radical au moins divalent issu d'un mono- ou polyéthylène glycol amine choisi dans le groupe constitué par l'éthanolamine, le diéthylène glycol amine et le triéthylène glycol amine.

Dans un mode de réalisation, la composition selon l'invention est caractérisée en ce que le polymère anionique hydrophobisé est choisi parmi les polymères anioniques hydrophobisés de formules VIII à XI, dans lesquelles le radical - *k*-[E]-($o$)$_t$ est un radical au moins divalent issu d'un mono- ou polyéthylène glycol diamine.

Dans un mode de réalisation, la composition selon l'invention est caractérisée en ce que le polymère anionique hydrophobisé est choisi parmi les polymères anioniques hydrophobisés de formules VIII à XI, dans lesquelles le radical - *k*-[E]-($o$)$_t$ est un radical au moins divalent issu d'un mono- ou polyéthylène glycol diamine choisi dans le groupe constitué par le diéthylène glycol diamine et le triéthylène glycol diamine.

Dans un mode de réalisation, la composition selon l'invention est caractérisée en ce que le polymère anionique hydrophobisé est choisi parmi les polymères anioniques hydrophobisés de formules VIII à XI, dans lesquelles le radical - *k*-[E]-($o$)$_t$ est un radical au moins divalent issu de l'éthylènediamine.

Dans un mode de réalisation, la composition selon l'invention est caractérisée en ce que le polymère anionique hydrophobisé est choisi parmi les polymères anioniques hydrophobisés de formules VIII à XI, dans lesquelles la fonction o est une fonction ester.

Dans un mode de réalisation, la composition selon l'invention est caractérisée en ce que le polymère anionique hydrophobisé est choisi parmi les polymères anioniques hydrophobisés de formules VIII à XI, dans lesquelles la fonction o est une fonction amide.

Dans un mode de réalisation, la composition selon l'invention est caractérisée en ce que le polymère anionique hydrophobisé est choisi parmi les polymères anioniques hydrophobisés de formules VIII à XI, dans lesquelles la fonction o est une fonction carbamate.

Dans un mode de réalisation, la composition selon l'invention est caractérisée en ce que le polymère anionique hydrophobisé est choisi parmi les polymères anioniques hydrophobisés de formules VIII à XI, dans lesquelles la fonction o est une fonction urée (carbamide).

Dans un mode de réalisation, la composition selon l'invention est caractérisée en ce que le polymère anionique hydrophobisé est choisi parmi les polymères anioniques hydrophobisés de formules VIII à XI, dans lesquelles le groupe - [Hy] est un groupe alkyle issu d'un alcool hydrophobe, insaturé et/ou saturé, ramifié ou non ramifié comprenant de 8 à 30 carbones.

Dans un mode de réalisation, la composition selon l'invention est caractérisée en ce que le polymère anionique hydrophobisé est choisi parmi les polymères anioniques hydrophobisés de formules VIII à XI, dans lesquelles le groupe

- [Hy] est un groupe alkyle issu d'un alcool hydrophobe choisi dans le groupe constitué par l'octanol, le décanol, le dodécanol (alcool laurylique), le tétradécanol (alcool myristilique), l'hexadécanol (alcool cétylique), l'alcool stéarylique, l'alcool cétéarylique et l'alcool oléylique.

Dans un mode de réalisation, la composition selon l'invention est caractérisée en ce que le polymère anionique hydrophobisé est choisi parmi les polymères anioniques hydrophobisés de formules VIII à XI, dans lesquelles le groupe - [Hy] est un groupe issu d'un stérol.

Dans un mode de réalisation, la composition selon l'invention est caractérisée en ce que le polymère anionique hydrophobisé est choisi parmi les polymères anioniques hydrophobisés de formules VIII à XI, dans lesquelles le groupe - [Hy] est un groupe issu d'un stérol, choisi dans le groupe constitué par le cholestérol et ses dérivés.

Dans un mode de réalisation, la composition selon l'invention est caractérisée en ce que le polymère anionique hydrophobisé est choisi parmi les polymères anioniques hydrophobisés de formules VIII à XI, dans lesquelles le groupe - [Hy] est un groupe issu du cholestérol.

Dans un mode de réalisation, la composition selon l'invention est caractérisée en ce que le polymère anionique hydrophobisé est choisi parmi les polymères anioniques hydrophobisés de formules VIII à XI, dans lesquelles le groupe - [Hy] est un groupe issu d'un tocophérol.

Dans un mode de réalisation, la composition selon l'invention est caractérisée en ce que le polymère anionique hydrophobisé est choisi parmi les polymères anioniques hydrophobisés de formules VIII à XI, dans lesquelles le groupe - [Hy] est un groupe issu d'un dérivé du tocophérol, choisi parmi le racémique, l'isomère L ou l'isomère D de l'α-tocophérol.

Dans un mode de réalisation, la composition selon l'invention est caractérisée en ce que le polymère anionique hydrophobisé est choisi parmi les polymères anioniques hydrophobisés de formules VIII à XI, dans lesquelles le groupe - [Hy] est un groupe issu du DL-α-tocophérol.

Dans un mode de réalisation, la composition selon l'invention est caractérisée en ce que le polymère anionique hydrophobisé est choisi parmi les polymères anioniques hydrophobisés de formules VIII à XI, dans lesquelles le groupe - [Hy] est un groupe issu du menthol.

Dans un mode de réalisation, la composition selon l'invention est caractérisée en ce que le polymère anionique hydrophobisé est choisi parmi les polymères anioniques hydrophobisés de formules VIII à XI, dans lesquelles le groupe - [Hy] est un groupe issu du menthol ou de ses dérivés, choisi parmi le racémique, l'isomère L ou l'isomère D du menthol.

Dans un mode de réalisation, la composition selon l'invention est caractérisée en ce que le polymère anionique hydrophobisé est choisi parmi les polymères anioniques hydrophobisés de formules VIII à XI, dans lesquelles le groupe - [Hy] est un groupe alkyle issu d'un acide hydrophobe.

Dans un mode de réalisation, la composition selon l'invention est caractérisée en ce que le polymère anionique hydrophobisé est choisi parmi les polymères anioniques hydrophobisés de formules VIII à XI, dans lesquelles le groupe - [Hy] est un groupe alkyle issu d'un acide hydrophobe linéaire, choisi dans le groupe constitué par l'acide dodécanoïque, l'acide tétradécanoïque et l'acide hexadécanoïque.

Dans un mode de réalisation, la composition selon l'invention est caractérisée en ce que le polymère anionique hydrophobisé est choisi parmi les polymères anioniques hydrophobisés de formules VIII à XI, dans lesquelles le groupe - [Hy] est un groupe alkyle issu d'un acide gras.

Dans un mode de réalisation, la composition selon l'invention est caractérisée en ce que le polymère anionique hydrophobisé est choisi parmi les polymères anioniques hydrophobisés de formules VIII à XI, dans lesquelles le groupe - [Hy] est un groupe alkyle issu d'un acide gras choisi dans le groupe constitué par les acides constitués d'une chaîne alkyle insaturée ou saturée, ramifiée ou non ramifiée, comprenant de 8 à 30 carbones.

Dans un mode de réalisation, la composition selon l'invention est caractérisée en ce que le polymère anionique hydrophobisé est choisi parmi les polymères anioniques hydrophobisés de formules VIII à XI, dans lesquelles le groupe - [Hy] est un groupe alkyle issu d'un acide gras choisi dans le groupe constitué par les acides gras linéaires.

Dans un mode de réalisation, la composition selon l'invention est caractérisée en ce que le polymère anionique hydrophobisé est choisi parmi les polymères anioniques hydrophobisés de formules VIII à XI, dans lesquelles le groupe - [Hy] est un groupe alkyle issu d'un acide gras linéaire saturé choisi dans le groupe constitué par l'acide caprylique (acide octanoïque), l'acide nonanoïque, l'acide caprique (acide décanoïque), l'acide undécanoïque, l'acide laurique (acide dodécanoïque), l'acide myristique (tétradécanoïque), l'acide palmitique (hexadécanoïque), l'acide stéarique (octadécanoïque), l'acide arachidique (eicosanoïque), l'acide béhénique (docosanoïque), l'acide tricosanoïque, l'acide lignocérique (tétracosanoïque), l'acide heptacosanoïque, l'acide octacosanoïque et l'acide mélissique (tricontanoïque).

Dans un mode de réalisation, la composition selon l'invention est caractérisée en ce que le polymère anionique hydrophobisé est choisi parmi les polymères anioniques hydrophobisés de formules VIII à XI, dans lesquelles le groupe - [Hy] est un groupe alkyle issu d'un acide gras insaturé.

Dans un mode de réalisation, la composition selon l'invention est caractérisée en ce que le polymère anionique hydrophobisé est choisi parmi les polymères anioniques hydrophobisés de formules VIII à XI, dans lesquelles le groupe - [Hy] est un groupe alkyle issu d'un acide gras insaturé choisi dans le groupe constitué par l'acide myristoléique ((Z)-tétradéc-9-énoïque), l'acide palmitoléique ((Z)-hexadéc-9-énoïque), l'acide oléique ((Z)-octadéc-9-énoïque), l'acide

élaidique ((E)-octadéc-9-énoïque), l'acide linoléique ((9Z,12Z)-octadéca-9,12-diénoïque), l'acide alpha-linoléique ((9Z,12Z,15Z)-octadéca-9,12,15-triénoïque), l'acide arachidonique ((5Z,8Z,11Z,14Z)-octadéca-5,8,11,14-tétraénoïque), l'acide eicosapentaenoïque ((5Z,8Z,11Z,14Z,17Z)-eicosa-5,8,11,14,17-pentaénoïque), l'acide erucique (13-docoénoïque) et l'acide docosahexaenoïque ((4Z,7Z,10Z,13Z,16Z,19Z)-docosa-4,7,10, 13,16,19-hexaénoïque).

Dans un mode de réalisation, la composition selon l'invention est caractérisée en ce que le polymère anionique hydrophobisé est choisi parmi les polymères anioniques hydrophobisés de formules VIII à XI, dans lesquelles le groupe - [Hy] est un groupe alkyle issu d'un acide de la bile et ses dérivés.

Dans un mode de réalisation, la composition selon l'invention est caractérisée en ce que le polymère anionique hydrophobisé est choisi parmi les polymères anioniques hydrophobisés de formules VIII à XI, dans lesquelles le groupe - [Hy] est un groupe alkyle issu d'un acide de la bile et ses dérivés choisi dans le groupe constitué par l'acide cholique, l'acide déhydrocholique, l'acide désoxycholique et l'acide chénodésoxycholique.

Dans un mode de réalisation, la composition selon l'invention est caractérisée en ce que le polymère anionique hydrophobisé est choisi parmi les polymères anioniques hydrophobisés de formule XI, dans laquelle $-R_5$ est soit un groupe -COOH, soit un radical -k-[D], dans lequel -[D] est un radical -[E]-o-[Hy] et k étant une fonction amide, le radical -[E]- étant issu de la leucine, o étant une fonction ester et -[Hy] étant issu du cholestérol.

Dans un mode de réalisation, la composition selon l'invention est caractérisée en ce que le polymère anionique hydrophobisé est choisi parmi les polymères anioniques hydrophobisés de formule X, dans laquelle -R' est soit un groupement -OH, soit un radical -f-[A]-COOH, soit un radical -g-[B]-k-[D], f étant une fonction éther, le radical -[A]- étant un radical $-CH_2-$, g étant une fonction éther, le radical -[B]- étant un radical $-CH_2-$, k étant une fonction amide, -[D] étant un radical - [E]-o-[Hy], le radical -[E]- étant issu de l'éthylènediamine, o étant une fonction amide, p et t = 1 et -[Hy] étant issu de l'acide laurique.

On entend par insuline basale dont le point isoélectrique est compris entre 5,8 et 8,5 une insuline insoluble à pH 7 et dont la durée d'action est comprise entre 8 et 24 heures ou supérieure dans les modèles standards de diabète.

Ces insulines basales dont le point isoélectrique est compris entre 5,8 et 8,5 sont des insulines recombinantes dont la structure primaire a été modifiée principalement par introduction d'aminoacides basiques comme l'Arginine ou la Lysine. Elles sont décrites par exemple dans les brevets, demandes de brevets ou publications suivants WO 2003/053339, WO 2004/096854, US 5,656,722 et US 6,100,376, dont le contenu est incorporé par référence.

Dans un mode de réalisation, l'insuline basale dont le point isoélectrique est compris entre 5,8 et 8,5 est l'insuline glargine.

Dans un mode de réalisation, les compositions selon l'invention comprennent entre 40 et 500 UI/mL d'insuline basale dont le point isoélectrique est compris entre 5,8 et 8,5.

Dans un mode de réalisation, les compositions selon l'invention comprennent entre 100 et 350 UI/mL d'insuline basale dont le point isoélectrique est compris entre 5,8 et 8,5.

Dans un mode de réalisation, les compositions selon l'invention comprennent 40 UI/mL d'insuline basale dont le point isoélectrique est compris entre 5,8 et 8,5.

Dans un mode de réalisation, les compositions selon l'invention comprennent 100 UI/mL (soit environ 3,6 mg/mL) d'insuline basale dont le point isoélectrique est compris entre 5,8 et 8,5.

Dans un mode de réalisation, les compositions selon l'invention comprennent 200 UI/mL d'insuline basale dont le point isoélectrique est compris entre 5,8 et 8,5.

Dans un mode de réalisation, les compositions selon l'invention comprennent 300 UI/mL d'insuline basale dont le point isoélectrique est compris entre 5,8 et 8,5.

Dans un mode de réalisation, les compositions selon l'invention comprennent 400 UI/mL d'insuline basale dont le point isoélectrique est compris entre 5,8 et 8,5.

Dans un mode de réalisation, les compositions selon l'invention comprennent 500 UI/mL d'insuline basale dont le point isoélectrique est compris entre 5,8 et 8,5.

Dans un mode de réalisation, le ratio massique entre l'insuline basale, dont le point isoélectrique est compris entre 5,8 et 8,5, et le polymère anionique hydrophobisé, soit polymère anionique hydrophobisé /insuline basale, est compris entre 0,2 et 30.

Dans un mode de réalisation, le ratio massique est compris entre 0,2 et 15.

Dans un mode de réalisation, le ratio massique est compris entre 0,2 et 10.

Dans un mode de réalisation, le ratio massique est compris entre 0,2 et 4.

Dans un mode de réalisation, le ratio massique est compris entre 0,2 et 3.

Dans un mode de réalisation, le ratio massique est compris entre 0,2 et 2.

Dans un mode de réalisation, le ratio massique est compris entre 0,2 et 1.

Dans un mode de réalisation, le ratio massique est égal à 1.

Dans un mode de réalisation, le ratio massique est compris entre 0,5 et 3.

Dans un mode de réalisation, le ratio massique est compris entre 1 et 3.

Dans un mode de réalisation, la concentration en polymère anionique hydrophobisé est au plus de 100 mg/mL.

Dans un mode de réalisation, la concentration en polymère anionique hydrophobisé est au plus de 80 mg/mL.

Dans un mode de réalisation, la concentration en polymère anionique hydrophobisé est au plus de 60 mg/mL.

Dans un mode de réalisation, la concentration en polymère anionique hydrophobisé est au plus de 40 mg/mL.

Dans un mode de réalisation, la concentration en polymère anionique hydrophobisé est au plus de 20 mg/mL.

Dans un mode de réalisation, la concentration en polymère anionique hydrophobisé est au plus de 10 mg/mL.

Dans un mode de réalisation, la concentration en polymère anionique hydrophobisé est au plus de 5 mg/mL.

Dans un mode de réalisation, les compositions selon l'invention comprennent en outre une insuline prandiale. Les insulines prandiales sont solubles à pH 7.

On entend par insuline prandiale une insuline dite rapide ou « regular ».

Les insulines prandiales dites rapides sont des insulines qui doivent répondre aux besoins provoqués par l'ingestion des protéines et des glucides durant un repas, elles doivent agir en moins de 30 minutes.

Dans un mode de réalisation, l'insuline prandiale dite « regular » est de l'insuline humaine.

Dans un mode de réalisation, l'insuline est une insuline humaine recombinante telle que décrite dans la Pharmacopée Européenne et la Pharmacopée américaine.

L'insuline humaine est par exemple commercialisée sous les marques Humulin® (Eli Lilly) et Novolin® (Novo Nordisk).

Les insulines prandiales dites très rapides (fast acting) sont des insulines qui sont obtenues par recombinaison et dont la structure primaire a été modifiée pour diminuer leur temps d'action.

Dans un mode de réalisation, les insulines prandiales dites rapides (fast acting) sont choisies dans le groupe comprenant l'insuline lispro (Humalog®), l'insuline glulisine (Apidra®) et l'insuline aspart (Novolog®).

Dans un mode de réalisation, l'insuline prandiale est l'insuline lispro.

Dans un mode de réalisation, l'insuline prandiale est l'insuline glulisine.

Dans un mode de réalisation, l'insuline prandiale est l'insuline aspart.

Dans un mode de réalisation, les compositions selon l'invention comprennent au total entre 40 et 800 UI/mL d'insuline avec une combinaison d'insuline prandiale et d'insuline basale dont le point isoélectrique est compris entre 5,8 et 8,5.

Dans un mode de réalisation, les compositions selon l'invention comprennent au total entre 40 et 500 UI/mL d'insuline avec une combinaison d'insuline prandiale et d'insuline basale dont le point isoélectrique est compris entre 5,8 et 8,5.

Dans un mode de réalisation, les compositions selon l'invention comprennent au total 800 UI/mL d'insuline avec une combinaison d'insuline prandiale et d'insuline basale dont le point isoélectrique est compris entre 5,8 et 8,5.

Dans un mode de réalisation, les compositions selon l'invention comprennent au total 700 UI/mL d'insuline avec une combinaison d'insuline prandiale et d'insuline basale dont le point isoélectrique est compris entre 5,8 et 8,5.

Dans un mode de réalisation, les compositions selon l'invention comprennent au total 600 UI/mL d'insuline avec une combinaison d'insuline prandiale et d'insuline basale dont le point isoélectrique est compris entre 5,8 et 8,5.

Dans un mode de réalisation, les compositions selon l'invention comprennent au total 500 UI/mL d'insuline avec une combinaison d'insuline prandiale et d'insuline basale dont le point isoélectrique est compris entre 5,8 et 8,5.

Dans un mode de réalisation, les compositions selon l'invention comprennent au total 400 UI/mL d'insuline avec une combinaison d'insuline prandiale et d'insuline basale dont le point isoélectrique est compris entre 5,8 et 8,5.

Dans un mode de réalisation, les compositions selon l'invention comprennent au total 300 UI/mL d'insuline avec une combinaison d'insuline prandiale et d'insuline basale dont le point isoélectrique est compris entre 5,8 et 8,5.

Dans un mode de réalisation, les compositions selon l'invention comprennent au total 200 UI/mL d'insuline avec une combinaison d'insuline prandiale et d'insuline basale dont le point isoélectrique est compris entre 5,8 et 8,5.

Dans un mode de réalisation, les compositions selon l'invention comprennent au total 100 UI/mL d'insuline avec une combinaison d'insuline prandiale et d'insuline basale dont le point isoélectrique est compris entre 5,8 et 8,5.

Dans un mode de réalisation, les compositions selon l'invention comprennent au total 40 UI/mL d'insuline avec une combinaison d'insuline prandiale et d'insuline basale dont le point isoélectrique est compris entre 5,8 et 8,5.

Les proportions entre l'insuline basale dont le point isoélectrique est compris entre 5,8 et 8,5 et l'insuline prandiale sont par exemple en pourcentage de 25/75, 30/70, 40/60, 50/50, 60/40, 70/30, 75/25, 80/20, 90/10 pour des formulations telles que décrites ci-dessus comprenant de 40 à 800 UI/mL. Cependant, toute autre proportion peut être réalisée.

Dans un mode de réalisation, les compositions selon l'invention comprennent en outre une hormone gastrointestinale.

On entend par « hormones gastrointestinales », les hormones choisies dans le groupe constitué par le GLP-1 (Glucagon like peptide-1) et le GIP (Glucose-dépendent insulinotropic peptide), l'oxyntomoduline (un dérivé du proglucagon), le peptide YY, l'amyline, la cholecystokinine, le polypeptide pancréatique (PP), la ghreline et l'entérostatine, leurs analogues ou dérivés et/ou leurs sels pharmaceutiquement acceptables.

Dans un mode de réalisation, les hormones gastro-intestinales sont des analogues ou dérivés de GLP-1 choisis

dans le groupe constitué par l'exenatide ou Byetta®, développé par ELI LILLY & CO et AMYLIN PHARMACEUTICALS, le liraglutide ou Victoza® développé par NOVO NORDISK, ou le lixisenatide ou Lyxumia® developpé par SANOFI-AVENTIS, leurs analogues ou dérivés et leurs sels pharmaceutiquement acceptables.

Dans un mode de réalisation, l'hormone gastrointestinale est l'exenatide ou Byetta®, ses analogues ou dérivés et leurs sels pharmaceutiquement acceptables.

Dans un mode de réalisation, l'hormone gastrointestinale est le liraglutide ou Victoza®, ses analogues ou dérivés et leurs sels pharmaceutiquement acceptables.

Dans un mode de réalisation, l'hormone gastrointestinale est le lixisenatide ou Lyxumia®, ses analogues ou dérivés et leurs sels pharmaceutiquement acceptables.

On entend par « analogue », lorsqu'il est utilisé par référence à un peptide ou une protéine, un peptide ou une protéine, dans lequel un ou plusieurs résidus d'acides aminés constitutifs ont été substitués par d'autres résidus d'acides aminés et/ou dans lequel un ou plusieurs résidus d'acides aminés constitutifs ont été supprimés et/ou dans lequel un ou plusieurs résidus d'acides aminés constitutifs ont été ajoutés. Le pourcentage d'homologie admis pour la présente définition d'un analogue est de 50 %.

On entend par « dérivé », lorsqu'il est utilisé par référence à un peptide ou une protéine, un peptide ou une protéine ou un analogue chimiquement modifié par un substituant qui n'est pas présent dans le peptide ou la protéine ou l'analogue de référence, c'est-à-dire un peptide ou une protéine qui a été modifié par création de liaisons covalentes, pour introduire des substituants.

Dans un mode de réalisation, le substituant est choisi dans le groupe constitué des chaines grasses.

Dans un mode de réalisation, les compositions selon l'invention comprennent en outre des sels de zinc à une concentration comprise entre 0 et 5000 μM.

Dans un mode de réalisation, les compositions selon l'invention comprennent en outre des sels de zinc à une concentration comprise entre 0 et 4000 μM.

Dans un mode de réalisation, les compositions selon l'invention comprennent en outre des sels de zinc à une concentration comprise entre 0 et 3000 μM.

Dans un mode de réalisation, les compositions selon l'invention comprennent en outre des sels de zinc à une concentration comprise entre 0 et 2000 μM.

Dans un mode de réalisation, les compositions selon l'invention comprennent en outre des sels de zinc à une concentration comprise entre 0 et 1000 μM.

Dans un mode de réalisation, les compositions selon l'invention comprennent en outre des sels de zinc à une concentration comprise entre 50 et 600 μM.

Dans un mode de réalisation, les compositions selon l'invention comprennent en outre des sels de zinc à une concentration comprise entre 100 et 500 μM.

Dans un mode de réalisation, les compositions selon l'invention comprennent en outre des sels de zinc à une concentration comprise entre 200 et 500 μM.

Dans un mode de réalisation, les compositions selon l'invention comprennent en outre des tampons.

Dans un mode de réalisation, les compositions selon l'invention comprennent des tampons à des concentrations comprises entre 0 et 100 mM.

Dans un mode de réalisation, les compositions selon l'invention comprennent des tampons à des concentrations comprises entre 15 et 50 mM.

Dans un mode de réalisation, les compositions selon l'invention comprennent un tampon choisi dans le groupe constitué par un tampon phosphate, le Tris (trishydroxyméthylaminométhane) ou le citrate de sodium.

Dans un mode de réalisation, le tampon est le phosphate de sodium.

Dans un mode de réalisation, le tampon est le Tris (trishydroxyméthylaminométhane).

Dans un mode de réalisation, le tampon est le citrate de sodium.

Dans un mode de réalisation, les compositions selon l'invention comprennent en outre des conservateurs.

Dans un mode de réalisation, les conservateurs sont choisis dans le groupe constitué par le m-crésol et le phénol seuls ou en mélange.

Dans un mode de réalisation, la concentration des conservateurs est comprise entre 10 et 50 mM.

Dans un mode de réalisation, la concentration des conservateurs est comprise entre 10 et 40 mM.

Dans un mode de réalisation, les compositions selon l'invention comprennent en outre un tensioactif.

Dans un mode de réalisation, le tensioactif est choisi dans le groupe constitué par le propylène glycol ou le polysorbate.

Les compositions selon l'invention peuvent en outre comprendre des additifs tels que des agents de tonicité.

Dans un mode de réalisation, les agents de tonicité sont choisis dans le groupe constitué par la glycérine, le chlorure de sodium, le mannitol et la glycine.

Les compositions selon l'invention peuvent comprendre en outre tous les excipients conformes aux pharmacopées et compatibles avec les insulines utilisées aux concentrations d'usage.

L'invention concerne également une formulation pharmaceutique selon l'invention, caractérisée en ce qu'elle est obtenue par séchage et/ou lyophilisation.

Dans le cas des libérations locale et systémique, les modes d'administration envisagés sont par voie intraveineuse, sous-cutanée, intradermique ou intramusculaire.

Les voies d'administration transdermique, orale, nasale, vaginale, oculaire, buccale, pulmonaire sont également envisagées.

L'invention concerne également des formulations unidoses à pH compris entre 6,6 et 7,8 comprenant une insuline basale dont le point isoélectrique est compris entre 5,8 et 8,5.

L'invention concerne également des formulations unidoses à pH compris entre 6,6 et 7,8 comprenant une insuline basale dont le point isoélectrique est compris entre 5,8 et 8,5 et une insuline prandiale.

L'invention concerne également des formulations unidoses à pH compris entre 6,6 et 7,8 comprenant une insuline basale dont le point isoélectrique est compris entre 5,8 et 8,5 et une hormone gastrointestinale, telle que définie précédemment.

L'invention concerne également des formulations unidoses à pH compris entre 6,6 et 7,8 comprenant une insuline basale dont le point isoélectrique est compris entre 5,8 et 8,5, une insuline prandiale et une hormone gastrointestinale, telle que définie précédemment.

L'invention concerne également des formulations unidoses à pH compris entre 7 et 7,8 comprenant une insuline basale dont le point isoélectrique est compris entre 5,8 et 8,5.

L'invention concerne également des formulations unidoses à pH compris entre 7 et 7,8 comprenant une insuline basale dont le point isoélectrique est compris entre 5,8 et 8,5 et une insuline prandiale.

L'invention concerne également des formulations unidoses à pH compris entre 7 et 7,8 comprenant une insuline basale dont le point isoélectrique est compris entre 5,8 et 8,5 et une hormone gastrointestinale, telle que définie précédemment.

L'invention concerne également des formulations unidoses à pH compris entre 7 et 7,8 comprenant une insuline basale dont le point isoélectrique est compris entre 5,8 et 8,5 , une insuline prandiale et une hormone gastrointestinale, telle que définie précédemment.

Dans un mode de réalisation les formulations unidoses comprennent en outre un polymère anionique hydrophobisé anionique hydrophobisé s tel que défini précédemment.

Dans un mode de réalisation, les formulations sont sous forme d'une solution injectable.

Dans un mode de réalisation, l'insuline basale dont le point isoélectrique est compris entre 5,8 et 8,5 est l'insuline glargine.

Dans un mode de réalisation, l'insuline prandiale est l'insuline humaine.

Dans un mode de réalisation, l'insuline est une insuline humaine recombinante telle que décrite dans la Pharmacopée Européenne et la Pharmacopée américaine.

Dans un mode de réalisation, l'insuline prandiale est choisie dans le groupe comprenant l'insuline lispro (Humalog®), l'insuline glulisine (Apidra®) et l'insuline aspart (NovoLog®).

Dans un mode de réalisation, l'insuline prandiale est l'insuline lispro.

Dans un mode de réalisation, l'insuline prandiale est l'insuline glulisine.

Dans un mode de réalisation, l'insuline prandiale est l'insuline aspart.

Dans un mode de réalisation, le GLP-1, analogue ou dérivé de GLP-1 est choisi dans le groupe comprenant exenatide (Byetta®), liraglutide (Victoza®), lixisenatide (Lyxumia®) ou l'un de leurs dérivés.

Dans un mode de réalisation, l'hormone gastrointestinale est l'exenatide.

Dans un mode de réalisation, l'hormone gastrointestinale est le liraglutide.

Dans un mode de réalisation, l'hormone gastrointestinale est le lixisenatide.

La solubilisation à pH compris entre 6,6 et 7,8 des insulines basales dont le point isoélectrique est compris entre 5,8 et 8,5, par les polymères anioniques hydrophobisés de formule I et IV à XII, peut être constatée et contrôlée de manière simple, à l'oeil nu, grâce à un changement d'aspect de la solution.

La solubilisation à pH compris entre 7 et 7,8 des insulines basales dont le point isoélectrique est compris entre 5,8 et 8,5, par les polymères anioniques hydrophobisés de formule I et IV à XII, peut être constatée et contrôlée de manière simple, à l'oeil nu, grâce à un changement d'aspect de la solution.

Par ailleurs et de façon tout aussi importante, la demanderesse a pu vérifier qu'une insuline basale dont le point isoélectrique est compris entre 5,8 et 8,5, solubilisée à pH compris entre 6,6 et 7,8 en présence d'un polymère anionique hydrophobisé de formule I et IV à XII, conserve une action d'insuline lente, que ce soit seule ou en combinaison avec une insuline prandiale ou une hormone gastrointestinale.

La demanderesse a également pu vérifier qu'une insuline prandiale mélangée à pH compris entre 6,6 et 7,8 en présence d'un polymère anionique hydrophobisé de formule I et IV à XII, et d'une insuline basale dont le point isoélectrique est compris entre 5,8 et 8,5, conserve une action d'insuline rapide.

La préparation d'une composition selon l'invention présente l'avantage de pouvoir être réalisée par simple

mélange d'une solution aqueuse d'insuline basale dont le point isoélectrique est compris entre 5,8 et 8,5, et d'un polymère anionique hydrophobisé de formule I et IV à XII, en solution aqueuse ou sous forme lyophilisée. Si nécessaire, le pH de la préparation est ajusté à pH 7.

La préparation d'une composition selon l'invention présente l'avantage de pouvoir être réalisée par simple mélange d'une solution aqueuse d'insuline basale dont le point isoélectrique est compris entre 5,8 et 8,5, d'une solution d'insuline prandiale, et d'un polymère anionique hydrophobisé de formule I et IV à XII, en solution aqueuse ou sous forme lyophilisée. Si nécessaire, le pH de la préparation est ajusté à pH 7.

La préparation d'une composition selon l'invention présente l'avantage de pouvoir être réalisée par simple mélange d'une solution aqueuse d'insuline basale dont le point isoélectrique est compris entre 5,8 et 8,5, d'une solution de GLP-1, un analogue ou un dérivé de GLP-1, et d'un polymère anionique hydrophobisé de formule I et IV à XII, en solution aqueuse ou sous forme lyophilisée. Si nécessaire, le pH de la préparation est ajusté à pH 7.

La préparation d'une composition selon l'invention présente l'avantage de pouvoir être réalisée par simple mélange d'une solution aqueuse d'insuline basale dont le point isoélectrique est compris entre 5,8 et 8,5, d'une solution d'insuline prandiale, d'une solution de GLP-1 ou d'un analogue ou dérivé de GLP-1 et d'un polymère anionique hydrophobisé de formule I et IV à XII, en solution aqueuse ou sous forme lyophilisée. Si nécessaire, le pH de la préparation est ajusté à pH 7.

Dans un mode de réalisation le mélange d'insuline basale et de polymère anionique hydrophobisé aqueuse ou sous forme lyophilisée.

Description des figures :

Figure 1 : Courbes moyennes de glycémie ± erreur standard de la moyenne pour les administrations simultanées de Humalog® (100 UI/mL, 0,075 UI/kg) et Lantus® (100 UI/mL, 0,225 UI/kg) en comparaison avec l'administration d'une formulation selon l'invention décrite dans l'exemple B57 (400 UI/mL, 0,3 UI/kg).

Exemples

Partie A : Synthèse des polymères anioniques hydrophobisés

Tableau 1a : liste des polymères anioniques hydrophobisés exemplifiés (AA1 et AB1-AB3)

| N°polymère anionique hydrophobisé | AA1 | AB1 | AB2 | AB3 |
|---|---|---|---|---|
| Formule | X | V | V | V |
| Squelette anionique | 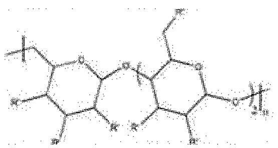 | 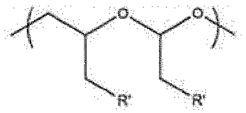 | 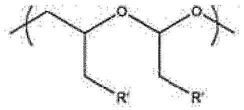 | 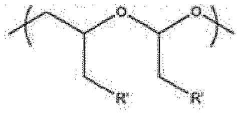 |
| -$R_5$ (Degré de substitution) | n/a | n/a | n/a | |
| -R' (Degré de substitution) | -(*f*-[A]-COOH) 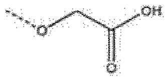 1,06 | -(*f*-[A]-COOH) 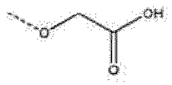 1,26 | -(*f*-[A]-COOH) 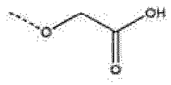 1,25 | -(*f*-[A]-COOH) 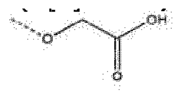 1,00 |
| | ou -*g*-[B] -*k*-[E]-*o*-[Hy] 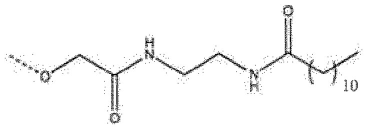 0,11 | ou -*g*-[B] -*k*-[E]-*o*-[Hy] 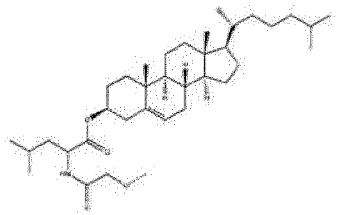 0,04 | ou -*g*-[B]-*k*-[E]-*o*-[Hy] 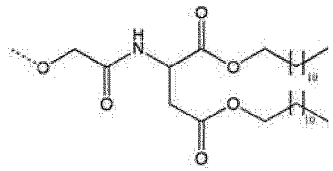 0,05 | ou -*g*-[B]-*k*-[E]-*o*-[Hy] 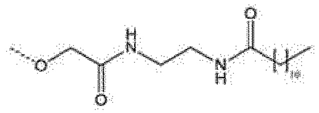 0,1 |

Tableau 1b : liste des polymères anioniques hydrophobisés exemplifiés (AB4-AB7)

| N° polymère anionique hydrophobisé | AB4 | AB5 | AB6 | AB7 |
|---|---|---|---|---|
| Formule | V | V | V | V |
| Squelette anionique | 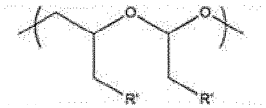 | 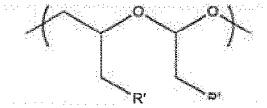 | 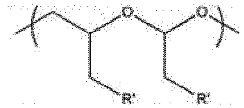 | 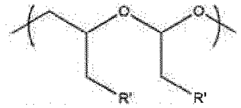 |
| $-R_5$ (Degré de substitution) | n/a | n/a | n/a | n/a |
| | | | | |
| -R' (Degré de substitution) | -(*f*-[A]-COOH) 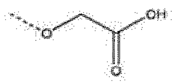 1,20 | -(*f*-[A]-COOH) 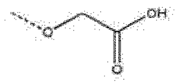 1,26 | -(*f*-[A]-COOH) 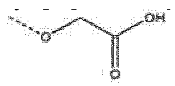 1,20 | -(*f*-[A]-COOH) 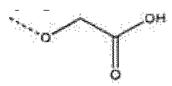 1,26 |
| | ou -*g*-[B]-*k*-[E]-*o*-[Hy] 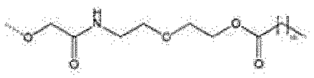 0,1 | ou -*g*-[B]-*k*-[E]-*o*-[Hy] 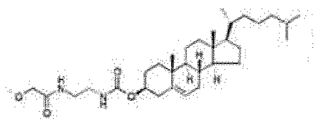 0,04 | ou -*g*-[B]-*k*-[E]-*o*-[Hy] 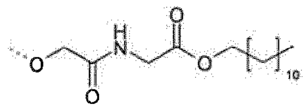 0,1 | ou -*g*-[B]-*k*-[E]-*o*-[Hy] 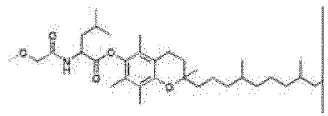 0,04 |

Tableau 1c : liste des polymères anioniques hydrophobisés exemplifiés (AB8-AB10)

| N° polymère anionique hydrophobisé | AB8 | AB9 | AB10 |
|---|---|---|---|
| Formule | V | V | V |
| Squelette anionique | 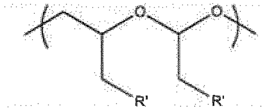 | 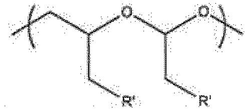 | 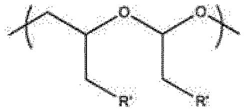 |
| -$R_5$ (Degré de substitution) | n/a | n/a | n/a |
| | | | |
| -R' (Degré de substitution) | -(*f*-[A]-COOH)<br>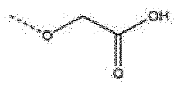<br>1,20 | -(*f*-[A]-COOH)<br>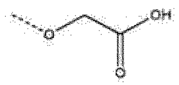<br>1,25 | -(*f*-[A]-COOH)<br>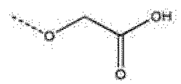<br>1,10 |
| | ou -*g*-[B]-*k*-[E]-*o*-[Hy]<br>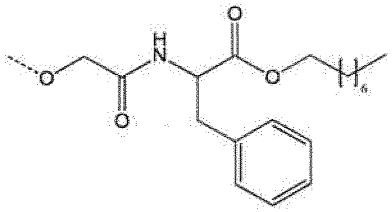<br>0,1 | ou -*g*-[B]-*k*-[E]-*o*-[Hy]<br>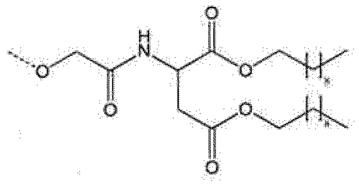<br>0,05 | ou -*g*-[B]-*k*-[E]-*o*-[Hy]<br>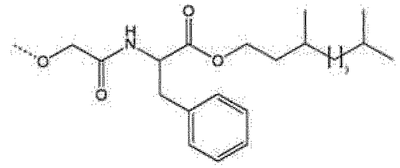<br>0,20 |

**Exemple AA1** : **Pullulaneméthylcarboxylate de sodium modifié par le N-(2-aminoéthyl)dodécanamide**

**Polymère anionique hydrophobisé AA1**

Le N-(2-aminoethyl)dodécanamide est obtenu selon le procédé décrit dans le brevet US 2,387,201 (Weiner, N ; et al.) à partir du méthyle ester de l'acide dodécanoïque (Sigma) et de l'éthylènediamine (Roth).
8 g (soit 148 mmol de fonctions hydroxyles) de pullulane de masse molaire moyenne en poids d'environ 100 kg/mol (Fluka) sont solubilisés dans de l'eau. A cette solution sont ajoutés 15 mL de NaOH 10 N (148 mmol NaOH). Le mélange est porté à 35°C puis 23 g (198 mmol) de chloroacétate de sodium sont ajoutés. La température du milieu réactionnel est progressivement portée à 60°C puis maintenue à 60°C pendant 100 minutes. Le milieu réactionnel est dilué avec de l'eau, neutralisé à l'acide acétique et purifié par ultrafiltration sur membrane PES de 5 kD contre 6 volumes d'eau. La solution finale est dosée par extrait sec pour déterminer la concentration en polymère; puis dosée par dosage acide/base dans de l'eau/acétone 50 / 50 (V/V) pour déterminer le degré de substitution en méthylcarboxylate de sodium.
D'après l'extrait sec : [polymère] = 31,5 mg/g
D'après le dosage acide/base : le degré de substitution en méthylcarboxylate de sodium est de 1,17 par unité monomérique.
La solution de pullulane méthylcarboxylate de sodium est passée sur une résine Purolite (anionique) pour obtenir l'acide pullulane méthylcarboxylique qui est ensuite lyophilisé pendant 18 heures.
2 g de pullulane méthylcarboxylique acide (10 mmol fonctions méthylcarboxylique acide) sont solubilisés dans le DMF à 30 g/L puis refroidis à 0°C. Une fois la solution de polymère à 0°C, 1,1 g (11 mmol) de NMM et 1,2 g (11 mmol) de EtOCOCl sont ensuite ajoutés. Après 10 min de réaction, 0,2 g de N-(2-aminoéthyl)dodécanamide (0,9 mmol) est introduit et le milieu est porté à 30°C durant 90 minutes. Une solution aqueuse d'imidazole à 600 g/L et 40 mL d'eau sont ajoutés et le milieu est ensuite chauffé à 50°C. Après 1h30 d'agitation à 50°C, la solution obtenue est ultrafiltrée sur membrane PES 10 kD contre NaCl 0,9%, de la soude 0,01N et de l'eau. La solution est lyophilisée et analysée par RMN $^1H$ dans $D_2O$ pour déterminer le degré de substitution en méthylcarboxylate modifié par le N-(2-ami noéthyl)dodécanamide.
D'après la RMN $^1H$ : le degré de substitution en méthylcarboxylate modifié par le N-(2-aminoéthyl)dodécanamide est de 0,11.

**Exemple AB1 : Polyacétal méthylcarboxylate modifié par le leucinate de chloestéryle**

**Polymère anionique hydrophobisé AB1**

Par un procédé similaire à celui décrit dans Biomacromolecules 2005, 6, 2659-2670 à partir d'un dextrane de masse molaire moyenne en poids 5 kg/mol (Pharmacosmos), est synthétisé le poly(1-hydroxyméthyléthylène hydroxyméthylformal). Par un procédé similaire à celui décrit dans l'exemple AA1, un poly(1-hydroxyméthyléthylène hydroxyméthylformal) fonctionnalisé avec un degré de substitution en méthylcarboxylate de sodium de 1,3 par unité monomérique est obtenu. Par un procédé similaire à celui décrit dans l'exemple AA1, un poly(1-hydroxyméthyléthylène hydroxyméthylformal) méthylcarboxylate de sodium modifié par le leucinate de cholestéryle avec un degré de substitution en méthylcarboxylate modifié par le leucinate de cholestéryle de 0,04 est obtenu.

**Exemple AB2 : Polyacétal méthylcarboxylate modifié par l'aspartate de dilauryle**

**Polymère anionique hydrophobisé AB2**

L'aspartate de dilauryle, sel d'acide paratoluènesulfonique est préparé à partir de dodécanol et d'acide aspartique selon le procédé décrit dans le brevet US 4,826,818 (Kenji M., et al.).
Par un procédé similaire à celui décrit dans l'exemple AB1, un poly(1-hydroxyméthyléthylène hydroxyméthylformal) méthylcarboxylate de sodium modifié par l'aspartate de dilauryle avec un degré de substitution en méthylcarboxylate de sodium de 1,25 et un degré de substitution en méthylcarboxylates modifiés par l'aspartate de dilauryle de 0,05 est obtenu.

**Exemple AB3 : Polyacétal méthylcarboxylate modifié par le N-(2-aminoéthyl)dodécanamide**

**Polymère anionique hydrophobisé AB3**

Le N-(2-aminoéthyl)dodécanamide est obtenu selon le procédé décrit dans le brevet US 2,387,201 (Weiner et al.) à partir de l'ester méthylique de l'acide dodécanoïque (Sigma) et de l'éthylènediamine (Roth).

Par un procédé similaire à celui décrit dans Biomacromolecules 2005, 6, 2659-2670 à partir d'un dextrane de masse molaire moyenne en poids 10 kg/mol (Pharmacosmos), est synthétisé le poly(1-hydroxyméthyléthylène hydroxyméthylformal).

Par un procédé similaire à celui décrit dans l'exemple AB1, un poly(1-hydroxyméthyléthylène hydroxyméthylformal) méthylcarboxylate de sodium modifié par le N-(2-aminoéthyl)dodécanamide avec un degré de substitution en méthylcarboxylate de sodium de 1,0 et un degré de substitution en méthylcarboxylates modifiés par le N-(2-aminoéthyl)dodécanamide de 0,1 est obtenu.

**Exemple AB4 : Polyacétal méthylcarboxylate modifié par le 2-(2-amino-éthoxy)éthyle dodécanoate**

**Polymère anionique hydrophobisé AB4**

Le 2-(2-amino-éthoxy)éthyle dodécanoate, sel d'acide paratoluènesulfonique est obtenu selon le procédé décrit dans le brevet US 4,826,818 (Kenji M., et al.).

Par un procédé similaire à celui décrit dans l'exemple AB1, un poly(1-hydroxyméthyléthylène hydroxyméthylformal) méthylcarboxylate de sodium modifié par le 2-(2-amino-éthoxy)éthyle dodécanoate avec un degré de substitution en méthylcarboxylate de sodium de 1,2 et un degré de substitution en méthylcarboxylates modifiés par le 2-(2-aminoéthoxy)éthyle dodécanoate de 0,1 est obtenu.

**Exemple AB5: Polyacétal méthylcarboxylate modifié par le 2-aminoéthylcarbamate de cholestéryle**

**Polymère anionique hydrophobisé AB5**

Le 2-aminoéthylcarbamate de cholestéryle, sel d'acide chlorhydrique, est préparé selon le procédé décrit dans la demande WO2010053140 (Akiyoshi K., et al.*).*

Par un procédé similaire à celui décrit dans l'exemple AB1, un poly(1-hydroxyméthyléthylène hydroxyméthylformal) méthylcarboxylate de sodium modifié par le 2-aminoéthylcarbamate de cholestéryle avec un degré de substitution en méthylcarboxylate de sodium de 1,26 et un degré de substitution en méthylcarboxylates modifiés par le 2-aminoéthylcarbamate de cholestéryle de 0,04 est obtenu.

**Exemple AB6 : Polyacétal méthylcarboxylate modifié par le glycinate de lauryle**

**Polymère anionique hydrophobisé AB6**

Le glycinate de lauryle, sel d'acide paratoluènesulfonique est préparé à partir du dodécanol et de la glycine selon le procédé décrit dans le brevet US 4,826,818 (Kenji M., et al.).

Par un procédé similaire à celui décrit dans l'exemple AB3, un poly(1-hydroxyméthyléthylène hydroxyméthylformal) méthylcarboxylate de sodium modifié par le glycinate de lauryle avec un degré de substitution en méthylcarboxylate de sodium de 1,2 et un degré de substitution en méthylcarboxylates modifiés par le glycinate de lauryle de 0,1 est obtenu.

**Exemple AB7** : **Polyacétal méthylcarboxylate modifié par le leucinate de (±)a-tocophérol**

**Polymère anionique hydrophobisé AB7**

Le leucinate de (±)a-tocophérol, sel d'acide chlorhydrique est obtenu selon le procédé décrit dans la publication de Takata, J et al., Journal of Pharmaceutical Sciences 1995, 84(1), 96-100.

Par un procédé similaire à celui décrit dans l'exemple AB1, un poly(1-hydroxyméthyléthylène hydroxyméthylformal) méthylcarboxylate de sodium modifié par le leucinate de (±)a-tocophérol avec un degré de substitution en méthylcarboxylate de sodium de 1,26 et un degré de substitution en méthylcarboxylates modifiés par le leucinate de (±)a-tocophérol de 0,04 est obtenu.

**Exemple AB8** : **Polyacétal méthylcarboxylate modifié par le phénylalaninate d'octanoyle**

**Polymère anionique hydrophobisé AB8**

Le phénylalaninate d'octanoyle, sel d'acide paratoluènesulfonique est préparé à partir du 1-octanol et de la L-phénylalanine selon le procédé décrit dans le brevet US 4,826,818 (Kenji M., et al.).

Par un procédé similaire à celui décrit dans l'exemple AB3, un poly(1-hydroxyméthyléthylène hydroxyméthylformal) méthylcarboxylate de sodium modifié par le phénylalaninate d'octanoyle avec un degré de substitution en méthylcarboxylate de sodium de 1,2 et un degré de substitution en méthylcarboxylates modifiés par le phénylalaninate d'octanoyle de 0,1 est obtenu.

**Exemple AB9 : Polyacétal méthylcarboxylate modifié par l'aspartate de didécyle**

**Polymère anionique hydrophobisé AB9**

L'aspartate de didécyle, sel d'acide paratoluènesulfonique est préparé à partir de dodécanol et d'acide aspartique selon le procédé décrit dans le brevet US 4,826,818 (Kenji M., et al.).
Par un procédé similaire à celui décrit dans l'exemple AB3, un poly(1-hydroxyméthyléthylène hydroxyméthylformal) méthylcarboxylate de sodium modifié par l'aspartate de didécyle avec un degré de substitution en méthylcarboxylate de sodium de 1,25 et un degré de substitution en méthylcarboxylates modifiés par l'aspartate de didécyle de 0,05 est obtenu.

**Exemple AB10** : **Polyacétal méthylcarboxylate modifié par le phénylalaninate de 3,7-diméthyloctanoyle**

**Polymère anionique hydrophobisé AB10**

Le phénylalaninate de 3,7-diméthyloctanoyle, sel d'acide paratoluènesulfonique est préparé à partir du 3,7-diméthyloctan-1-ol et de la L-phénylalanine selon le procédé décrit dans le brevet US 4,826,818 (Kenji et al.).
Par un procédé similaire à celui décrit dans l'exemple AB1, un poly(1-hydroxyméthyléthylène hydroxyméthylformal) méthylcarboxylate de sodium modifié par le phénylalaninate de 3,7-diméthyloctanoyle avec un degré de substitution en méthylcarboxylate de sodium de 1,1 et un degré de substitution en méthylcarboxylates modifiés par la phénylalaninate de 3,7-diméthyloctanoyle de 0,2 est obtenu.

**Exemple comparatif AC1: Dextraneméthylcarboxylate de sodium fonctionnalisé par le leucinate de cholestéryle**

**Polymère AC1**

Selon le procédé décrit dans la demande de brevet WO2012/153070, un dextraneméthylcarboxylate de sodium synthétisé à partir d'un dextrane de masse molaire moyenne en poids 1 kg/mol (Pharmacosmos) est fonctionnalisé par le leucinate de cholestéryle.
Le degré de substitution en méthylcarboxylate de sodium est de 1,60.
Le degré de substitution en méthylcarboxylates fonctionnalisés par le leucinate de cholestéryle est de 0,05.

Partie B : Mise en évidence des propriétés des compositions selon l'invention

Exemple B1 : Solution d'insuline analogue rapide (NovoLog®) à 100 UI/mL

Cette solution est une solution commerciale d'insuline aspart commercialisée par la société NOVO NORDISK sous le nom de NovoLog® aux Etats-Unis d'Amérique et Novorapid® en Europe. Ce produit est une insuline analogue rapide.

**Exemple B2** : **Solution d'insuline analogue rapide (Humalog®) à 100 UI/mL**

Cette solution est une solution commerciale d'insuline lispro commercialisée par la société ELI LILLY sous le nom de Humalog®. Ce produit est une insuline analogue rapide.

**Exemple B3** : **Solution d'insuline analogue rapide (Apidra®) à 100 UI/mL**

Cette solution est une solution commerciale d'insuline glulisine commercialisée par la société SANOFI-AVENTIS sous le nom d'Apidra®. Ce produit est une insuline analogue rapide.

**Exemple B4 : Solution d'insuline analogue lente (Lantus®) à 100 UI/mL**

Cette solution est une solution commerciale d'insuline glargine commercialisée par la société SANOFI-AVENTIS

sous le nom de Lantus®. Ce produit est une insuline analogue lente.

**Exemple B5 : Solution d'insuline humaine (ActRapid®) à 100 UI/mL**

Cette solution est une solution commerciale d'insuline humaine de NOVO NORDISK vendue sous le nom d'ActRapid®. Ce produit est une insuline humaine.

**Exemple B6 : Solubilisation de l'insuline glargine à 100 UI/mL et à pH 7 à l'aide d'un polymère anionique hydrophobisé.**

Une masse d'au plus 120 mg d'un lyophilisat de polymère anionique hydrophobisé choisi parmi ceux décrits dans le tableau 1 est pesée précisément. Le lyophilisat est repris par 2 mL de la solution d'insuline glargine de l'exemple B4 afin d'obtenir une solution dont la concentration en polymère anionique hydrophobisé est d'au plus 60 mg/mL. Après agitation mécanique sur rouleaux à température ambiante, la solution devient limpide. Le pH de cette solution est d'environ 6,3. Le pH est ajusté à 7 avec une solution de soude 0,1 N. Cette solution limpide est filtrée sur membrane (0,22 μm) puis est placée à +4°C.

L'essai de solubilisation selon le protocole ci-dessus a été réalisé avec différents polymères anioniques hydrophobisés. Ces solutions sont référencées dans le Tableau 2.

Tableau 2 : Solutions selon l'exemple B6 avec les polymères anioniques hydrophobisés

| Solution exemple B6 | Polymère anionique hydrophobisé | $C_{\text{polymère anionique hydrophobisé}}$ (mg/mL) | $C_{\text{insuline glargine}}$ (UI/mL) |
|---|---|---|---|
| B6(a) | AA1 | 60 | 100 |
| B6(b) | AB1 | 10 | 100 |
| B6(c) | AB2 | 10 | 100 |
| B6(d) | AB3 | 10 | 100 |
| B6(e) | AB4 | 10 | 100 |
| B6(f) | AB5 | 10 | 100 |
| B6(g) | AB6 | 10 | 100 |
| B6(h) | AB7 | 10 | 100 |
| B6(i) | AB8 | 10 | 100 |
| B6(j) | AB9 | 10 | 100 |
| B6(k) | AB10 | 10 | 100 |

**Exemple B7 : Préparation d'une composition polymère anionique hydrophobisé AB1/insuline glargine/insuline glulisine avec un ratio insuline glargine/insuline glulisine 75/25 à pH 7.**

A 0,75 mL de la solution de polymère anionique hydrophobisé AB1/insuline glargine préparée selon le protocole décrit à l'exemple B6(b) est ajouté 0,25 mL de la solution d'insuline glulisine de l'exemple B3 pour former 1 mL d'une composition à pH 7. La composition est limpide, attestant de la bonne solubilité de l'insuline glargine et l'insuline glulisine dans ces conditions de formulation. Cette solution limpide est filtrée sur 0,22 μm puis est placée à +4°C.

**Exemple B8 : Préparation d'une composition polymère anionique hydrophobisé AB1/insuline glargine/insuline lispro avec un ratio insuline glargine/insuline lispro 75/25 à pH 7**

A 0,75 mL de la solution de polymère anionique AB1/insuline glargine préparée selon le protocole décrit à l'exemple B6(b) est ajouté 0,25 mL de la solution d'insuline lispro de l'exemple B2 pour former 1 mL d'une composition à pH 7. La composition est limpide, attestant de la bonne solubilité de l'insuline glargine et de l'insuline lispro dans ces conditions de formulation. Cette solution limpide est filtrée sur 0,22 μm puis est placée à +4°C.

**Exemple B9 : Préparation d'une composition polymère anionique hydrophobisé AB1/insuline glargine/insuline aspart avec un ratio insuline glargine/insuline aspart 75/25 à pH 7**

A 0,75 mL de la solution de polymère anionique AB1/ insuline glargine préparée à l'exemple B6(b) est ajouté 0,25 mL de la solution d'insuline aspart de l'exemple B1 pour former 1 mL d'une composition à pH 7. La composition est limpide, attestant de la bonne solubilité de l'insuline glargine et de l'insuline aspart dans ces conditions de formulation. Cette solution limpide est filtrée sur 0,22 μm puis est placée à +4°C.

**Exemple B10 : Préparation d'une composition polymère anionique hydrophobisé AB1/ insuline glargine/insuline humaine avec un ratio insuline glargine/insuline humaine 75/25 à pH 7**

A 0,75 mL de la solution de polymère anionique hydrophobisé AB1/ insuline glargine préparée à l'exemple B6(b) est ajouté 0,25 mL de la solution d'insuline humaine de l'exemple B5 pour former 1 mL d'une composition à pH 7. La composition est limpide, attestant de la bonne solubilité de l'insuline glargine et de l'insuline humaine dans ces conditions de formulation. Cette solution limpide est filtrée sur 0,22 μm puis est placée à +4°C.

**Exemple B11 : Préparation d'une composition polymère anionique hydrophobisé AB1/ insuline glargine/insuline lispro avec un ratio insuline glargine/insuline lispro 60/40 à pH 7**

A 0,6 mL de la solution de polymère anionique hydrophobisé AB1/insuline glargine préparée à l'exemple B6(b) est ajouté 0,4 mL de la solution d'insuline lispro de l'exemple B2 pour former 1 mL d'une composition à pH 7. La composition est limpide, attestant de la bonne solubilité de l'insuline glargine et de l'insuline lispro dans ces conditions de formulation. Cette solution limpide est filtrée sur 0,22 μm puis est placée à +4°C.

**Exemple B12 : Préparation d'une composition polymère anionique hydrophobisé AB1/ insuline glargine/insuline lispro avec un ratio insuline glargine/insuline lispro 40/60 à pH 7**

A 0,4 mL de la solution de polymère anionique AB1/insuline glargine préparée à l'exemple B6(b) est ajouté 0,6 mL de la solution d'insuline lispro de l'exemple B2 pour former 1 mL d'une composition à pH 7. La composition est limpide, attestant de la bonne solubilité de l'insuline glargine et de l'insuline lispro dans ces conditions de formulation. Cette solution limpide est filtrée sur 0,22 μm puis est placée à +4°C.

**Exemple B13 : Précipitation de l'insuline glargine**

1 mL de la solution d'insuline glargine de l'exemple B4 est ajouté dans 2 mL d'une solution de PBS (tampon phosphate, phosphate buffered saline) contenant 20 mg/mL de BSA (albumine du sérum bovin, bovine serum albumin). Le mélange PBS/BSA simule la composition du milieu sous-cutané. Un précipité apparaît, ce qui est en bon accord avec le mécanisme de fonctionnement de l'insuline glargine (précipitation à l'injection due à l'augmentation du pH).

Une centrifugation à 4000 trs/min est effectuée pour séparer le précipité du surnageant. Ensuite, l'insuline glargine est dosée dans le surnageant par chromatographie liquide en phase inverse (RP-HPLC). Il en résulte que l'insuline glargine se retrouve majoritairement sous une forme précipitée.

**Exemple B14 : Précipitation d'une composition polymère anionique hydrophobisé AA1/insuline glargine.**

1 mL de solution polymère anionique hydrophobisé AA1/insuline glargine préparée à l'exemple B6(a) est ajouté dans 2 mL d'une solution de PBS contenant 20 mg/mL de BSA. Le mélange PBS/BSA simule la composition du milieu sous-cutané. Un précipité apparaît.

Une centrifugation à 4000 trs/min est effectuée pour séparer le précipité du surnageant. Ensuite l'insuline glargine est dosée dans le surnageant par RP-HPLC. Il en résulte que l'insuline glargine se retrouve majoritairement sous une forme précipitée.

Des essais de solubilisation et précipitation identiques à ceux décrits à l'exemple B6 et B14 ont été effectués avec d'autres polymères anioniques hydrophobisés avec une concentration d'au plus 60 mg/mL pour une concentration en insuline glargine de 100 UI/mL. Il en résulte que pour l'ensemble des compositions B6(b) à B6(k), l'insuline glargine se retrouve majoritairement sous une forme précipitée à l'issue de l'ajout d'1 mL de la composition à 2 mL d'une solution de PBS contenant 20 mg/mL de BSA. Les résultats sont résumés dans le tableau 4.

Tableau 4 : Essais de solubilisation et de précipitation d'une composition de polymère anionique hydrophobisé /insuline glargine

| Polymère anionique hydrophobisé | Solubilisation de l'insuline glargine | Précipitation de l'insuline glargine |
|---|---|---|
| AA1 | Oui | Oui |
| AB1 | Oui | Oui |
| AB2 | Oui | Oui |
| AB3 | Oui | Oui |
| AB4 | Oui | Oui |
| AB5 | Oui | Oui |
| AB6 | Oui | Oui |
| AB7 | Oui | Oui |
| AB8 | Oui | Oui |
| AB9 | Oui | Oui |
| AB10 | Oui | Oui |

**Exemple B15 : Précipitation d'une composition polymère anionique hydrophobisé AB1/insuline glargine/insuline lispro avec un ratio insuline glargine/insuline lispro 75/25 à pH 7**

1 mL de la composition polymère anionique hydrophobisé AB1/insuline glargine/insuline lispro 75/25 préparée selon le protocole de l'exemple B8 est ajouté dans 2 mL d'une solution de PBS contenant 20 mg/mL de BSA. Le mélange PBS/BSA simule la composition du milieu sous-cutané. Un précipité apparaît.

Une centrifugation à 4000 trs/min est effectuée pour séparer le précipité du surnageant. Ensuite, l'insuline glargine est dosée dans le surnageant par RP-HPLC. Il en résulte que l'insuline glargine se retrouve majoritairement sous une forme précipitée.

**Exemple B16 : Précipitation de différentes compositions en faisant varier la nature du polymère anionique hydrophobisé.**

D'autres essais de précipitation de l'insuline glargine dans les mêmes conditions que celles de l'exemple B15 ont été effectués en présence d'autres polymères anioniques hydrophobisés.

Les résultats sont regroupés dans le tableau 5 suivant et on observe que la solubilisation ainsi que la précipitation de l'insuline glargine sont conservées.

Tableau 5 : Essais de solubilisation et de précipitation d'une composition polymère anionique hydrophobisé /insuline glargine/insuline lispro 75/25 à pH 7

| Polymère anionique hydrophobisé | Solubilisation insuline glargine/insuline lispro 75/25 | Précipitation de l'insuline glargine |
|---|---|---|
| AA1 | Oui | Oui |
| AB1 | Oui | Oui |
| AB2 | Oui | Oui |
| AB3 | Oui | Oui |
| AB4 | Oui | Oui |
| AB5 | Oui | Oui |
| AB6 | Oui | Oui |
| AB7 | Oui | Oui |
| AB8 | Oui | Oui |

(suite)

| Polymère anionique hydrophobisé | Solubilisation insuline glargine/insuline lispro 75/25 | Précipitation de l'insuline glargine |
|---|---|---|
| AB9 | Oui | Oui |
| AB10 | Oui | Oui |

**Exemple B17 : Précipitation de différentes compositions en faisant varier la nature de l'insuline prandiale**

Des compositions sont préparées en mélangeant 0,75 mL de la solution de polymère anionique hydrophobisé AB1/insuline glargine préparée selon le protocole de l'exemple B6(b) avec 0,25 mL d'une insuline prandiale pour former 1 mL de composition polymère anionique hydrophobisé AB1/insuline glargine/insuline prandiale (contenant 7,5 mg/mL de polymère anionique hydrophobisé AB1, 75 UI/mL d'insuline glargine et 25 UI/mL d'insuline prandiale).

Cette composition est ajoutée dans 2 mL de PBS contenant 20 mg/mL de BSA. Le mélange PBS/BSA simule la composition du milieu sous-cutané. Un précipité apparaît. Une centrifugation à 4000 trs/min est effectuée pour séparer le précipité du surnageant. Ensuite, l'insuline glargine est dosée dans le surnageant par RP-HPLC. Il en résulte que l'insuline glargine se retrouve majoritairement sous une forme précipitée. En présence des 4 insulines prandiales testées, l'insuline glargine précipite dans le mélange PBS/BSA. Les résultats sont regroupés dans le tableau 6.

Tableau 6 : Essais de solubilisation et de précipitation d'une composition polymère anionique hydrophobisé AB1/insuline glargine/insuline prandiale 75/25

| Nature de l'insuline prandiale | Solubilisation insuline glargine/insuline prandiale 75/25 | Précipitation de l'insuline glargine |
|---|---|---|
| Insuline qlulisine (Apidra®) | Oui | Oui |
| Insuline aspart (NovoLog®) | Oui | Oui |
| Insuline lispro (Humalog®) | Oui | Oui |
| Insuline humaine (ActRapid®) | Oui | Oui |

**Exemple B18 : Préparation d'une solution d'insuline analogue lente (insuline glargine) concentrée.**

Une solution commerciale d'insuline glargine commercialisée par la société SANOFI-AVENTIS sous le nom de Lantus® est concentrée par ultrafiltration sur une membrane 3 kDa en cellulose régénérée (Amicon® Ultra-15 commercialisée par la société Millipore). A l'issue de cette étape d'ultrafiltration la concentration en insuline glargine est dosée dans le rétentat par RP-HPLC. La concentration finale en insuline glargine est ensuite ajustée par l'ajout de solution commerciale d'insuline glargine à 100 UI/mL pour obtenir la concentration finale souhaitée. Ce procédé permet d'obtenir des solutions concentrées d'insuline glargine notées $C_{insuline\ glargine}$ à diverses concentrations supérieures à 100 UI/mL, telles que $C_{insuline\ glargine}$ = 200, 250, 300 et 333 UI/mL. Les solutions concentrées sont filtrées sur 0,22 $\mu$m puis stockées à +4°C.

**Exemple B19 : Dialyse d'une solution commerciale d'insuline analogue rapide (insuline lispro).**

Une solution commerciale d'insuline lispro (Exemple B2) commercialisée par la société ELI LILLY sous le nom d'Humalog® est dialysée par ultrafiltration sur une membrane 3 kDa en cellulose régénérée (Amicon® Ultra-15 commercialisée par la société Millipore). La dialyse est réalisée dans un tampon phosphate 1 mM à pH 7. A l'issue de cette étape de dialyse, la concentration $C_{insuline\ lispro\ dialysé}$ dans le rétentat est déterminée par RP-HPLC. La solution dialysée est conservée dans un congélateur à - 80°C.

**Exemple B20 : Lyophilisation d'une solution d'insuline analogue rapide (insuline lispro) sous sa forme commerciale.**

Un volume $V_{Humalog}$ d'une solution d'insuline rapide lispro (Exemple B2) à une concentration de 100 UI/mL dans sa forme commerciale est placé dans un lyogard® préalablement stérilisé dans un autoclave. Le lyogard® est placé dans un congélateur à -80°C pendant environ 1h, puis une lyophilisation avec les paramètres de température 20°C et de pression 0,31 mbar est effectuée.

Le lyophilisat stérile ainsi obtenu est conservé à température ambiante.

**Exemple B21 : Lyophilisation d'une solution commerciale d'insuline analogue rapide (insuline lispro) dialysée.**

Un volume $V_{Humalog\ dialysé}$ d'une solution d'insuline rapide lispro obtenu selon l'exemple B19 à une concentration de $C_{insuline\ lispro}$ dialysé est placé dans un lyogard® préalablement stérilisé dans un autoclave. Le lyogard® est placé dans un congélateur à -80°C pendant environ 1h, puis une lyophilisation avec les paramètres de température 20°C et de pression 0,31 mbar est effectuée.

Le lyophilisat stérile ainsi obtenu est conservé à température ambiante.

**Exemple B22 : Préparation d'une composition polymère anionique hydrophobisé /insuline glargine à pH 7, suivant un procédé utilisant de l'insuline glargine concentrée sous forme liquide (en solution) et un polymère anionique hydrophobisé sous forme solide (lyophilisée).**

Une masse $m_{polymère\ anionique\ hydrophobisé}$ de polymère anionique hydrophobisé est pesée précisément. Ce lyophilisat est repris par un volume $V_{insuline\ glargine}$ d'une solution concentrée d'insuline glargine préparée selon l'exemple B18 de manière à obtenir une composition présentant une concentration de polymère anionique hydrophobisé $C_{polymère\ anionique\ hydrophobisé}$ (mg/mL) = $m_{polymère\ anionique\ hydrophobisé}$ /$V_{insuline\ glargine}$ et une concentration en insuline glargine $C_{insuline\ glargine}$ (UI/mL). La solution est opalescente. Le pH de cette solution est d'environ 6,3. Le pH est ajusté à 7 par ajout de NaOH concentrée puis la solution est placée en statique dans une étuve à 37°C pendant environ 1 heure jusqu'à solubilisation complète. Un volume $V_{polymère\ anionique\ hydrophobisé\ /insuline\ glargine}$ de cette solution visuellement limpide est placée à +4°C.

**Exemple B23 : Préparation d'une composition polymère anionique hydrophobisé /insuline glargine à pH 7, suivant un procédé utilisant de l'insuline glargine sous forme liquide (en solution) et un polymère anionique hydrophobisé sous forme liquide (en solution).**

A une solution mère de polymère anionique hydrophobisé à pH 7 présentant une concentration $C_{mère\ polymère\ anionique\ hydrophobisé}$ sont ajoutées des solutions concentrées de m-crésol, glycérine et Tween® 20 de manière à obtenir une solution de polymère anionique hydrophobisé de concentration $C_{mère\ polymère\ anionique\ hydrophobisé/excipients}$ (mg/mL) en présence de ces excipients à des teneurs équivalentes à celles décrites dans la solution commerciale Lantus® en flacon de 10 mL.

Dans un pot stérile, un volume $V_{Lantus}$ d'une solution commerciale d'insuline lente glargine commercialisée sous le nom de Lantus® à une concentration de 100 UI/mL est ajouté à un volume $V_{mère\ polymère\ anionique\ hydrophobisé\ /excipients}$ d'une solution de polymère anionique hydrophobisé à la concentration $C_{mère\ polymère\ anionique\ hydrophobisé\ /excipients}$ (mg/mL). Un trouble apparaît. Le pH est ajusté à pH 7 par ajout de NaOH concentrée et la solution est placée en statique dans une étuve à 37°C pendant environ 1h jusqu'à solubilisation complète. Cette solution visuellement limpide est placée à +4°C.

**Exemple B24** : **Préparation d'une composition polymère anionique hydrophobisé /insuline glargine concentrée à pH = 7, suivant un procédé de concentration d'une composition diluée.**

Une composition polymère anionique hydrophobisé /insuline glargine diluée à pH 7 décrite dans l'exemple B23 est concentrée par ultrafiltration sur une membrane 3 kDa en cellulose régénérée (Amicon® Ultra-15 commercialisée par la société Millipore). A l'issue de cette étape d'ultrafiltration, le rétentat est limpide et la concentration en insuline glargine dans la composition est déterminée par RP-HPLC. Si nécessaire, la concentration en insuline glargine dans la composition est ensuite ajustée à la valeur souhaitée par dilution dans une solution d'excipients m-crésol/Glycérine/Tween®20 présentant, pour chaque espèce une concentration équivalente à celle décrite dans la solution commerciale Lantus® (en flacon de 10 mL). Cette solution à pH 7, visuellement limpide, présentant une concentration en insuline glargine $C_{insuline\ glargine}$ (UI/mL) et une concentration en polymère anionique hydrophobisé $C_{polymère\ anionique\ hydrophobisé}$ (mg/mL), est placée à +4°C.

**Exemple B25 : Préparation d'une composition polymère anionique hydrophobisé /insuline glargine/insuline lispro à pH 7, à partir d'un lyophilisat d'une insuline rapide lispro sous sa forme commerciale (Humalog®).**

Un volume $V_{polymère\ anionique\ hydrophobisé\ /insuline\ glargine}$ de Solution de polymère anionique hydrophobisé /insuline glargine à pH 7 présentant une concentration en insuline glargine $C_{insuline\ glargine}$ (UI/mL) et une concentration de polymère anionique hydrophobisé $C_{polymère\ anionique\ hydrophobisé}$ (mg/mL) préparé selon l'exemple B22 est ajouté sur un lyophilisat

d'insuline lispro obtenu par lyophilisation d'un volume $V_{insuline\ lispro}$ dont la préparation est décrite dans l'exemple B20, de telle manière que le ratio $V_{polymère\ anionique\ hydrophobisé\ /insuline\ glargine}$ /$V_{insuline\ lispro}$ = 100 / $C_{insuline\ lispro}$ où $C_{insuline\ lispro}$ est la concentration en insuline lispro (UI/mL) visée dans la composition. La solution est limpide. La teneur en zinc de la formulation est ajustée à la concentration $C_{zinc}$ (µM) souhaitée par ajout d'une solution concentrée de chlorure de zinc. Le pH final est ajusté à 7 par ajout de NaOH ou HCl concentré.

La formulation est limpide, attestant de la bonne solubilité de l'insuline glargine et de l'insuline lispro dans ces conditions de formulation. Cette solution est filtrée sur 0,22 µm et placée à +4°C.

**Exemple B26 : Préparation d'une composition polymère anionique hydrophobisé /insuline glargine/insuline lispro à pH 7, à partir d'un lyophilisat d'une insuline rapide lispro obtenue par dialyse d'une solution commerciale (Humalog®).**

Un volume $V_{polymère\ anionique\ hydrophobisé\ /insuline\ glargine}$ de solution de polymère anionique hydrophobisé /insuline glargine pH 7 présentant une concentration en insuline glargine $C_{insuline\ glargine}$ (UI/mL) et une concentration de polymère anionique hydrophobisé $C_{polymère\ anionique\ hydrophobisé}$ (mg/mL) préparé selon l'exemple B22 est ajouté sur un lyophilisat d'insuline lispro obtenu par lyophilisation d'un volume $V_{insuline\ lispro\ dialysé}$ dont la préparation est décrite dans l'exemple B21, de telle manière que le ratio $V_{polymère\ anionique\ hydrophobisé\ /insuline\ glargine}$/$V_{insuline\ lispro\ dialysé}$ = $C_{insuline\ lispro\ dialysé}$/$C_{insuline\ lispro}$ où $C_{insuline\ lispro}$ dialysé est la concentration en insuline lispro (UI/mL) obtenue à l'issue de la dialyse de la solution commerciale, étape décrite dans l'exemple B19, et $C_{insuline\ lispro}$ est la concentration en insuline lispro (UI/mL) visée dans la composition. La solution est limpide. La teneur en zinc de la formulation est ajustée à la concentration $C_{zinc}$ (µM) souhaitée par ajout d'une solution concentrée de chlorure de zinc. Le pH final est ajusté à 7 par ajout de NaOH ou HCl concentré.

La formulation est limpide, attestant de la bonne solubilité des insulines glargine et lispro dans ces conditions de formulation. Cette solution est filtrée sur 0,22 µm et placée à +4°C.

**Exemple B27 : Préparation d'une composition polymère anionique hydrophobisé AB1/insuline glargine/insuline lispro à pH 7 présentant une concentration en insuline glargine de 200 UI/mL et une concentration d'insuline lispro de 66 UI/mL (proportion en pourcentage d'insuline : 75/25 en insuline glargine/insuline lispro).**

Une solution d'insuline glargine concentrée à 200 UI/mL est préparée selon l'exemple B18. Une composition polymère anionique hydrophobisé AB1 (15 mg/mL)/insuline glargine 200 UI/mL pH 7 est préparée à partir d'un polymère anionique hydrophobisé AB1 et selon le mode de préparation décrit à l'exemple B22. Cette composition polymère anionique hydrophobisé AB1/insuline glargine 200 UI/mL est ajoutée à un lyophilisat d'insuline lispro obtenu par lyophilisation de la solution d'analogue rapide issue de la dialyse d'une solution commerciale, selon le mode de préparation décrit dans l'exemple B26. La solution est limpide. La teneur en zinc de la formulation est ajustée à la concentration souhaitée par ajout d'une solution concentrée de chlorure de zinc. Le pH final est ajusté à 7 par ajout de NaOH ou HCl concentré.

La formulation est limpide, attestant de la bonne solubilité des insulines glargine et lispro dans ces conditions de formulation. Cette solution est filtrée sur 0,22 µm et placée à +4°C.

**Exemple B28 : Préparation d'une composition polymère anionique hydrophobisé AB1/insuline glargine/insuline lispro à pH 7 présentant une concentration en insuline glargine de 300 UI/mL et une concentration en insuline lispro de 100 UI/mL (proportion en pourcentage d'insuline : 75/25 en insuline glargine/insuline lispro).**

Une solution d'insuline glargine concentrée à 300 UI/mL est préparée selon l'exemple B18. Une composition polymère anionique hydrophobisé AB1 (23 mg/mL)/insuline glargine 300 UI/mL pH 7 est préparée à partir du polymère anionique hydrophobisé AB1 et selon le mode de préparation décrit à l'exemple B22. Cette composition polymère anionique hydrophobisé AB1/insuline glargine 300 UI/mL est ajoutée à un lyophilisat d'insuline lispro obtenu par lyophilisation de la solution d'analogue rapide issue de la dialyse d'une solution commerciale, selon le mode de préparation décrit dans l'exemple B26. La solution est limpide. La teneur en zinc de la formulation est ajustée à la concentration souhaitée par ajout d'une solution concentrée de chlorure de zinc. Le pH final est ajusté à 7 par ajout de NaOH ou HCl concentré.

La formulation est limpide, attestant de la bonne solubilité des insulines glargine et lispro dans ces conditions de formulation. Cette solution est filtrée sur 0,22 µm et placée à +4°C.

Des compositions polymère anionique hydrophobisé/insuline glargine/insuline lispro 300/100 à pH 7 ont également été préparées avec d'autres polymères anioniques hydrophobisés selon un mode de préparation identique à celui décrit dans l'exemple B28 avec une concentration en polymère anionique hydrophobisé de 30 mg/mL. Ces formulations sont limpides, attestant de la bonne solubilité des insulines glargine et lispro dans ces conditions de formulation.

Ces compositions conduisent aux exemples décrits dans le tableau 7.

Tableau 7

| Exemple | Polymère anionique hydrophobisé | $C_{\text{polymère anionique hydrophobisé}}$ (mg/mL) | $C_{\text{insuline glargine}}$ (UI/mL) | $C_{\text{insuline lispro}}$ (UI/mL) | $C_{\text{insuline glargine}}/C_{\text{insuline lispro}}$ (%/%) |
|---|---|---|---|---|---|
| B29 | AB2 | 30 | 300 | 100 | 75/25 |
| B30 | AB3 | 30 | 300 | 100 | 75/25 |
| B31 | AB4 | 30 | 300 | 100 | 75/25 |
| B32 | AB5 | 30 | 300 | 100 | 75/25 |
| B33 | AB6 | 30 | 300 | 100 | 75/25 |
| B34 | AB7 | 30 | 300 | 100 | 75/25 |
| B35 | AB8 | 30 | 300 | 100 | 7525 |
| B36 | AB9 | 30 | 300 | 100 | 75/25 |
| B37 | AB10 | 30 | 300 | 100 | 7525 |

**Exemple B38: Préparation d'une composition polymère anionique hydrophobisé AB2/insuline glargine/insuline lispro à pH 7 présentant une concentration en insuline glargine de 200 UI/mL et une concentration de insuline lispro de 66 UI/mL (proportion en pourcentage d'insuline : 75/25 en insuline glargine/insuline lispro).**

Une composition polymère anionique hydrophobisé AB2 (20mg/mL)/insuline glargine 200 UI/mL pH 7 est préparée à partir d'un polymère anionique hydrophobisé AB2 et selon les modes de préparations décrit à l'exemple B22. Cette composition polymère anionique hydrophobisé AB2 /insuline glargine 200 UI/mL est ajoutée à un lyophilisat d'insuline lispro obtenu par lyophilisation de la solution d'analogue rapide issue de la dialyse d'une solution commerciale, selon le mode de préparation décrit dans l'exemple B26. La solution est limpide. La teneur en zinc de la formulation est ajustée à la concentration souhaitée par ajout d'une solution concentrée de chlorure de zinc. Le pH final est ajusté à 7 par ajout de NaOH ou HCl concentré.

La formulation est limpide, attestant de la bonne solubilité des insulines glargine et lispro dans ces conditions de formulation. Cette solution est filtrée sur 0,22 μm et placée à +4°C.

Des compositions polymère anionique hydrophobisé/insuline glargine/insuline lispro 200/66 à pH 7 ont également été préparées avec d'autres polymères anioniques hydrophobisés selon un mode de préparation identique à celui décrit dans l'exemple B38 avec une concentration en polymère anionique hydrophobisé de 20 mg/ml. Ces formulations sont limpides, attestant de la bonne solubilité des insulines glargine et lispro dans ces conditions de formulation. Ces compositions conduisent aux exemples décrits dans le tableau 8.

Tableau 8

| Exemple | Polymère anionique hydrophobisé | $C_{\text{polymère anionique hydrophobisé}}$ (mg/mL) | $C_{\text{insuline glargine}}$ (UI/mL) | $C_{\text{insuline lispro}}$ (UI/mL) | $C_{\text{insuline glargine}}/C_{\text{insuline lispro}}$ (%/%) |
|---|---|---|---|---|---|
| B39 | AB3 | 20 | 200 | 66 | 75/25 |
| B40 | AB4 | 20 | 200 | 66 | 75/25 |
| B41 | AB5 | 20 | 200 | 66 | 75/25 |
| B42 | AB6 | 20 | 200 | 66 | 75/25 |
| B43 | AB7 | 20 | 200 | 66 | 75/25 |
| B44 | AB8 | 20 | 200 | 66 | 75/25 |
| B45 | AB9 | 20 | 200 | 66 | 75/25 |
| B46 | AB10 | 20 | 200 | 66 | 75/25 |

**Exemple B47 : Préparation d'une composition polymère anionique hydrophobisé AB1/insuline glargine/insuline lispro à pH 7 présentant une concentration en insuline glargine de 250 UI/mL et une concentration en insuline lispro de 150 UI/mL (proportion en pourcentage d'insuline : 63/37 en insuline glargine/insuline lispro).**

Une composition polymère anionique hydrophobisé AB1 (19 mg/mL)/insuline glargine 250 UI/mL pH 7 est préparée à partir d'un polymère anionique hydrophobisé AB1 et selon les modes de préparations décrit à l'exemple B22. Cette composition polymère anionique hydrophobisé AB1/insuline glargine 200 UI/mL est ajoutée à un lyophilisat d'insuline lispro obtenu par lyophilisation de la solution d'analogue rapide issue de la dialyse d'une solution commerciale, selon le mode de préparation décrit dans l'exemple B26. La solution est limpide. La teneur en zinc de la formulation est ajustée à la concentration souhaitée par ajout d'une solution concentrée de chlorure de zinc. Le pH final est ajusté à 7 par ajout de NaOH ou HCl concentré.

La formulation est limpide, attestant de la bonne solubilité des insulines glargine et lispro dans ces conditions de formulation. Cette solution est filtrée sur 0,22 µm et placée à +4°C.

Des compositions polymère anionique hydrophobisé/insuline glargine/insuline lispro 250/150 à pH 7 ont également été préparées avec d'autres polymères anioniques hydrophobisés selon un mode de préparation identique à celui décrit dans l'exemple B47 avec une concentration en polymère anionique hydrophobisé de 25 mg/mL. Ces formulations sont limpides, attestant de la bonne solubilité des insulines glargine et lispro dans ces conditions de formulation. Ces compositions conduisent aux exemples décrits dans le tableau 9.

Tableau 9

| Exemple | Polymère anionique hydrophobisé | $C_{\text{polymère anionique hydrophobisé}}$ (mg/mL) | $C_{\text{insuline glargine}}$ (UI/mL) | $C_{\text{insuline lispro}}$ (UI/mL) | $C_{\text{insuline glargine}}/C_{\text{insuline lispro}}$ (%/%) |
|---|---|---|---|---|---|
| B48 | AB2 | 25 | 250 | 150 | 63/37 |
| B49 | AB3 | 25 | 250 | 150 | 63/37 |
| B50 | AB4 | 25 | 250 | 150 | 63/37 |
| B51 | AB5 | 25 | 250 | 150 | 63/37 |
| B52 | AB6 | 25 | 250 | 150 | 63/37 |
| B53 | AB7 | 25 | 250 | 150 | 63/37 |
| B54 | AB8 | 25 | 250 | 150 | 63/37 |
| B55 | AB9 | 25 | 250 | 150 | 63/37 |
| B56 | AB10 | 25 | 250 | 150 | 63/37 |

**Exemple B57: Préparation d'une composition polymère anionique hydrophobisé AB1/insuline glargine/insuline lispro à pH 7 présentant une concentration en insuline glargine de 300 UI/mL et une concentration d'insuline lispro de 100 UI/mL (proportion en pourcentage d'insuline : 75/25 en insuline glargine/insuline lispro).**

Une composition polymère anionique hydrophobisé AB1 (13 mg/mL)/insuline glargine 300 UI/mL pH 7 est préparée à partir d'un polymère anionique hydrophobisé AB1 et selon les modes de préparations décrit aux exemples B23 et B24. Cette composition polymère anionique hydrophobisé AB1/insuline glargine 300 UI/mL est ajoutée à un lyophilisat d'insuline lispro obtenu par lyophilisation de la solution d'analogue rapide issue de la dialyse d'une solution commerciale, selon le mode de préparation décrit dans l'exemple B26. La solution est limpide. La teneur en zinc de la formulation est ajustée à la concentration souhaitée par ajout d'une solution concentrée de chlorure de zinc. Le pH final est ajusté à 7 par ajout de NaOH ou HCl concentré.

La formulation est limpide, attestant de la bonne solubilité des insulines glargine et lispro dans ces conditions de formulation. Cette solution est filtrée sur 0,22 µm et placée à +4°C.

**Exemple B58** : **Précipitation de différentes compositions polymère anionique hydrophobisé /insuline glargine/insuline lispro à pH 7 présentant différentes concentrations en insulines glargine et lispro.**

1 mL de composition polymère anionique hydrophobisé /insuline glargine/insuline lispro préparée aux exemples B27 à B57 est ajouté dans 2 mL d'une solution de PBS contenant 20 mg/mL de BSA. Le mélange PBS/BSA simule la composition du milieu sous-cutané. Un précipité apparaît. Une centrifugation à 4000 trs/min est effectuée pour séparer

le précipité du surnageant. Ensuite, l'insuline glargine est dosée dans le surnageant par RP-HPLC. Il en résulte que l'insuline glargine se retrouve majoritairement sous une forme précipitée.

Les résultats de solubilisation et de précipitation sont résumés dans le tableau 10.

Tableau 10 : Essais de solubilisation et de précipitation de différentes compositions polymère anionique hydrophobisé /insuline glargine/insuline lispro à pH 7 présentant différentes concentrations en insuline glargine et lispro

| Exemples | Solubilisation insuline glargine et insuline lispro à pH 7 | Précipitation de l'insuline glargine |
|---|---|---|
| B27 | OUI | OUI |
| B28 | OUI | OUI |
| B29 | OUI | OUI |
| B30 | OUI | OUI |
| B31 | OUI | OUI |
| B32 | OUI | OUI |
| B33 | OUI | OUI |
| B34 | OUI | OUI |
| B35 | OUI | OUI |
| B36 | OUI | OUI |
| B37 | OUI | OUI |
| B38 | OUI | OUI |
| B39 | OUI | OUI |
| B40 | OUI | OUI |
| B41 | OUI | OUI |
| B42 | OUI | OUI |
| B43 | OUI | OUI |
| B44 | OUI | OUI |
| B45 | OUI | OUI |
| B46 | OUI | OUI |
| B47 | OUI | OUI |
| B48 | OUI | OUI |
| B49 | OUI | OUI |
| B50 | OUI | OUI |
| B51 | OUI | OUI |
| B52 | OUI | OUI |
| B53 | OUI | OUI |
| B54 | OUI | OUI |
| B55 | OUI | OUI |
| B56 | OUI | OUI |
| B57 | OUI | OUI |

**Exemple B59** : **Solubilisation de l'insuline glargine à 1 mg/mL et à pH 7 à l'aide du polymère anionique hydrophobisé AB1 ou de AC1 à la concentration de 10 mg/mL**

10 mg d'un polymère anionique hydrophobisé AB1 ou de AC1 sont pesés précisément. Ce lyophilisat est repris par 1 mL d'une solution d'insuline glargine à 1 mg/mL obtenue par dilution de la solution commerciale de l'exemple B4. Ce mélange conduit à l'obtention d'une solution dont la concentration en polymère anionique hydrophobisé est égale à 10 mg/mL et la concentration en insuline glargine est égale à 1 mg/mL. Le pH est ajusté à 7 avec une solution de soude 0,1 N. La solution est limpide. Cette solution est filtrée sur membrane (0,22 $\mu$m) puis est placée à +4°C.

**Exemple B60** : **Solubilisation de la BMP-7 à 1 mg/mL et à pH 7 à l'aide du polymère anionique hydrophobisé AB1 ou de AC1 à la concentration de 10 mg/mL**

La Bone Morphogenetic Protein 7 (BMP-7) est soluble à pH acide et possède une limite de solubilité très basse à pH 7, de l'ordre de quelques microgrammes/mL.

10 mg du polymère anionique hydrophobisé AB1 ou de AC1 sont pesés précisément. Ce est repris par 1 mL d'une solution de BMP-7 à 1 mg/mL à pH acide, par exemple dans un tampon lactate 10 mM à pH 3. Ce mélange conduit à l'obtention d'une solution dont la concentration en polymère anionique hydrophobisé AB1 est égale à 10 mg/mL et la concentration en BMP-7 est égale à 1 mg/mL. Après mélange le pH final est ajusté à 7 par ajout d'une solution de soude 0,1 N. La solution est limpide. Cette solution est filtrée sur membrane (0,22 $\mu$m) puis est placée à +4°C.

**Exemple B61 : Précipitation d'une composition polymère anionique hydrophobisé / insuline glargine.**

1 mL de solution polymère anionique hydrophobisé / insuline glargine préparée à l'exemple B59 est ajouté dans 2 mL d'une solution de PBS contenant 20 mg/mL de BSA. Le mélange PBS/BSA simule la composition du milieu sous-cutané. Un précipité apparaît.

Une centrifugation à 4000 trs/min est effectuée pour séparer le précipité du surnageant. Ensuite l'insuline glargine est dosée dans le surnageant par RP-HPLC. Il en résulte que l'insuline glargine se retrouve majoritairement sous une forme précipitée. Les résultats sont résumés dans le tableau 11.

**Exemple B62** : **Précipitation d'une composition polymère anionique hydrophobisé / BMP-7.**

1 mL de solution polymère anionique hydrophobisé / BMP-7 préparée à l'exemple B60 est ajouté dans 2 mL d'une solution de PBS contenant 20 mg/mL de BSA. Le mélange PBS/BSA simule la composition du milieu sous-cutané. La solution est limpide, aucun précipité n'est observé. Les résultats sont résumés dans le tableau 11 ci-dessous.

Tableau 11 : Essais de solubilisation et de précipitation d'une composition polymère anionique hydrophobisé AB1 ou de AC1 avec l'insuline glargine ou avec BMP-7

| Polymère 10 mg/mL | Protéine 1 mg/mL | Solubilisation protéine | Précipitation protéine |
|---|---|---|---|
| AC1 | BMP-7 | Oui | Non |
| AB1 | | Oui | Non |
| AC1 | glargine | Oui | Oui |
| AB1 | | Oui | Oui |

Ces résultats montrent que le comportement de la BMP-7 est différent de celui de l'insuline glargine, en particulier dans les conditions simulant milieu sous-cutané. En effet, dans ces conditions, les compositions comprenant de l'insuline glargine conduisent à la précipitation de l'insuline glargine, alors qu'avec les compositions comprenant de la BMP-7, celle-ci reste soluble.

**C Pharmacodynamie**

**C1. Protocole de mesure de la pharmacodynamie des solutions d'insuline**

Une étude pré-clinique a été réalisée sur porcs en vue d'évaluer une composition selon l'invention :

insuline glargine/insuline lispro (75/25), formulée avec le polymère anionique hydrophobisé AB1 (13 mg/mL) décrit

à l'exemple B57.

Les effets hypoglycémiants de ces compositions ont été comparés à ceux obtenus après injections simultanées de Lantus® (pH 4) et d'une insuline prandiale Humalog® dans les mêmes proportions (75/25) et à la même dose totale.

Dix cochons domestiques d'environ 50 kg, préalablement cathétérisés au niveau de la jugulaire, sont mis à jeun 2,5 heures avant le début de l'expérience. Dans l'heure précédant l'injection d'insuline, 3 prélèvements sanguins sont réalisés afin de déterminer le niveau basal de glucose.

L'injection d'insuline à la dose de 0,3 UI/kg est réalisée en injection sous-cutanée au niveau du cou, sous l'oreille de l'animal à l'aide du stylo à insuline Novopen® junior équipé d'une aiguille 31 G.

Des prélèvements sanguins sont ensuite réalisés après 10, 20, 30, 40, 50 minutes et 1, 1,5, 2, 3, 4, 5, 6, 7, 8, 9, 10, 11, 12, 13, 14, 15 et 16 heures. Après chaque prélèvement, le cathéter est rincé avec une solution diluée d'héparine.

Une goutte de sang est prélevée pour déterminer la glycémie au moyen d'un glucomètre.

La courbe de pharmacodynamie moyenne du glucose exprimée en pourcents du niveau basal est représentée dans la Figure 1.

Figure 1 : Courbes moyennes de glycémie ± erreur standard de la moyenne pour les administrations simultanées de Humalog® (100 UI/mL, 0,075 UI/kg) et Lantus® (100 UI/mL, 0,225 UI/kg) en comparaison avec l'administration d'une formulation selon l'invention décrite dans l'exemple B57 (400 UI/mL, 0,3 UI/kg).

**C2.** Résultats de pharmacodynamie de la solution d'insuline de l'exemple B57

Les résultats de pharmacodynamie obtenus avec les administrations séquentielles de Humalog® et Lantus® en comparaison avec la formulation décrite dans l'exemple B57 sont présentés sur la figure 1. L'activité hypoglycémiante de la formulation décrite dans l'exemple B57 est biphasique. La première phase, rapide, est caractérisée par une décroissance marquée de la glycémie durant les 30 premières minutes (similaire à celle induite par la double injection de Lantus® / Humalog®), indiquant que la présence du polymère anionique hydrophobisé AB1 ne perturbe pas le caractère rapide d'Humalog®. Après 30 minutes, la glycémie remonte jusqu'à 3 heures avant une seconde phase caractérisée par une activité hypoglycémiante moins marquée et prolongée jusqu'à 16 heures post-injection. Cette seconde phase apparait similaire pour les deux formulations indiquant que l'effet basal de glargine est bien conservé dans la formulation selon l'invention décrite dans l'exemple B57.

## Revendications

1. Composition sous forme d'une solution aqueuse injectable, dont le pH est compris entre 6,6 et 7,8, comprenant au moins :

   a) une insuline basale dont le point isoélectrique pI est compris entre 5,8 et 8,5 ;
   b) un polymère anionique hydrophobisé de formule XII :

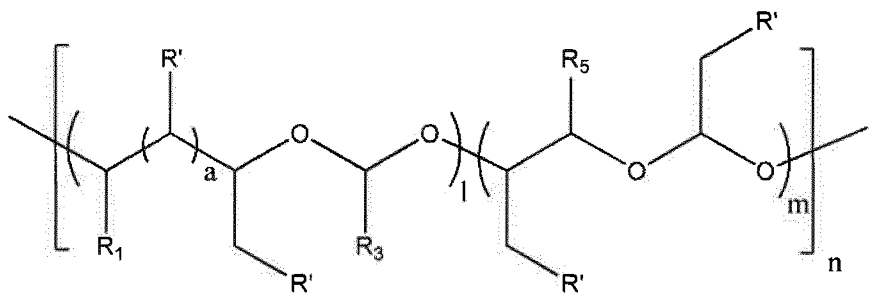

**Formule XII**

dans laquelle,

- l = 0 ou 1,
- m = 0, 1 ou 2,
- a = 0 ou 1,
- n étant le degré de polymérisation, compris entre 3 et 1000, et,

et

- $-R_1$ est un hydrogène -H,
- $-R_3$ est un radical $-CH_2R'$,
- $-R_5$ est soit un groupe -COOH, soit un radical $-CH_2R'$, soit un radical -*k*-[D], dans lequel :

  - -[D] est un radical -[Hy] ou -[E]-(o-[Hy])t ;
  - -[E]- est un radical au moins divalent comprenant de 1 à 15 atomes de carbone comprenant au moins un hétéroatome choisi parmi O, N et S, éventuellement porteur de fonctions carboxyle ou amine et/ou - k-[E]-(o)t, comprenant de 2 à 16 atomes de carbone, est issu d'un acide aminé, d'un dialcool, d'une diamine, ou d'un amine-alcool ;
  - -[Hy] est un groupe alkyle, linéaire ou cyclique, un alkylaryle ou un arylalkyle, en C8 à C30, éventuellement substitué par un ou plusieurs groupes alkyles en C1 à C3, issu d'un composé hydrophobe ;
  - *k* résultant de la réaction entre une fonction carboxyle, amine ou alcool du précurseur de -*k*-[E]-$(o)_t$ et une fonction alcool, carboxyle ou amine du polymère et est une fonction choisie dans le groupe constitué par les fonctions ester, amide, carbonate et carbamate;
  - *o* résultant de la réaction entre une fonction carboxyle, amine ou alcool du précurseur de -k-[E]-(o)t et une fonction alcool ou acide du précurseur de -[Hy] est une fonction choisie dans le groupe constitué par les fonctions ester, amide, urée (carbamide), carbonate et carbamate ;
  - t est un entier positif égal à 1 ou 2 ;

ou

- $-R_1$ et $-R_3$ forment un cycle à six chaînons $-R_1-R_3- = -CH(NHCOCH_3)-$, et,
- -R' est choisi dans le groupe constitué par les radicaux :

  ◦ -OH
  ◦ -O-Alk, Alk étant une chaine alkyle en C1 à C3,
  ◦ -(*f*-[A]-COOH), dans lequel :

    - -[A]- est un radical au moins divalent comprenant de 1 à 15 atomes de carbones comprenant au moins un hétéroatome choisi parmi O, N et S, éventuellement porteur de fonctions carboxyle ou amine et/ou
    - *f*-[A]-COOH est issu d'un acide aminé, d'un diacide ou d'un alcoolacide et est lié au squelette de la molécule par une fonction f ;
    - f résultant de la réaction entre une fonction carboxyle ou alcool ou amine du précurseur de -*f*-[A]-COOH et une fonction hydroxyle du squelette est choisie dans le groupe constitué par les fonctions éther, ester carbamate ou carbonate ;

  o -*g*-[B]-$(k\text{-}[D])_p$, dans lequel :

    - -[B]- est un radical au moins divalent comprenant de 1 à 15 atomes de carbones comprenant au moins un hétéroatome choisi parmi O, N et S, éventuellement porteurs de fonctions carboxyle ou amine et/ou
    - *g*-[B]-$(k\text{-})_p$ est issu d'un acide aminé, d'un diacide, d'un dialcool, d'un alcoolacide, d'une diamine ou d'un amine-alcool et est lié au squelette de la molécule par une fonction g et est lié à au moins un radical -[D] par une fonction k,
    - g résultant de la réaction entre une fonction carboxyle ou alcool ou amine du précurseur de -*g*-[B]-$(k\text{-})_p$ et une fonction du squelette est choisie dans le groupe constitué par les fonctions éther, amine, ester, carbamate ou carbonate,
    - k résultant de la réaction entre une fonction carboxyle ou alcool ou amine du précurseur de -*g*-[B]-$(k\text{-})_p$ et une fonction alcool ou acide du précurseur de -[D] choisie dans le groupe constitué par les fonctions ester, amide ou carbamate ;
    - p est un entier positif égal à 1 ou 2;

et, -[A]-, -[B]- et -[E]- sont identiques ou différents,
et k et *o* sont identiques ou différentes ;
et, si -[B]- est un radical trivalent, alors -[D] est un radical -[Hy],
et, le degré de substitution en charges carboxylates est le nombre moyen de charges carboxylates par monomère divisé par (l+m) et est supérieur ou égal à 0,4,
et, le degré de substitution en radicaux hydrophobes est le nombre moyen de radicaux hydrophobes par monomère

divisé par (l+m) et est inférieur ou égal à 0,5, et, si le polymère anionique hydrophobisé est un polysaccharide, alors les liaisons glycosidiques identiques ou différentes, peuvent être de type $\alpha$ et/ou de type $\beta$.

2. Composition selon la revendication 1, **caractérisée en ce que** le polymère anionique hydrophobisé est choisi parmi les polymères anioniques hydrophobisés de formule XII, dans laquelle, l = 0 et m = 1, choisi parmi les polymères anioniques hydrophobisés de formule IV :

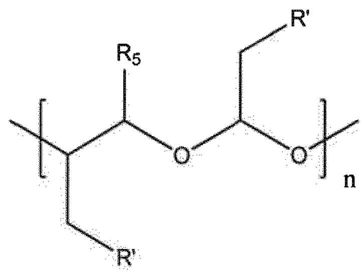

Formule IV

dans laquelle, -Rs est soit un groupe -COOH, soit un radical $-CH_2R'$, soit un radical -*k*-[D], -R' et n tels que définis en revendication 1.

3. Composition selon la revendication 2, **caractérisée en ce que** le polymère anionique hydrophobisé est choisi parmi les polymères anioniques hydrophobisés de formule IV :

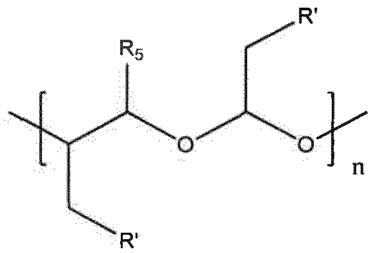

Formule IV

dans laquelle, -Rs est un radical $-CH_2R'$, -R' et n sont tels que définis en revendication 1.

4. Composition selon la revendication 1, **caractérisée en ce que** le polymère anionique hydrophobisé est choisi parmi les polymères anioniques hydrophobisés de formule XII, dans laquelle, m = 0, l = 1, a = 0, autrement exprimé, il est choisi parmi les polymères anioniques hydrophobisés de formule V :

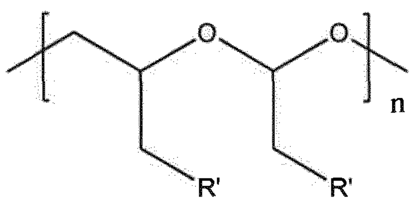

Formule V

et, -R' et n sont tels que définis en revendication 1.

5. Composition selon la revendication 1, **caractérisée en ce que**, le polymère anionique hydrophobisé est choisi parmi les polymères anioniques hydrophobisés de formule XII, dans laquelle, l = 1, m = 2 et a = 0, autrement exprimé, il est choisi parmi les polymères anioniques hydrophobisés de formule VI :

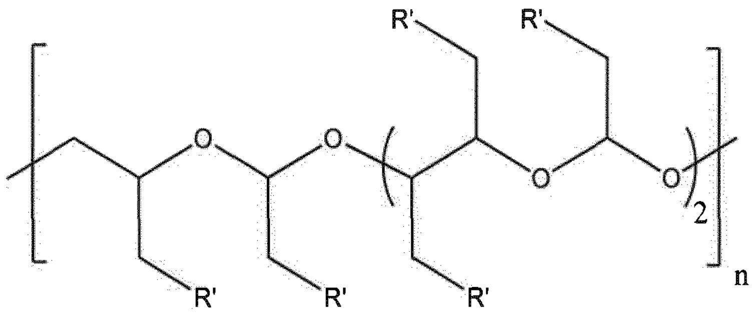

Formule VI

et, -R' et n sont tels que définis en revendication 1.

**6.** Composition selon la revendication 1, **caractérisée en ce que** le polymère anionique hydrophobisé est choisi parmi les polymères anioniques hydrophobisés de formule XII, dans laquelle, m = 1, l = 1, a = 1, $-R_1-R_3-$ = $-CH(NHCOCH_3)-$, $-R_2 = -CH_2R'$, $-R_4 = -CH_2R'$, -Rs est soit un groupe -COOH, soit un radical -k-[D], $-R_6 = -CH_2R'$, autrement exprimé, il est choisi parmi les polymères anioniques hydrophobisés de formule VII :

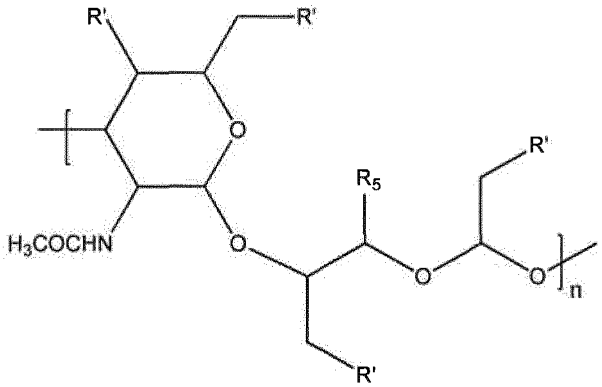

Formule VII

R' et n sont tels que définis en revendication 1.

**7.** Composition selon l'une quelconque des revendications précédentes, **caractérisée en ce que** le polymère anionique hydrophobisé est choisi parmi les polymères anioniques hydrophobisés de formules IV à VII et XII, dans lesquelles le radical -f-[A]-COOH est choisi parmi les radicaux de formule II suivante :

-*f*-[A]-COOH =

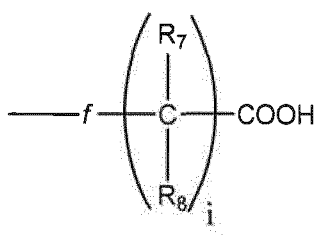

Formule II

dans laquelle :

- i supérieur ou égal à 1 et inférieur ou égal à 12, et,
- $-R_7$ et $-R_8$, identiques ou différents, sont choisis dans le groupe constitué par un atome d'hydrogène, un alkyle saturé ou insaturé, linéaire, ramifié ou cyclique en C1 à C6, un benzyle, un alkyle-aryle et comportant éventuellement des hétéroatomes choisis dans le groupe constitué par O, N et/ou S, ou des fonctions choisies dans le groupe constitué par les fonctions acide carboxylique, amine, alcool ou thiol.

8. Composition selon l'une quelconque des revendications précédentes, **caractérisée en ce que** le polymère anionique hydrophobisé est choisi parmi les composés de formules IV à VII et XII, dans lesquelles le radical -f-[A]-COOH, comprenant de 2 à 8 atomes de carbone, est issu d'un acide aminé, d'un dialcool, d'une diamine, d'un diacide ou d'un amine-alcool.

9. Composition selon l'une quelconque des revendications précédentes, **caractérisée en ce que** le polymère anionique hydrophobisé est choisi parmi les composés de formules IV à VII et XII, dans lesquelles le radical -*f*-[A]-COOH, comprenant de 2 à 6 atomes de carbone, est issu d'un acide aminé, d'un dialcool, d'une diamine, d'un diacide ou d'un amine-alcool.

10. Composition selon l'une quelconque des revendications précédentes, **caractérisée en ce que** le polymère anionique hydrophobisé est choisi parmi les polymères anioniques hydrophobisés de formules IV à VII et XII répondant aux conditions suivantes :

    - lorsque -*g*-[B]-(*k*-[D])$_p$ comporte une chaîne Hy et que Hy est un alkyle en C8 à C15, alors le produit entre le degré de substitution en radicaux hydrophobes et le degré moyen de polymérisation (n) est supérieur ou égal à 2.
    - lorsque -*g*-[B]-(*k*-[D])$_p$ comporte une chaine Hy et que Hy est un alkyle en C16 à C20, alors le produit entre le degré de substitution en radicaux hydrophobes et le degré moyen de polymérisation (n) est supérieur ou égal à 1
    - lorsque -*g*-[B]-(*k*-[D])$_p$ comporte deux chaînes Hy et que Hy est un alkyle en C8 à C9, alors le produit du degré de substitution en radicaux hydrophobes et du degré moyen de polymérisation (n) est supérieur ou égal à 2,
    - lorsque -*g*-[B]-(*k*-[D])$_p$ comporte deux chaînes Hy et que Hy est un alkyle en C10 à C16, alors le produit du degré de substitution en radicaux hydrophobes et du degré moyen de polymérisation (n) est supérieur ou égal à 0,2.

11. Composition selon l'une quelconque des revendications précédentes, **caractérisée en ce que** le polymère anionique hydrophobisé est choisi parmi les polymères anioniques hydrophobisés de formules IV à VII et XII, dans lesquelles le radical -f-[A]-COOH est choisi dans le groupe constitué par les radicaux suivants, *f* ayant la signification donnée ci-dessus :

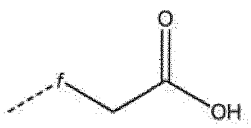
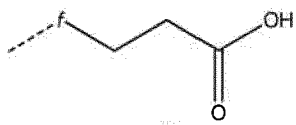

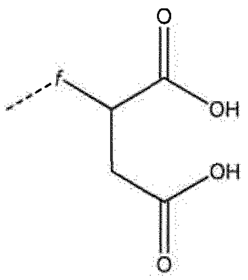
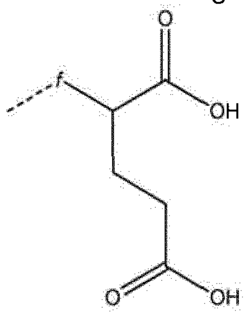

ou leur sels de cations alcalins choisis dans le groupe constitué de $Na^+$ et $K^+$.

12. Composition selon l'une quelconque des revendications précédentes, **caractérisée en ce que** l'insuline basale dont le point isoélectrique est compris entre 5,8 et 8,5 est l'insuline glargine.

13. Composition selon l'une quelconque des revendications précédentes, **caractérisée en ce qu'**elle comprend entre 40 et 500 UI/mL d'insuline basale dont le point isoélectrique est compris entre 5,8 et 8,5.

14. Composition selon l'une quelconque des revendications précédentes, **caractérisée en ce que** la concentration en polymère anionique hydrophobisé est au plus de 100 mg/mL.

**15.** Composition selon l'une quelconque des revendications précédentes, **caractérisée en ce qu'**elle comprend en outre une insuline prandiale.

**16.** Composition selon l'une quelconque des revendications précédentes, **caractérisée en ce qu'**elle comprend au total entre 40 et 800 UI/mL d'insuline avec une combinaison d'insuline prandiale et d'insuline basale dont le point isoélectrique est compris entre 5,8 et 8,5.

**17.** Composition selon l'une quelconque des revendications 15 ou 16, **caractérisée en ce que** les proportions entre l'insuline basale dont le point isoélectrique est compris entre 5,8 et 8,5 et l'insuline prandiale sont par exemple en pourcentage de 25/75, 30/70, 40/60, 50/50, 60/40, 70/30, 75/25, 80/20, 90/10 pour des formulations comprenant de 40 à 800 UI/mL.

**Patentansprüche**

**1.** Mischung in Form einer einspritzbaren wässrigen Lösung, deren pH-Wert zwischen 6,6 und 7,8 liegt, die mindestens aus folgenden Bestandteilen besteht:

a) ein basales Insulin, dessen isoelektrischer Punkt pI zwischen 5,8 und 8,5 liegt;
b) ein hydrophobiertes anionisches Polymer mit der Formel XII:

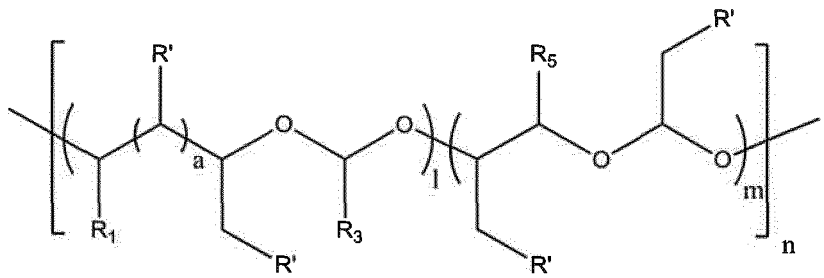

Formel XII

bei der

- l = 0 oder 1,
- m = 0, 1 oder 2,
- a = 0 oder 1,
- wobei n der Polymerisierungsgrad ist, der zwischen 3 und 1.000 liegt,

und

- -$R_1$ ein Wasserstoff -H ist,
- -$R_3$ ein Radikal -$CH_2R'$ ist,
- -$R_5$ entweder eine Gruppe -COOH, oder ein Radikal -$CH_2R'$, oder ein Radikal -*k*-[D] ist,

in dem:

- -[D] ein Radikal -[Hy] oder -[E]-(*o*-[Hy])$_t$ ist;
- -[E]- ein mindestens zweiwertiges Radikal aus 1 bis 15 Kohlenstoffatome ist, das mindestens ein Heteroatom enthält, das unter O, N und S gewählt wird, welches ggf. Carboxyl- oder Amino- und/oder -*k*-[E]-(*o*)$_t$-Funktionen umfasst; es enthält 2 bis 16 Kohlenstoffatome und ist aus einer Aminosäure, einem zweiwertigen Alkohol oder einem Alkamin hervorgegangen;
- -[Hy] eine lineare oder zyklische Alkylgruppe, ein Alkylaryl oder ein C8 bis 30-Arylalkyl ist, welches ggf. durch eine oder mehrere C1 bis C3-Alkylgruppen ersetzt wird, das aus einer hydrophoben Mischung hervorgegangen ist;
- wobei k aus der Reaktion zwischen einer Carboxyl-, Amino- oder Alkoholfunktion des Zwischenstoffs von -*k*-[E]-(*o*)$_t$ und einer Alkohol-, Carboxyl- oder Aminofunktion des Polymers entstanden ist und einer Funktion entspricht, die in der Gruppe aus den Ester-, Amid-, Carbonat- und Carbamatfunktionen besteht;
- wobei o aus der Reaktion zwischen einer Carboxyl-, Amino- oder Alkoholfunktion des Zwischenstoffs von -*k*-[E]-(*o*)$_t$ und einer Alkohol- oder Säurefunktion des Zwischenstoffs von -[Hy] entstanden ist und einer

Funktion entspricht, die in der Gruppe aus den Ester-, Amid-, Harnstoff-(Carbamid), Carbonat- und Carbamatfunktionen besteht;
- t eine positive Ganzzahl gleich 1 oder 2 ist;

oder
• $-R_1$ und $-R_3$ einen Zyklus mit 6 $-R_1$-$R_3$- Gliedern bilden = $-CH(NHCOCH_3)-$, und,
• -R' aus der von den Radikalen gebildeten Gruppe gewählt wird:

◦ -OH
◦ -O-Alk, wobei Alk eine C1 bis C3-Alkylkette ist,
◦ -(f-[A]-COOH), wobei:

- -[A]- ein mindestens zweiwertiges Radikal aus 1 bis 15 Kohlenstoffatomen ist, das mindestens ein Heteroatom enthält, das unter O, N und S gewählt wird, welches ggf. Carboxyl- oder Amino- und/oder - *f*-[A]-COOH-Funktionen umfasst, aus einer Aminosäure, einer zweiwertigen Säure oder einer Alkoholsäure entstanden ist und mit dem Molekulargerüst durch eine *f*-Funktion verbunden ist;
- wobei *f* aus der Reaktion zwischen einer Carboxyl- oder Alkohol- oder Aminofunktion des Zwischenstoffs von -*f*-[A]-COOH und einer Hydroxylfunktion des Gerüsts hervorgeht und aus der Gruppe der Ether-, Ester-Carbamat- oder Carbonatfunktionen hervorgeht;

◦ -*g*-[B]-(*k*-[D])$_p$, wobei:

- -[A]- ein mindestens zweiwertiges Radikal aus 1 bis 15 Kohlenstoffatomen ist, das mindestens ein Heteroatom enthält, das unter O, N und S gewählt wird, welches ggf. Carboxyl- oder Amino- und/oder -*g*-[B]-(*k*-)$_p$-Funktionen umfasst, aus einer Aminosäure, einer zweiwertigen Säure, einem zweiwertigen Alkohol, einer Alkoholsäure, einem zweiwertigen Amin oder einem Aminoalkohol entstanden ist und mit dem Molekulargerüst durch eine g-Funktion verbunden ist sowie mittels einer k-Funktion mit mindestens einem Radikal -[D] verbunden ist,
- wobei g aus der Reaktion zwischen einer Carboxyl- oder Alkohol- oder Aminofunktion des Zwischenstoffs von -*g*-[B]-(k-)$_p$ und einer Funktion des Gerüsts hervorgeht und aus der Gruppe der Ether-, Amino-, Ester-Carbamat- oder Carbonatfunktionen hervorgeht;
- wobei k aus der Reaktion zwischen einer Carboxyl- oder Alkohol- oder Aminofunktion des Zwischenstoffs von -*g*-[B]-(k-)$_p$ und einer Alkohol- oder Säurefunktion des Zwischenstoffs von -[D] hervorgeht, die aus der Gruppe der Ester-, Amid- oder Caramatfunktionen hervorgeht;
- p eine positive Ganzzahl gleich 1 oder 2 ist;

und -[A]-, -[B]- und -[E]- identisch oder unterschiedlich sind,
und *k* und o identisch oder unterschiedlich sind;
und, wenn -[B]- ein dreiwertiges Radikal ist, dann ist -[D] ein Radikal -[Hy],
und der Austauschgrad der Carboxylatfüllstoffe der durchschnittlichen Anzahl der Carboxylatfüllstoffe pro Monomer geteilt durch (l+m) entspricht und größer oder gleich 0,4 ist.
und der Austauschgrad der hydrophoben Radikale der durchschnittlichen Anzahl der hydrophoben Radikale pro Monomer geteilt durch (l+m) entspricht und kleiner oder gleich 0,5 ist.
und, wenn das hydrophobierte anionische Polymer ein Polysaccharid ist, dann sind die Glycosidverbindungen identisch oder unterschiedlich und können dem Typ α und/oder dem Typ β entsprechen.

2. Mischung gemäß Anspruch 1, **dadurch gekennzeichnet, dass** das hydrophobierte anionische Polymer aus der Gruppe der hydrophobierten anionischen Polymere der Formel XII gewählt wird, bei der l = 0 und m = 1 sind, das aus der Gruppe der hydrophobierten anionischen Polymere der Formel IV gewählt wird:

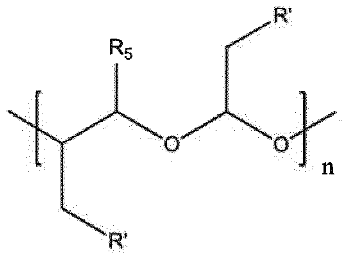

Formel IV

in der -Rs entweder eine Gruppe -COOH, oder ein Radikal -$CH_2$R', oder ein Radikal -*k*-[D] ist, wobei -R' und n der Definition in Anspruch 1 entsprechen.

3. Mischung gemäß Anspruch 2, **dadurch gekennzeichnet, dass** das hydrophobierte anionische Polymer unter den hydrophobierten anionischen Polymeren der Formel IV gewählt wird:

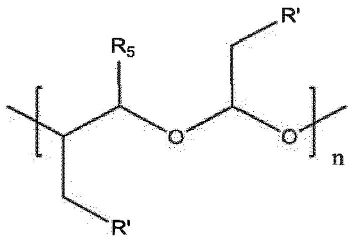

Formel IV

bei der -Rs ein Radikal -$CH_2$R' ist, wobei -R' und n der Definition in Anspruch 1 entsprechen.

4. Mischung gemäß Anspruch 1, **dadurch gekennzeichnet, dass** das hydrophobierte anionische Polymer aus der Gruppe der hydrophobierten anionischen Polymere der Formel XII gewählt wird, bei der m = 0, l = 1 und a = 0 sind, d,h. die Auswahl erfolgt unter den hydrophobierten anionischen Polymeren der Formel V:

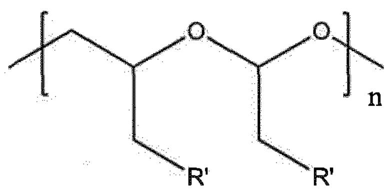

Formel V

wobei -R' und n der Definition in Anspruch 1 entsprechen.

5. Mischung gemäß Anspruch 1, **dadurch gekennzeichnet, dass** das hydrophobierte anionische Polymer aus der Gruppe der hydrophobierten anionischen Polymere der Formel XII gewählt wird, bei der l = 1, m = 2 und a = 0 sind, d,h. die Auswahl erfolgt unter den hydrophobierten anionischen Polymeren der Formel VI:

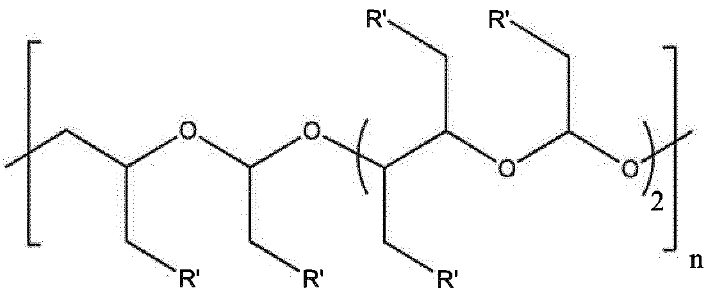

Formel VI

wobei -R’ und n der Definition in Anspruch 1 entsprechen.

6. Mischung gemäß Anspruch 1, **dadurch gekennzeichnet, dass** das hydrophobierte anionische Polymer aus der Gruppe der hydrophobierten anionischen Polymere der Formel XII gewählt wird, bei der m = 1, l = 1, a = 1, $-R_1-R_3-$ = $-CH(NHCOCH_3)-$, $-R_2 = -CH_2R'$, $-R_4 = -CH_2R'$ sind, -Rs entweder einer Gruppe -COOH, oder einem Radikal -k-[D] entspricht, $-R_6 = -CH_2R'$ ist, d,h. die Auswahl erfolgt unter den hydrophobierten anionischen Polymeren der Formel VII:

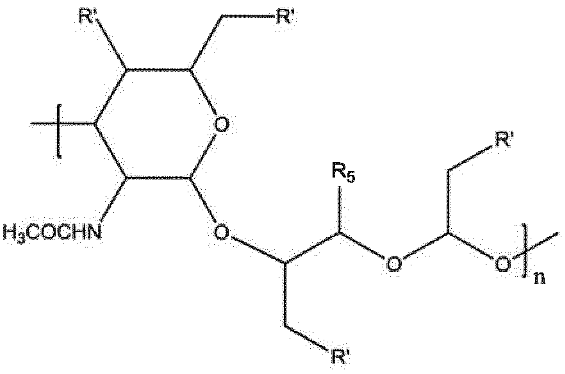

Formel VII

wobei R’ und n der Definition in Anspruch 1 entsprechen.

7. Mischung gemäß einem der obigen Ansprüche, **dadurch gekennzeichnet, dass** das hydrophobierte anionische Polymer unter den hydrophobierten anionischen Polymeren der Formeln IV bis VII und XII gewählt wird, in denen das Radikal -f-[A]-COOH unter den Radikalen der folgenden Formel II gewählt wird:

-*f*-[A]-COOH = 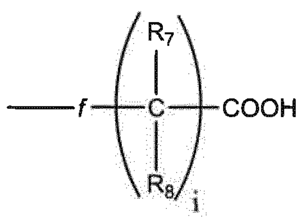

Formel II

wobei:

- i größer oder gleich 1 und kleiner oder gleich 12 ist, und
- $-R_7$ und $-R_8$ identisch oder gleich sind und in der Gruppe gewählt werden, die aus einem Wasserstoffatom, einem gesättigten oder ungesättigten, linearen, verzweigten oder C1 bis C6-zyklischen Alkyl, einem Benzyl und einem Alkyl-Aryl bestehen und ggf. Heteroatome enthalten, die aus der Gruppe der O, N und S gewählt werden, oder Funktionen, die aus der Gruppe bestehend aus den Carboxylsäure-, Amino-, Alkohol- oder Thiolfunktionen gewählt wurden.

**8.** Mischung gemäß einem der obigen Ansprüche, **dadurch gekennzeichnet, dass** das hydrophobierte anionische Polymer unter den Gemischen der Formeln IV bis VII und XII gewählt wird, bei denen das Radikal -*f*-[A]-COOH, das 2 bis 8 Kohlenstoffatome umfasst, aus einer Aminosäure, einem zweiwertigen Alkohol, einem zweiwertigen Amin, einer zweiwertigen Säure oder einem Aminoalkohol entsteht.

**9.** Mischung gemäß einem der obigen Ansprüche, **dadurch gekennzeichnet, dass** das hydrophobierte anionische Polymer unter den Gemischen der Formeln IV bis VII und XII gewählt wird, bei denen das Radikal -f-[A]-COOH, das 2 bis 6 Kohlenstoffatome umfasst, aus einer Aminosäure, einem zweiwertigen Alkohol, einem zweiwertigen Amin, einer zweiwertigen Säure oder einem Aminoalkohol entsteht.

**10.** Mischung gemäß einem der obigen Ansprüche, **dadurch gekennzeichnet, dass** das hydrophobierte anionische Polymer unter den hydrophobierten anionischen Polymeren der Formeln IV bis VII und XII gewählt wird, die den folgenden Bedingungen entsprechen:

- Wenn -*g*-[B]-(*k*-[D])$_p$ eine Hy-Kette umfasst und Hy ein C8 bis C15-Alkyl ist, ist das Ergebnis aus dem Austauschgrad der hydrophoben Radikale und dem durchschnittlichen Polymerisierungsgrad (n) größer oder gleich 2.
- Wenn -*g*-[B]-(*k*-[D])$_p$ eine Hy-Kette umfasst und Hy ein C16 bis C20-Alkyl ist, ist das Ergebnis aus dem Austauschgrad der hydrophoben Radikale und dem durchschnittlichen Polymerisierungsgrad (n) größer oder gleich 1.
- Wenn -*g*-[B]-(*k*-[D])$_p$ zwei Hy-Ketten umfasst und Hy ein C8 bis C9-Alkyl ist, ist das Ergebnis aus dem Austauschgrad der hydrophoben Radikale und dem durchschnittlichen Polymerisierungsgrad (n) größer oder gleich 2.
- Wenn -*g*-[B]-(*k*-[D])$_p$ zwei Hy-Ketten umfasst und Hy ein C10 bis C16-Alkyl ist, ist das Ergebnis aus dem Austauschgrad der hydrophoben Radikale und dem durchschnittlichen Polymerisierungsgrad (n) größer oder gleich 0,2.

**11.** Mischung gemäß einem der obigen Ansprüche, **dadurch gekennzeichnet, dass** das hydrophobierte anionische Polymer unter den hydrophobierten anionischen Polymeren der Formeln IV bis VII und XII gewählt wird, in denen das Radikal -*f*-[A]-COOH aus der Gruppe der folgenden Radikale gewählt wird, wobei *f* die unten aufgeführte Bedeutung hat:

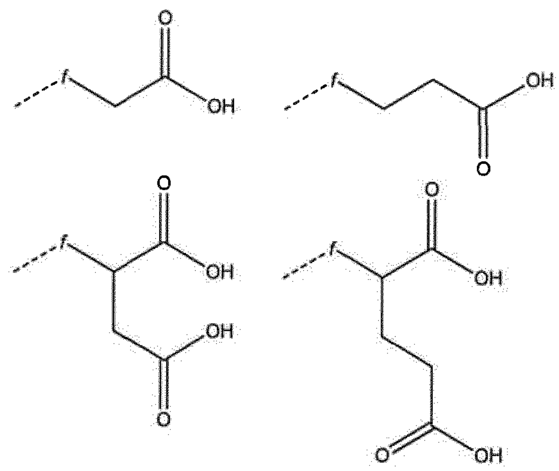

oder ihrer alkalischen Kationensalze, die aus der Gruppe gewählt werden, die aus $Na^+$ und $K^+$ besteht.

**12.** Mischung gemäß einem der obigen Ansprüche, **dadurch gekennzeichnet, dass** das basale Insulin, dessen isoelektrischer Punkt zwischen 5,8 und 8,5 liegt, das Insulin glargin ist.

**13.** Mischung gemäß einem der obigen Ansprüche, **dadurch gekennzeichnet, dass** sie zwischen 40 und 500 UI/ml des basalen Insulins enthält, dessen isoelektrischer Punkt zwischen 5,8 und 8,5 liegt.

**14.** Mischung gemäß einem der obigen Ansprüche, **dadurch gekennzeichnet, dass** die Konzentration der hydrophobierten anionischen Polymere maximal 100 mg/ml beträgt.

**15.** Mischung gemäß einem der obigen Ansprüche, **dadurch gekennzeichnet, dass** sie unter Anderem ein prandiales Insulin enthält.

**16.** Mischung gemäß einem der obigen Ansprüche, **dadurch gekennzeichnet, dass** sie insgesamt zwischen 40 und 800 UI/ml Insulin mit einer Kombination aus prandialem und basalem Insulin enthält, deren isoelektrischer Punkt zwischen 5,8 und 8,5 liegt.

**17.** Mischung gemäß einem der Ansprüche 15 oder 16, **dadurch gekennzeichnet, dass** das Verhältnis zwischen dem basalen Insulin, dessen isoelektrischer Punkt zwischen 5,8 und 8,5 liegt, und dem prandialen Insulin bei Formeln, die 40 bis 800 UI/ml enthalten, zum Beispiel 25/75, 30/70, 40/60, 50/50, 60/40, 70/30, 75/25, 80/20, 90/10 beträgt.

## Claims

**1.** A composition in the form of an injectable aqueous solution having a pH of between 6.6 and 7.8, including at least:

a) a basal insulin having an isoelectric point pI of between 5.8 and 8.5;
b) a hydrophobized anionic polymer of formula XII:

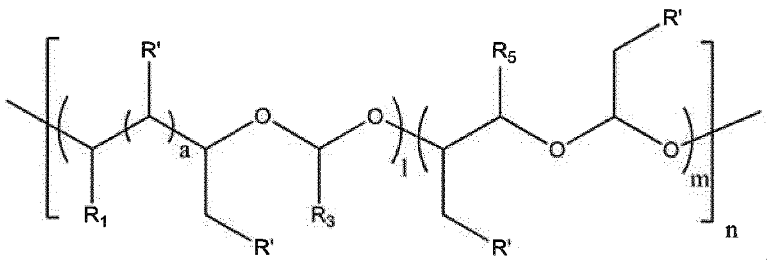

Formula XII

in which,

- l = 0 or 1,
- m = 0, 1 or 2,
- a = 0 or 1,
- n being the polymerisation degree, between 3 and 1000,
and
- $R_1$ is a hydrogen -H,
- $R_3$ is a $-CH_2R'$ radical,
- Rs is a -COOH group, or a $-CH_2R'$ radical, or a -*k*-[D] radical, in which:

- -[D] is a -[Hy] or -[E]-(o-[Hy])t radical;
- -[E]- is an at least divalent radical including from 1 to 15 carbon atoms including at least one heteroatom selected from O, N and S, optionally bearing carboxyl or amine functions and/or -*k*-[E]-(*o*)$_t$, including from 2 to 16 carbon atoms, is derived from an amino acid, from a dialcohol, from a diamine or from an amine alcohol;
- -[Hy] is a linear or cyclic alkyl group, an alkyl aryl or aryl alkyl, in C8-C30, optionally substituted by one or more C1-C3 alkyl groups, derived from a hydrophobic compound;
- *k* resulting from the reaction between a carboxyl, amine or alcohol function of the precursor of -*k*-[E]-(*o*)$_t$ and an alcohol, carboxyl or amine function of the polymer and is a function selected from the group consisting of the ester, amide, carbonate and carbamate functions;
- *o* resulting from the reaction between a carboxyl, amine or alcohol function of the precursor of -k-[E]-(o)t and an alcohol or acid function of the precursor of -[Hy] is a function selected from the group consisting of the ester, amide, urea (carbamide), carbonate and carbamate function;
- t is a positive integer equal to 1 or 2;

or

• $-R_1$ and -R3 form a six-member ring $-R_1-R_3- = -CH(NHCOCH_3)-$,

and

• -R' is selected from the group consisting of the radicals:

◦ -OH
◦ -O-Alk, Alk being a C1-C3 alkyl chain,
◦ -(*f*-[A]-COOH), in which:

- [A]- is an at least divalent radical including from 1 to 15 carbon atoms including at least one heteroatom selected from O, N and S, optionally bearing carboxyl or amine functions and/or -*f*-[A]-COOH is derived from an amine function, from a diacid or from an alcohol acid and is bound to the backbone of the molecule by a function *f*;
- *f* resulting from the reaction between a carboxyl or alcohol or amine function of the precursor of -*f*-[A]-COOH and a hydroxyl function of the backbone is selected from the group consisting of the ether, ester, carbamate or carbonate functions;

◦ -*g*-[B]-(*k*-[D])$_p$, in which:

- [B]- is an at least divalent radical including from 1 to 15 carbon atoms including at least one heteroatom selected from O, N and S, optionally bearing carboxyl or amine functions and/or -*g*-[B]-(k-)$_p$ is derived from an amino acid, from a diacid, from a dialcohol, from an alcohol acid, from a diamine or from an amine alcohol and is bound to the backbone of the molecule by a function g and is bound to at least a -[D] radical by a function k,
- *g* resulting from the reaction between a carboxyl or alcohol or amine function of the precursor of -*g*-[B]-(k-)$_p$ and a function of the backbone is selected from the group consisting of the ether, amine, ester, carbamate or carbonate functions,
- *k* resulting from the reaction between a carboxyl or alcohol or amine function of the precursor of -*g*-[B]-(k-)$_p$ and an alcohol or acid function of the precursor of -[D] selected from the group consisting of the ester, amide or carbamate functions;
- p is a positive integer equal to 1 or 2;

and [A]-, -[B]- and -[E]- are identical or different,
and *k* and o are identical or different;
and, if -[B]- is a trivalent radial, then -[D] is a -[Hy] radical,
and the degree of substitution with carboxylate charges is the average number of carboxylate charges per monomer divided by (l+m) and is greater than or equal to 0.4,
and the degree of substitution with hydrophobic radicals is the average number of hydrophobic radicals per monomer divided by [l+m] and is less than or equal to 0.5,
and, if the hydrophobized anionic polymer is a polysaccharide, then the identical or different glycosidic bonds can be of $\alpha$ type and/or of $\beta$ type.

2. The composition according to Claim 1, **characterised in that** the hydrophobized anionic polymer is selected from the hydrophobized anionic polymers of formula XII, in which l = 0 and m = 1, selected from the hydrophobized anionic polymers of formula IV:

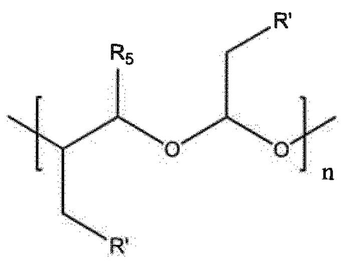

Formula IV

in which -Rs is a -COOH group, or a $-CH_2R'$ radical, or a -*k*-[D] radical, -R' and n being as defined in Claim 1.

3. The composition according to Claim 2, **characterised in that** the hydrophobized anionic polymer is selected from the hydrophobized anionic polymers of formula IV:

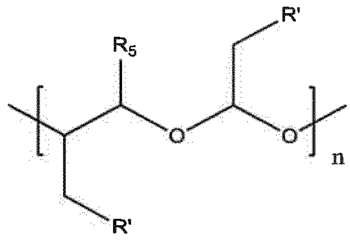

Formula IV

in which $-R_5$ is a $-CH_2R'$ radical, -R' and n are as defined in Claim 1.

4. The composition according to Claim 1, **characterised in that** the hydrophobized anionic polymer is selected from the hydrophobized anionic polymers of formula XII, in which m = 0, l = 1, a = 0, in other words it is selected from the hydrophobized anionic polymers
of formula V:

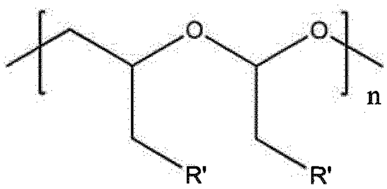

Formula V

and -R' and n are as defined in Claim 1.

5. The composition according to Claim 1, **characterised in that** the hydrophobized anionic polymer is selected from the hydrophobized anionic polymers of formula XII, in which l = 1, m = 2 and a = 0, in other words it is selected from the hydrophobized anionic polymers of formula VI

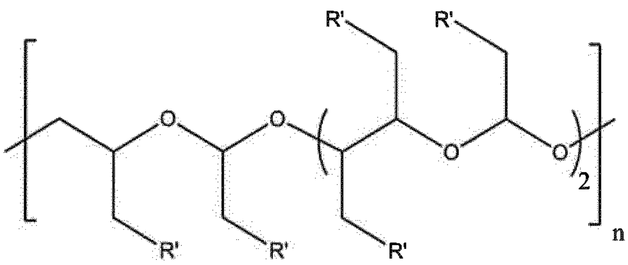

Formula VI

and -R' and n are as defined in Claim 1.

6. The composition according to Claim 1, **characterised in that** the hydrophobized anionic polymer is selected from the hydrophobized anionic polymers of formula XII, in which m = 1, l = 1, a = 1, $-R_1$-$R_3$- = $-CH(NHCOCH_3)-$, $-R_2$ = $-CH_2R'$, $-R_4$ = $-CH_2R'$, $-R_5$ is either a -COOH group or a -k-[D] radical, $-R_6$ = $-CH_2R'$, in other words it is selected from the hydrophobized anionic polymers of formula VII:

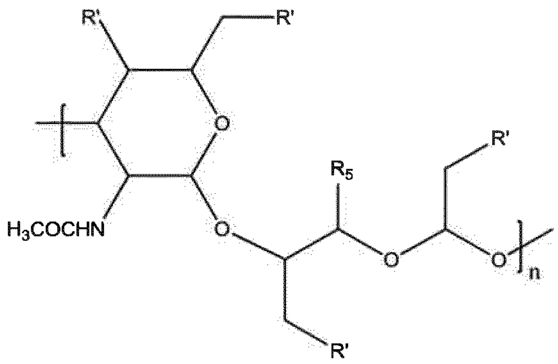

Formula VII

R' and n are as defined in Claim 1.

7. The composition according to any one of the preceding claims, **characterised in that** the hydrophobized anionic polymer is selected from the hydrophobized anionic polymers of formulas IV to VII and XII, in which the -*f*-[A]-COOH radical is selected from the radicals of the following formula II:

-*f*-[A]-COOH =

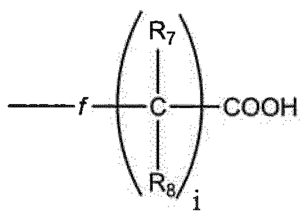

Formula II

in which:

- i being greater than or equal to 1 and less than or equal to 12, and,
- $-R_7$ and $-R_8$, which are identical or different, are selected from the group consisting of a hydrogen atom, a C1-C6 saturated or unsaturated, linear, branched or cyclic alkyl, a benzyl, an alkyl aryl and optionally comprising heteroatoms selected from the group consisting of O, N and/or S, or functions selected from the group consisting of the carboxylic acid, amine, alcohol or thiol functions.

8. The composition according to any one of the preceding claims, **characterised in that** the hydrophobized anionic polymer is selected from the compounds of formulas IV to VII and XII, in which the -*f*-[A]-COOH radical, including from 2 to 8 carbon atoms, is derived from an amino acid, from a dialcohol, from a diamine, from a diacid and from an amine alcohol.

9. The composition according to any one of the preceding claims, **characterised in that** the hydrophobized anionic polymer is selected from the compounds of formulas IV to VII and XII, in which the radical -f-[A]-COOH, including from 2 to 6 carbon atoms, is derived from an amino acid, from a dialcohol, from a diamine, from a diacid or from an amine alcohol.

10. The composition according to any one of the preceding claims, **characterised in that** the hydrophobized anionic polymer is selected from the hydrophobized anionic polymers of formulas IV to VII and XII satisfying the following conditions:

- when -*g*-[B]-(*k*-[D])$_p$ comprises an Hy chain, and when Hy is a C8-C15 alkyl, then the product of the degree of substitution with hydrophobic radicals and the average polymerisation degree (n) is greater than or equal to 2.
- when -*g*-[B]-(*k*-[D])$_p$ comprises an Hy chain, and Hy is a C16-C20 alkyl, then the product of the degree of substitution with hydrophobic radicals and the average polymerisation degree (n) is greater than or equal to 1
- when -*g*-[B]-(*k*-[D])$_p$ comprises an Hy chain, and when Hy is a C8-C9 alkyl, then the product of the degree of substitution with hydrophobic radicals and the average polymerisation degree (n) is greater than or equal to 2,
- when -*g*-[B]-(*k*-[D])$_p$ comprises an Hy chain, and when Hy is a C10-C16 alkyl, then the product of the degree of substitution with hydrophobic radicals and the average polymerisation degree (n) is greater than or equal to 0.2.

**11.** The composition according to any one of the preceding claims, **characterised in that** the hydrophobized anionic polymer is selected from the hydrophobized anionic polymers of formulas IV to VII and XII, in which the -*f*-[A]-COOH radical is selected from the group consisting of the following radicals, *f* having the meaning given above:

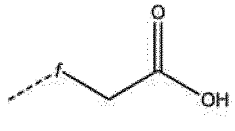
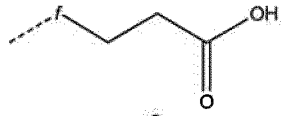
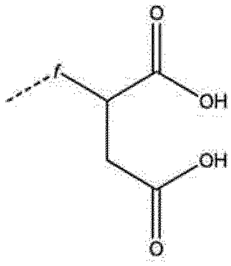
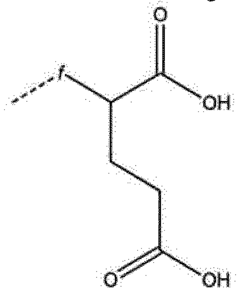

or their alkaline cation salts selected from the group consisting of $Na^+$ and $K^+$.

**12.** The composition according to any one of the preceding claims, **characterised in that** the basal insulin having an isoelectric point of between 5.8 and 8.5, is insulin glargine.

**13.** The composition according to any one of the preceding claims, **characterised in that** it includes between 40 and 500 IU/mL of basal insulin having an isoelectric point of between 5.8 and 8.5.

**14.** The composition according to any one of the preceding claims, **characterised in that** the concentration of hydrophobized anionic polymer is at most 100 mg/mL.

**15.** The composition according to any one of the preceding claims, **characterised in that** it additionally includes a prandial insulin.

**16.** The composition according to any one of the preceding claims, **characterised in that** it includes in total between 40 and 800 IU/mL of insulin with a combination of prandial insulin and basal insulin having an isoelectric point of between 5.8 and 8.5.

**17.** The composition according to either Claim 15 or 16, **characterised in that** the proportions between the basal insulin having an isoelectric point of between 5.8 and 8.5, and the prandial insulin are, for example, as a percentage, 25/75, 30/70, 40/60, 50/50, 60/40, 70/30, 75/25, 80/20, 90/10 for formulations including from 40 to 800 IU/mL.

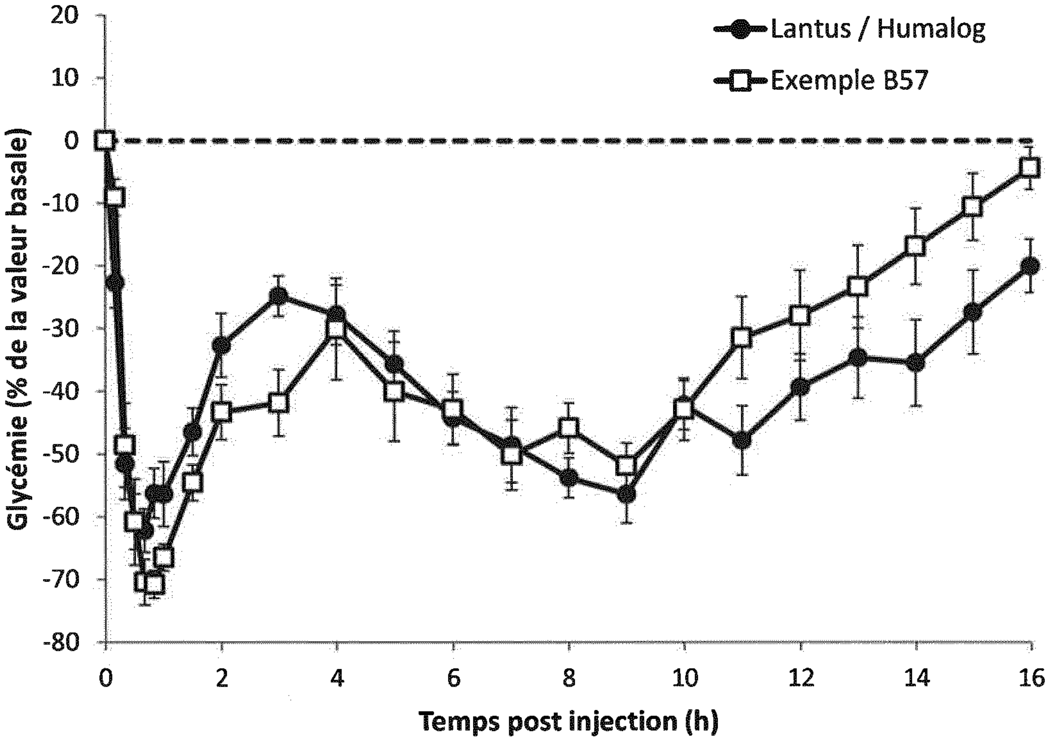
Lantus / Humalog
Exemple B57
Glycémie (% de la valeur basale)
20
10
0
-10
-20
-30
-40
-50
-60
-70
-80
0
2
4
6
8
10
12
14
16
Temps post injection (h)

Figure 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 5656722 A
- WO 2007121256 A
- WO 2009021955 A
- WO 2011144673 A
- WO 2011147980 A
- WO 2009063072 A
- US 7718609 B
- WO 2011144676 A
- WO 2003053339 A
- WO 2004096854 A
- US 6100376 A
- US 2387201 A, Weiner, N
- US 4826818 A, Kenji M.
- WO 2010053140 A, Akiyoshi K.
- WO 2012153070 A


### Littérature non-brevet citée dans la description


- *69th Scientific Sessions of the American Diabetes Association,* 05 Juin 2009
- **E. CENGIZ et al.** *Diabetes care,* 2010, vol. 33 (5), 1009-12
- Effect of sulfobutyl ether-β-cyclodextrin on bioavailability of insulin glargine and blood glucose level after subcutaneous injection to rats. *International Journal of Pharmaceutics,* 2011, vol. 419, 71-76
- *Carbohydrate Research,* 1978, vol. 64, 189-197
- *Biomacromolecules,* 2005, vol. 6, 2659-2670
- **TAKATA, J et al.** *Journal of Pharmaceutical Sciences,* 1995, vol. 84 (1), 96-100